# EUROPEAN PATENT SPECIFICATION

(11) **EP 2 477 985 B1**
(45) Date of publication and mention of the grant of the patent: **07.11.2018**
(21) Application number: 10817784.1
(22) Date of filing: 15.09.2010
(51) Int. Cl.: A61K 31/724, A61P 35/00, A61K 47/61

(54) **CRLX101 FOR USE IN THE TREATMENT OF CANCER**
CRLX101 ZUR VERWENDUNG BEI DER KREBSBEHANDLUNG
CRLX101 POUR SON UTILISATION DANS LE TRAITEMENT DU CANCER

(30) Priority: 15.09.2009 US 242752 P; 24.03.2010 US 317039 P; 06.05.2010 US 332150 P; 10.09.2010 US 381851 P
(43) Date of publication of application: 25.07.2012
(73) Proprietor: BlueLink Pharmaceuticals, Inc., Ames, Iowa 50010 (US)
(72) Inventor: YEN, Yun, Arcadia, CA 91006 (US); SCHLUEP, Thomas, Las Cresenta, CA 91214 (US); RYAN, John, Philadelphia, PA 19118 (US); DAVIS, Mark, Pasadena, CA 91105 (US); OLIVER, James, C., Raleigh, NC 27612 (US)
(74) Representative: Simpson, Tobias Rutger
(86) International application number: PCT/US2010/048973
(87) International publication number: WO 2011/034954

(56) References cited:
- WO-A1-2012/125232
- WO-A2-2006/089007
- WO-A2-2008/148080
- CN-A- 1 534 036
- US-A1- 2002 111 362
- US-A1- 2008 193 498
- US-A1- 2009 202 989
- US-B1- 6 630 124
- T. NUMBENJAPON ET AL: "Preclinical Results of Camptothecin-Polymer Conjugate (IT-101) in Multiple Human Lymphoma Xenograft Models", CLINICAL CANCER RESEARCH, vol. 15, no. 13, 23 June 2009 (2009-06-23) , pages 4365-4373, XP055021505, ISSN: 1078-0432, DOI: 10.1158/1078-0432.CCR-08-2619
- DAVIS ET AL: "Design and development of IT-101, a cyclodextrin-containing polymer conjugate of camptothecin", ADVANCED DRUG DELIVERY REVIEWS, ELSEVIER BV, AMSTERDAM, NL, vol. 61, no. 13, 12 November 2009 (2009-11-12), pages 1189-1192, XP026698382, ISSN: 0169-409X, DOI: 10.1016/J.ADDR.2009.05.005 [retrieved on 2009-08-12]
- J. C. Oliver, Y. Yen, T. W. Synold, T. Schluep and M. Davis: "A dose-finding pharmacokinetic study of IT-101, the first de novo designed nanoparticle therapeutic, in refractory solid tumors", , May 2008 (2008-05), XP002694709, Retrieved from the Internet: URL:http://meeting.ascopubs.org/cgi/conten t/abstract/26/15_suppl/14538 [retrieved on 2012-03-27]
- SCHLUEP ET AL: 'Preclinical efficacy of the camptothecin-polymer conjugate IT-101 in multiple cancer models' CLIN CANCER RES vol. 12, 2006, pages 1606 - 1614, XP008154116
- HOMSI ET AL: 'Phase I trial of poly-L-glutamate camptothecin (CT-2106) administered weekly in patients with advanced solid malignancies' CLIN CANCER RES vol. 13, 2007, pages 5855 - 5861, XP008154169

## Description

### Background of the Invention

Drug delivery and dosing of small molecule therapeutic agents, such as camptothecin, can be problematic due to a number issues including half-life, toxicity, distribution etc.

Numbenjapon et al. (Clin. Cancer Res., 15(13), 2009, 4365-4373), Oliver et al. (J. Clin. Oncol., 2008, 26 (15S), 2008, 14538), and Schluep et al. (Clin. Cancer Res., 12(5), 2006, 1606-1614) describe various studies of a camptothecin-polymer conjugate.

WO 2006/089007 discloses compositions of therapeutic polymeric compounds designed as carriers for the delivery of small molecule therapeutics.

### Summary of the Invention

The present invention is as defined in the appended claims.

More generally, the present disclosure features, in one aspect, a method of treating a proliferative disorder, e.g., a cancer, in a subject. The method comprises:
providing an initial administration of a CDP-topoisomerase inhibitor conjugate, particle or composition, e.g., a CDP-camptothecin conjugate, particle or composition or camptothecin derivative conjugate, particle or composition, e.g., a CDP-camptothecin conjugate, particle or composition or camptothecin derivative conjugate, particle or composition described herein, e.g., CRLX101, to said subject at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m², (wherein said dosage is expressed in mg of drug, as opposed to mg of conjugate) and
optionally, providing one or more subsequent administrations of said CDP-topoisomerase inhibitor conjugate, particle or composition, e.g., a CDP-camptothecin conjugate, particle or composition or camptothecin derivative conjugate, particle or composition, e.g., a CDP-camptothecin conjugate, particle or composition or camptothecin derivative conjugate, particle or composition described herein, e.g., CRLX101, at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m², wherein each subsequent administration is provided, independently, between 9, 10, 11, 12, 13, 14, 15 or 16 days after the previous, e.g., the initial, administration, to thereby treat the proliferative disorder.

In an embodiment, the dosage of at least 2, 3, 4, 5, 6, 7, 8, 9, 10, 12, 15 or 20 administrations is the same.

In an embodiment, the time between at least 2, 3, 4, 5, 6, 7, 8, 9, 10, 12, 15, or 20 administrations is the same.

In an embodiment, each subsequent administration is administered 12-16, e.g., 14, days after the previous administration.

In an embodiment, at least 2, 3, 4, 5, 6, 7, 8, 9, 10, 12, 15, 20, 50 or 100 administrations are administered to said subject.

In an embodiment, the drug is provided at 12-17 mg/m² /administration, e.g., 12 - 15 mg/m² /administration.

In an embodiment, the drug is provided at 18-60 mg/m² /month, e.g., 18 -30 mg/m² /month, 24-30 mg/m2/month or 36-60 mg/m²/month.

In an embodiment, the conjugate includes a topoisomerase I inhibitor and/or a topoisomerase II inhibitor. In an embodiment, the conjugate includes a topoisomerase I inhibitor or combination of topoisomerase I inhibitors, e.g., camptothecin, irinotecan, SN-38, topotecan, lamellarin D and derivatives thereof. In an embodiment, the conjugate includes a topoisomerase II inhibitor or a combination of topoisomerase II inhibitors, e.g., eptoposide, tenoposide, doxorubicin and derivatives thereof. In one embodiment, the conjugate includes a combination of one or more topoisomerase I inhibitors and one or more topoisomerase II inhibitors. In an embodiment, the CDP-topoisomerase inhibitor conjugate is a CDP-camptothecin or camptothecin derivate conjugate, e.g., a CDP-camptothecin or camptothecin derivative conjugate described herein, e.g., CRLX101.

In one embodiment, the CDP-topoisomerase inhibitor conjugate, particle or composition, e.g., a CDP-camptothecin or camptothecin derivative, a CDP-camptothecin or camptothecin derivative conjugate, particle or composition described herein, e.g., CRLX101, is administered by intravenous administration over a period equal to or less than about 30 minutes, 45 minutes, 60 minutes, 90 minutes, 120 minutes, 150 minutes, or 180 minutes. In one embodiment, the CDP-topoisomerase inhibitor conjugate, particle or composition, e.g., a CDP-camptothecin or camptothecin derivative conjugate, particle or composition, e.g., the CDP-camptothecin or camptothecin derivative conjugate, particle or composition described herein, e.g. CRLX101, is administered at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m² by intravenous administration over a period equal to or less than about 30 minutes, 45 minutes, 60 minutes or 90 minutes, e.g., a period equal to or less than 30 minutes, 45 minutes or 60 minutes.

In one embodiment, the CDP-topoisomerase inhibitor conjugate, particle or composition, e.g., a CDP-camptothecin or camptothecin derivative, a CDP-camptothecin or camptothecin derivative conjugate, particle or composition described herein, e.g., CRLX101, is administered by intravenous administration over a period of about 12 hours, 15 hours, 18 hours, 21 hours, 24 hours, 27 hours, or 30 hours. In one embodiment, the CDP-topoisomerase inhibitor conjugate, particle or composition, e.g., a CDP-camptothecin or camptothecin derivative conjugate, particle or composition, e.g., the CDP-camptothecin or camptothecin derivative conjugate, particle or composition described herein, e.g. CRLX101, is administered at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m² by intravenous administration over a period of about 12 hours, 15 hours, 18 hours, 21 hours, 24 hours, 27 hours, or 30 hours. Preferably, the CDP-topoisomerase inhibitor conjugate, particle or composition, e.g., a CDP-camptothecin or camptothecin derivative conjugate, particle or composition, e.g., the CDP-camptothecin or camptothecin derivative conjugate, particle or composition described herein, e.g. CRLX101, is administered at a dosage of 15 mg/m², 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m² by intravenous administration over a period of about 12 hours, 15 hours, 18 hours, 21 hours, 24 hours, 27 hours, or 30 hours.

In one embodiment, the CDP-topoisomerase inhibitor conjugate, particle or composition, e.g., a CDP-camptothecin or camptothecin derivative conjugate, particle or composition, e.g., the CDP-camptothecin or camptothecin derivative conjugate, particle or composition described herein, e.g. CRLX101, is administered at a dosage of 6 mg/m², 7 mg/m², 8 mg/m², 9 mg/m², 10 mg/m², 11 mg/m², 12 mg/m², 13 mg/m², or 14 mg/m² twice a day, and optionally, one or more subsequent administrations of said CDP-topoisomerase inhibitor conjugate, particle or composition, e.g., a CDP-camptothecin conjugate, particle or composition or camptothecin derivative conjugate, particle or composition, e.g., a CDP-camptothecin conjugate, particle or composition or camptothecin derivative conjugate, particle or composition described herein, e.g., CRLX101, is given at a dosage of 6 mg/m², 7 mg/m², 8 mg/m², 9 mg/m², 10 mg/m², 11 mg/m², 12 mg/m², 13 mg/m², or 14 mg/m² twice a day, wherein each subsequent administration is provided, independently, between 9, 10, 11, 12, 13, 14, 15 or 16 days after the previous, e.g., the initial, administration, to thereby treat the proliferative disorder. In one embodiment, the second daily dose is given 4, 5, 6, 7, 8, 9, 10, 12, 13, 14, 15, 16, 17, 18, 19, 20 hours after the initial daily dose.

In an embodiment, the method includes an initial administration of CRLX101 to said subject at a dosage of 12 mg/m², 13 mg/m², 14 mg/m², 15 mg/m², 16 mg/m², or 17 mg/m² and
one or more subsequent administrations of CRLX101 to said subject, at a dosage of 12 mg/m², 13 mg/m², 14 mg/m², 15 mg/m², 16 mg/m², or 17 mg/m², e.g., at the same dosage as the initial dosage, wherein each subsequent administration is administered, independently, 12-16, e.g., 14, days after the previous, e.g., the initial, administration, and the cancer is, e.g., lung cancer, e.g., non-small cell lung cancer and/or small cell lung cancer (e.g., squamous cell non-small cell lung cancer or squamous cell small cell lung cancer). In one embodiment, the lung cancer is refractory, relapsed or resistant to a platinum based agent (e.g., carboplatin, cisplatin, oxaliplatin) and/or a taxane (e.g., docetaxel, paclitaxel, larotaxel or cabazitaxel).

In an embodiment, the method includes an initial administration of CRLX101 to said subject at a dosage of 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m², and
one or more subsequent administrations of CRLX101 to said subject, at a dosage of 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m , e.g., at the same dosage as the initial dosage, wherein each subsequent administration is administered, independently, 12-16, e.g., 14, days after the previous, e.g., the initial, administration, and the cancer is, e.g., lung cancer, e.g., non-small cell lung cancer and/or small cell lung cancer (e.g., squamous cell non-small cell lung cancer or squamous cell small cell lung cancer). In one embodiment, the lung cancer is refractory, relapsed or resistant to a platinum based agent (e.g., carboplatin, cisplatin, oxaliplatin) and/or a taxane (e.g., docetaxel, paclitaxel, larotaxel or cabazitaxel).

In an embodiment, the method includes an initial administration of CRLX101 to said subject at a dosage of 12 mg/m², 13 mg/m², 14 mg/m², 15 mg/m², 16 mg/m², or 17 mg/m², and
one or more subsequent administrations of CRLX101 to said subject, at a dosage of 12 mg/m², 13 mg/m², 14 mg/m², 15 mg/m², 16 mg/m², or 17 mg/m², e.g., at the same dosage as the initial dosage, wherein each subsequent administration is administered, independently, 12-16, e.g., 14, days after the previous, e.g., the initial, administration, and the cancer is, e.g., ovarian cancer. In one embodiment, the ovarian cancer is refractory, relapsed or resistant to a platinum-based agent (e.g., carboplatin, cisplatin, oxaliplatin). In one embodiment, the CRLX101 is administered by intraperitoneal administration.

In an embodiment, the method includes an initial administration of CRLX101 to said subject at a dosage of 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m², and
one or more subsequent administrations of CRLX101 to said subject, at a dosage of 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m², e.g., at the same dosage as the initial dosage, wherein each subsequent administration is administered, independently, 12-16, e.g., 14, days after the previous, e.g., the initial, administration, and the cancer is, e.g., ovarian cancer. In one embodiment, the ovarian cancer is refractory, relapsed or resistant to a platinum-based agent (e.g., carboplatin, cisplatin, oxaliplatin). In one embodiment, the CRLX101 is administered by intraperitoneal administration.

In an embodiment, the method includes an initial administration of CRLX101 to said subject at a dosage of 12 mg/m², 13 mg/m², 14 mg/m², 15 mg/m², 16 mg/m², or 17 mg/m², and
one or more subsequent administrations of CRLX101 to said subject, at a dosage of 12 mg/m², 13 mg/m², 14 mg/m², 15 mg/m², 16 mg/m², or 17 mg/m², e.g., at the same dosage as the initial dosage, wherein each subsequent administration is administered, independently, 12-16, e.g., 14, days after the previous, e.g., the initial, administration, and the cancer is, e.g., gastric cancer, e.g., gastroesophageal, upper gastric or lower gastric cancer.

In an embodiment, the method includes an initial administration of CRLX101 to said subject at a dosage of 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m², and
one or more subsequent administrations of CRLX101 to said subject, at a dosage of 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m², e.g., at the same dosage as the initial dosage, wherein each subsequent administration is administered, independently, 12-16, e.g., 14, days after the previous, e.g., the initial, administration, and the cancer is, e.g., gastric cancer, e.g., gastroesophageal, upper gastric or lower gastric cancer.

In an embodiment, the method includes an initial administration of CRLX101 to said subject at a dosage of 12 mg/m², 13 mg/m², 14 mg/m² or 15 mg/m², and
one or more subsequent administrations of CRLX101 to said subject, at a dosage of 12 mg/m², 13 mg/m², 14 mg/m² or 15 mg/m², e.g., at the same dosage as the initial dosage, wherein each subsequent administration is administered, independently, 12-16, e.g., 14, days after the previous, e.g., the initial, administration, and the cancer is, e.g., pancreatic cancer.

In an embodiment, the method includes an initial administration of CRLX101 to said subject at a dosage of 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m², and
one or more subsequent administrations of CRLX101 to said subject, at a dosage of 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m , e.g., at the same dosage as the initial dosage, wherein each subsequent administration is administered, independently, 12-16, e.g., 14, days after the previous, e.g., the initial, administration, and the cancer is, e.g., pancreatic cancer.

In an embodiment, the method includes an initial administration of CRLX101 to said subject at a dosage of 12 mg/m², 13 mg/m², 14 mg/m², 15 mg/m², 16 mg/m², or 17 mg/m², and
one or more subsequent administrations of CRLX101 to said subject, at a dosage of 12 mg/m², 13 mg/m², 14 mg/m², 15 mg/m², 16 mg/m², or 17 mg/m², e.g., at the same dosage as the initial dosage, wherein each subsequent administration is administered, independently, 12-16, e.g., 14, days after the previous, e.g., the initial, administration, and the cancer is, e.g., colorectal cancer.

In an embodiment, the method includes an initial administration of CRLX101 to said subject at a dosage of 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m², and
one or more subsequent administrations of CRLX101 to said subject, at a dosage of 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m², e.g., at the same dosage as the initial dosage, wherein each subsequent administration is administered, independently, 12-16, e.g., 14, days after the previous, e.g., the initial, administration, and the cancer is, e.g., colorectal cancer.

In an embodiment, the method includes an initial administration of CRLX101 to said subject at a dosage of 12 mg/m², 13 mg/m², 14 mg/m², 15 mg/m², 16 mg/m², or 17 mg/m², and
one or more subsequent administrations of CRLX101 to said subject, at a dosage of 12 mg/m², 13 mg/m², 14 mg/m², 15 mg/m², 16 mg/m², or 17 mg/m², e.g., at the same dosage as the initial dosage, wherein each subsequent administration is administered, independently, 12-16, e.g., 14, days after the previous, e.g., the initial, administration, and the cancer is, e.g., breast cancer, e.g., estrogen receptor positive breast cancer, estrogen receptor negative breast cancer, HER-2 positive breast cancer, HER-2 negative breast cancer, triple negative breast cancer or inflammatory breast cancer.

In an embodiment, the method includes an initial administration of CRLX101 to said subject at a dosage of 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m², and
one or more subsequent administrations of CRLX101 to said subject, at a dosage of 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m², e.g., at the same dosage as the initial dosage, wherein each subsequent administration is administered, independently, 12-16, e.g., 14, days after the previous, e.g., the initial, administration, and the cancer is, e.g., breast cancer, e.g., estrogen receptor positive breast cancer, estrogen receptor negative breast cancer, HER-2 positive breast cancer, HER-2 negative breast cancer, triple negative breast cancer or inflammatory breast cancer.

In an embodiment, the cancer is a cancer described herein. For example, the cancer can be a cancer of the bladder (including accelerated and metastatic bladder cancer), breast (e.g., estrogen receptor positive breast cancer, estrogen receptor negative breast cancer, HER-2 positive breast cancer, HER-2 negative breast cancer, triple negative breast cancer, inflammatory breast cancer), colon (including colorectal cancer), kidney, liver, lung (including small cell lung cancer and non-small cell lung cancer (including adenocarcinoma, squamous cell carcinoma, bronchoalveolar carcinoma and large cell carcinoma)), genitourinary tract, e.g., ovary (including fallopian, endometrial and peritoneal cancers), cervix, prostate and testes, lymphatic system, rectum, larynx, pancreas (including exocrine pancreatic carcinoma), stomach (e.g., gastroesophageal, upper gastric or lower gastric cancer), gastrointestinal cancer (e.g., anal cancer), gall bladder, thyroid, lymphoma (e.g., Burkitt's, Hodgkin's or non-Hodgkin's lymphoma), leukemia (e.g., acute myeloid leukemia), Ewing's sarcoma, nasoesophageal cancer, nasopharyngeal cancer, neural and glial cell cancers (e.g., glioblastoma multiforme), and head and neck. Preferred cancers include breast cancer (e.g., metastatic or locally advanced breast cancer), prostate cancer (e.g., hormone refractory prostate cancer), renal cell carcinoma, lung cancer (e.g., small cell lung cancer and non-small cell lung cancer (including adenocarcinoma, squamous cell carcinoma, bronchoalveolar carcinoma and large cell carcinoma)), pancreatic cancer, gastric cancer (e.g., gastroesophageal, upper gastric or lower gastric cancer), colorectal cancer, squamous cell cancer of the head and neck, ovarian cancer (e.g., advanced ovarian cancer, platinum-based agent resistant or relapsed ovarian cancer), lymphoma (e.g., Burkitt's, Hodgkin's or non-Hodgkin's lymphoma), leukemia (e.g., acute myeloid leukemia) and gastrointestinal cancer.

In an embodiment, the cancer is ovarian cancer, colorectal, breast, lung, lymphoma or gastric cancer. In an embodiment, the cancer is a cancer other than pancreatic cancer, renal cell carcinoma and/or lung cancer (e.g., non-small cell lung cancer and/or small cell lung cancer). In an embodiment, the cancer is a cancer other than pancreatic cancer, renal cell carcinoma, lung cancer (e.g., non-small cell lung cancer and/or small cell lung cancer) and/or ovarian cancer.

In one embodiment, the subject has not been administered a CDP-topoisomerase inhibitor conjugate, particle or composition, e.g., a CDP-camptothecin or camptothecin derivative conjugate, particle or composition, a CDP-camptothecin or camptothecin derivative conjugate, particle or composition described herein, e.g., CRLX101, prior to the initial administration.

In an embodiment, the CDP-topoisomerase inhibitor conjugate, particle or composition is administered as a first line treatment for the cancer.

In an embodiment, the CDP-topoisomerase inhibitor conjugate, particle or composition is administered as a second, third or fourth line treatment for the cancer. In an embodiment, the cancer is sensitive to one or more chemotherapeutic agents, e.g., a platinum based agent, a taxane, an alkylating agent, an anthracycline (e.g., doxorubicin (e.g., liposomal doxorubicin)), an antimetabolite and/or a vinca alkaloid. In an embodiment, the cancer is a refractory, relapsed or resistant to one or more chemotherapeutic agents, e.g., a platinum based agent, a taxane, an alkylating agent, an antimetabolite and/or a vinca alkaloid. In one embodiment, the cancer is, e.g., ovarian cancer, and the ovarian cancer is refractory, relapsed or resistant to a platinum based agent (e.g., carboplatin, cisplatin, oxaliplatin), a taxane (e.g., paclitaxel, docetaxel, larotaxel, cabazitaxel) and/or an anthracycline (e.g., doxorubicin (e.g., liposomal doxorubicin)). In one embodiment, the cancer is, e.g., colorectal cancer, and the cancer is refractory, relapsed or resistant to an antimetabolite (e.g., an antifolate (e.g., pemetrexed, floxuridine, raltitrexed) and a pyrimidine analogue (e.g., capecitabine, cytrarabine, gemcitabine, 5FU)) and/or a platinum based agent (e.g., carboplatin, cisplatin, oxaliplatin). In one embodiment, the cancer is, e.g., lung cancer, and the cancer is refractory, relapsed or resistant to a taxane (e.g., paclitaxel, docetaxel, larotaxel, cabazitaxel), a platinum based agent (e.g., carboplatin, cisplatin, oxaliplatin), a vinca alkaloid (e.g., vinblastine, vincristine, vindesine, vinorelbine), a vascular endothelial growth factor (VEGF) pathway inhibitor, an epidermal growth factor (EGF) pathway inhibitor and/or an antimetabolite (e.g., an antifolate (e.g., pemetrexed, floxuridine, raltitrexed) and a pyrimidine analogue (e.g., capecitabine, cytrarabine, gemcitabine, 5FU)). In one embodiment, the cancer is, e.g., breast cancer, and the cancer is refractory, relapsed or resistant to a taxane (e.g., paclitaxel, docetaxel, larotaxel, cabazitaxel), a vascular endothelial growth factor (VEGF) pathway inhibitor, an anthracycline (e.g., daunorubicin, doxorubicin (e.g., liposomal doxorubicin), epirubicin, valrubicin, idarubicin), a platinum-based agent (e.g., carboplatin, cisplatin, oxaliplatin), and/or an antimetabolite (e.g., an antifolate (e.g., pemetrexed, floxuridine, raltitrexed) and a pyrimidine analogue (e.g., capecitabine, cytrarabine, gemcitabine, 5FU)). In one embodiment, the cancer is, e.g., gastric cancer, and the cancer is refractory, relapsed or resistant to an antimetabolite (e.g., an antifolate (e.g., pemetrexed, floxuridine, raltitrexed) and a pyrimidine analogue (e.g., capecitabine, cytrarabine, gemcitabine, 5FU)) and/or a platinum-based agent (e.g., carboplatin, cisplatin, oxaliplatin).

In one embodiment, the subject has ovarian cancer that is refractory, relapsed or resistant to a platinum-based agent, and the subject is administered a CDP-topoisomerase inhibitor conjugate, particle or composition, e.g., a CDP-topoisomerase inhibitor conjugate, particle or composition described herein. In one embodiment, the CDP-topoisomerase inhibitor conjugate, particle or composition is administered in combination with doxorubicin (e.g., liposomal doxorubicin). In one embodiment, the doxorubicin (e.g., the liposomal doxorubicin) is administered at a dose of about 20 mg/m², about 30 mg/m² or about 40 mg/m², every 24, 25, 26, 27, 28, 29, 30 or 31 days, e.g., 28 days. In one embodiment, when the CDP-topoisomerase inhibitor conjugate, particle or composition is administered in combination with doxorubicin (e.g., liposomal doxorubicin), the dose at which the CDP-topoisomerase inhibitor conjugate, particle or composition is administered is 1%, 3%, 5%, 10%, 15%, 20%, 25%, 30% less than a dose described herein.

In an embodiment, the cancer has been sensitized to a topoisomerase inhibitor, e.g., the subject has received radiation and/or the subject has received a phosphatase inhibitor (e.g., okadiac acid) prior to the administration of the CDP-topoisomerase inhibitor conjugate, particle or composition. In one embodiment, the cancer is sensitized to topoisomerase inhibitors, e.g., the subject receives radiation in combination with the administration of the CDP-topoisomerase inhibitor conjugate, particle or composition and/or the subject is administered a phosphatase inhibitor (e.g., okadiac acid) in combination with the administration of the CDP-topoisomerase inhibitor conjugate, particle or composition. In one embodiment, the cancer is sensitized or has been sensitized to topoisomerase inhibitors and the cancer is a glial cell cancer (e.g., glioblastoma multiforme) or head and neck cancer.

In one embodiment, the conjugate, particle or composition is administered in combination with one or more additional chemotherapeutic agent, e.g., a chemotherapeutic agent (such as an angiogenesis inhibitor) or combination of chemotherapeutic agents described herein. In one embodiment, the conjugate, particle or composition is administered in combination with one or more of: a platinum-based agent (e.g., carboplatin, cisplatin, oxaliplatin), a taxane (e.g., paclitaxel, docetaxel, larotaxel, cabazitaxel), a vinca alkaloid (e.g., vinblastine, vincristine, vindesine, vinorelbine), an antimetabolite (e.g., an antifolate (e.g., pemetrexed, floxuridine, raltitrexed) and a pyrimidine analogue (e.g., 5FU, capecitabine, cytrarabine, gemcitabine)), an alkylating agent (e.g., cyclophosphamide, decarbazine, melphalan, ifosfamide, temozolomide), a vascular endothelial growth factor (VEGF) pathway inhibitor, a poly ADP-ribose polymerase (PARP) inhibitor and an mTOR inhibitor. In one embodiment, when the CDP-topoisomerase inhibitor conjugate, particle or composition is administered in combination with an additional chemotherapeutic agent, the dose at which the CDP-topoisomerase inhibitor conjugate, particle or composition is administered is 1%, 3%, 5%, 10%, 15%, 20%, 25%, 30% less than a dose described herein.

In an embodiment, the method further comprises administering to said subject a treatment that reduces one or more side effect associated with administration of a CDP-camptothecin or camptothecin derivative conjugate, particle or composition, e.g., a treatment described herein.

In one aspect, the disclosure features, a method of treating a proliferative disorder, e.g., a cancer, in a subject, e.g., a human subject. The method comprises:
providing an initial administration of a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, to said subject at a dosage of 9 mg/m², 10 mg/m², 11 mg/m², 12 mg/m², 13 mg/m², 14 mg/m², 15 mg/m², 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m², 30 mg/m², 31 mg/m², 32 mg/m², 33 mg/m², 34 mg/m², 35 mg/m² or 36 mg/m² (wherein said dosage is expressed in mg of drug, as opposed to mg of conjugate) and
optionally, providing one or more subsequent administrations of said CDP-topoisomerase inhibitor conjugate, particle or composition , e.g., a CDP-camptothecin or camptothecin derivative conjugate, particle or composition, e.g., a CDP-camptothecin or camptothecin derivative conjugate, particle or composition described herein, e.g., CRLX101, at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m², 30 mg/m², 31 mg/m², 32 mg/m², 33 mg/m², 34 mg/m², 35 mg/m² or 36 mg/m², wherein each subsequent administration is provided, independently, between 17, 18, 19, 20, 21, 22, 23, 24, 25, 26, 27, 28, 29, 30, or 31 days after the previous, e.g., the initial, administration, to thereby treat the proliferative disorder.

In an embodiment, the dosage of at least 2, 3, 4, 5, 6, 7, 8, 9, 10, 12, 15 or 20 administrations are the same.

In an embodiment, the time between at least 2, 3, 4, 5, 6, 7, 8, 9, 10, 12 15, or 20 administrations is the same.

In an embodiment, each subsequent administration is administered 19-23, e.g., 21, or 25-29, e.g., 27 or 28 days after the previous administration.

In an embodiment, at least 2, 3, 4, 5, 6, 7, 8, 9, 10, 12, 15, 20, 50 or 100 administrations are administered to said subject.

In an embodiment, the drug is provided at 18 -60 mg/m² /month, e.g., 18 -30 mg/m² /month or 36-60 mg/m² /month. In one embodiment, when the drug is provided in combination with one or more additional chemotherapeutic agent, e.g., a chemotherapeutic agent described herein, the drug is provided at 6-12 mg/m²/month.

In an embodiment, the conjugate includes a topoisomerase I inhibitor and/or a topoisomerase II inhibitor. In an embodiment, the conjugate includes a topoisomerase I inhibitor or combination of topoisomerase I inhibitors, e.g., camptothecin, irinotecan, SN-38, topotecan, lamellarin D and derivatives thereof. In an embodiment, the conjugate includes a topoisomerase II inhibitor or a combination of topoisomerase II inhibitors, e.g., etoposide, tenoposide, doxorubicin and derivatives thereof. In one embodiment, the conjugate includes a combination of one or more topoisomerase I inhibitors and one or more topoisomerase II inhibitors. In an embodiment, the CDP-topoisomerase inhibitor conjugate is a CDP-camptothecin or camptothecin derivate conjugate, e.g., a CDP-camptothecin or camptothecin derivative conjugate described herein, e.g., CRLX101.

In one embodiment, the CDP-topoisomerase inhibitor conjugate, particle or composition, e.g., a CDP-camptothecin or camptothecin derivative, a CDP-camptothecin or camptothecin derivative conjugate, particle or composition described herein, e.g., CRLX101, is administered by intravenous administration over a period equal to or less than about 30 minutes, 45 minutes, 60 minutes, 90 minutes, 120 minutes, 150 minutes, or 180 minutes. In one embodiment, the CDP-topoisomerase inhibitor conjugate, particle or composition, e.g., a CDP-camptothecin or camptothecin derivative conjugate, particle or composition, e.g., the CDP-camptothecin or camptothecin derivative conjugate, particle or composition described herein, e.g. CRLX101, is administered at a dosage of 9 mg/m², 10 mg/m², 11 mg/m², 12 mg/m², 13 mg/m², 14 mg/m², 15 mg/m², 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m², 30 mg/m², 31 mg/m², 32 mg/m², 33 mg/m², 34 mg/m², 35 mg/m² or 36 mg/m² by intravenous administration over a period equal to or less than about 30 minutes, 45 minutes, 60 minutes or 90 minutes, e.g., a period equal to or less than 30 minutes, 45 minutes or 60 minutes.

In one embodiment, the CDP-topoisomerase inhibitor conjugate, particle or composition, e.g., a CDP-camptothecin or camptothecin derivative, a CDP-camptothecin or camptothecin derivative conjugate, particle or composition described herein, e.g., CRLX101, is administered by intravenous administration over a period of about 12 hours, 15 hours, 18 hours, 21 hours, 24 hours, 27 hours or 30 hours. In one embodiment, the CDP-topoisomerase inhibitor conjugate, particle or composition, e.g., a CDP-camptothecin or camptothecin derivative conjugate, particle or composition, e.g., the CDP-camptothecin or camptothecin derivative conjugate, particle or composition described herein, e.g. CRLX101, is administered at a dosage of 15 mg/m², 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m², 30 mg/m², 31 mg/m², 32 mg/m², 33 mg/m², 34 mg/m², 35 mg/m² or 36 mg/m² by intravenous administration over a period of about 12 hours, 15 hours, 18 hours, 21 hours, 24 hours, 27 hours or 30 hours.

In one embodiment, the CDP-topoisomerase inhibitor conjugate, particle or composition, e.g., a CDP-camptothecin or camptothecin derivative conjugate, particle or composition, e.g., the CDP-camptothecin or camptothecin derivative conjugate, particle or composition described herein, e.g. CRLX101, is administered at a dosage of 6 mg/m², 7 mg/m², 8 mg/m², 9 mg/m², 10 mg/m², 11 mg/m², 12 mg/m², 13 mg/m², or 14 mg/m² twice a day, and optionally, one or more subsequent administrations of said CDP-topoisomerase inhibitor conjugate, particle or composition, e.g., a CDP-camptothecin conjugate, particle or composition or camptothecin derivative conjugate, particle or composition, e.g., a CDP-camptothecin conjugate, particle or composition or camptothecin derivative conjugate, particle or composition described herein, e.g., CRLX101, is given at a dosage of 6 mg/m², 7 mg/m², 8 mg/m², 9 mg/m², 10 mg/m², 11 mg/m², 12 mg/m², 13 mg/m², or 14 mg/m² twice a day, wherein each subsequent administration is provided, independently, between 17, 18, 19, 20, 21, 22, 23, 24, 25, 26, 27, 28, 29, 20 or 31 days after the previous, e.g., the initial, administration, to thereby treat the proliferative disorder. In one embodiment, the second daily dose is given 4, 5, 6, 7, 8, 9, 10, 12, 13, 14, 15, 16, 17, 18, 19, 20 hours after the initial daily dose.

In an embodiment, the method includes an initial administration of CRLX101 to said subject at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² or 18 mg/m², and
one or more subsequent administrations of CRLX101 to said subject, at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² or 18 mg/m², e.g., at the same dosage as the initial dosage, wherein each subsequent administration is administered, independently, 19-22, e.g., 21, days after the previous, e.g., the initial, administration, and the cancer is, e.g., lung cancer, e.g., non-small cell lung cancer and/or small cell lung cancer (e.g., squamous cell non-small cell lung cancer or squamous cell small cell lung cancer). In one embodiment, the lung cancer is refractory, relapsed or resistant to a platinum based agent (e.g., carboplatin, cisplatin, oxaliplatin) and/or a taxane (docetaxel, paclitaxel, larotaxel or cabazitaxel).

In an embodiment, the method includes an initial administration of CRLX101 to said subject at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² or 18 mg/m², and
one or more subsequent administrations of CRLX101 to said subject, at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² or 18 mg/m², e.g., at the same dosage as the initial dosage, wherein each subsequent administration is administered, independently, 19-22, e.g., 21, days after the previous, e.g., the initial, administration, and the cancer is, e.g., ovarian cancer. In one embodiment, the ovarian cancer is refractory, relapsed or resistant to a platinum-based agent (e.g., carboplatin, cisplatin, oxaliplatin). In one embodiment, the CRLX101 is administered by intraperitoneal administration.

In an embodiment, the method includes an initial administration of CRLX101 to said subject at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² or 18 mg/m², and
one or more subsequent administrations of CRLX101 to said subject, at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² or 18 mg/m², e.g., at the same dosage as the initial dosage, wherein each subsequent administration is administered, independently, 19-22, e.g., 21, days after the previous, e.g., the initial, administration, and the cancer is, e.g., gastric cancer, e.g., gastroesophageal, upper gastric or lower gastric cancer.

In an embodiment, the method includes an initial administration of CRLX101 to said subject at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² or 18 mg/m2, and
one or more subsequent administrations of CRLX101 to said subject, at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² or 18 mg/m², e.g., at the same dosage as the initial dosage, wherein each subsequent administration is administered, independently, 19-22, e.g., 21, days after the previous, e.g., the initial, administration, and the cancer is, e.g., pancreatic cancer.

In an embodiment, the method includes an initial administration of CRLX101 to said subject at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² or 18 mg/m², and
one or more subsequent administrations of CRLX101 to said subject, at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² or 18 mg/m², e.g., at the same dosage as the initial dosage, wherein each subsequent administration is administered, independently, 19-22, e.g., 21, days after the previous, e.g., the initial, administration, and the cancer is, e.g., colorectal cancer.

In an embodiment, the method includes an initial administration of CRLX101 to said subject at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² or 18 mg/m², and
one or more subsequent administrations of CRLX101 to said subject, at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m² or 18 mg/m², e.g., at the same dosage as the initial dosage, wherein each subsequent administration is administered, independently, 19-22, e.g., 21, days after the previous, e.g., the initial, administration, and the cancer is, e.g., breast cancer, e.g., estrogen receptor positive breast cancer, estrogen receptor negative breast cancer, HER-2 positive breast cancer, HER-2 negative breast cancer, triple negative breast cancer or inflammatory breast cancer.

In an embodiment, the cancer is a cancer described herein. For example, the cancer can be a cancer of the bladder (including accelerated and metastatic bladder cancer), breast (e.g., estrogen receptor positive breast cancer, estrogen receptor negative breast cancer, HER-2 positive breast cancer, HER-2 negative breast cancer, triple negative breast cancer, inflammatory breast cancer), colon (including colorectal cancer), kidney, liver, lung (including small cell lung cancer and non-small cell lung cancer (including adenocarcinoma, squamous cell carcinoma, bronchoalveolar carcinoma and large cell carcinoma)), genitourinary tract, e.g., ovary (including fallopian, endometrial and peritoneal cancers), cervix, prostate and testes, lymphatic system, rectum, larynx, pancreas (including exocrine pancreatic carcinoma), stomach (e.g., gastroesophageal, upper gastric or lower gastric cancer), gastrointestinal cancer (e.g., anal cancer), gall bladder, thyroid, lymphoma (e.g., Burkitt's, Hodgkin's or non-Hodgkin's lymphoma), leukemia (e.g., acute myeloid leukemia), Ewing's sarcoma, nasoesophageal cancer, nasopharyngeal cancer, neural and glial cell cancers (e.g., glioblastoma multiforme), and head and neck. Preferred cancers include breast cancer (e.g., metastatic or locally advanced breast cancer), prostate cancer (e.g., hormone refractory prostate cancer), renal cell carcinoma, lung cancer (e.g., small cell lung cancer and non-small cell lung cancer (including adenocarcinoma, squamous cell carcinoma, bronchoalveolar carcinoma and large cell carcinoma)), pancreatic cancer, gastric cancer (e.g., gastroesophageal, upper gastric or lower gastric cancer), colorectal cancer, squamous cell cancer of the head and neck, ovarian cancer (e.g., advanced ovarian cancer, platinum-based agent resistant or relapsed ovarian cancer), lymphoma (e.g., Burkitt's, Hodgkin's or non-Hodgkin's lymphoma), leukemia (e.g., acute myeloid leukemia) and gastrointestinal cancer.

In an embodiment, the cancer is ovarian cancer, colorectal, breast, lung, lymphoma or gastric cancer. In an embodiment, the cancer is a cancer other than pancreatic cancer, renal cell carcinoma and/or lung cancer (e.g., non-small cell lung cancer). In an embodiment, the cancer is a cancer other than pancreatic cancer, renal cell carcinoma, lung cancer (e.g., non-small cell lung cancer) and/or ovarian cancer.

In one embodiment, the subject has not been administered a CDP-topoisomerase inhibitor conjugate, particle or composition, e.g., a CDP-camptothecin or camptothecin derivative conjugate, particle or composition, a CDP-camptothecin or camptothecin derivative conjugate, particle or composition described herein, e.g., CRLX101, prior to the initial administration.

In an embodiment, the CDP-topoisomerase inhibitor conjugate, particle or composition is administered as a first line treatment for the cancer.

In an embodiment, the CDP-topoisomerase inhibitor conjugate, particle or composition is administered as a second, third or fourth line treatment for the cancer. In an embodiment, the cancer is sensitive to one or more chemotherapeutic agents, e.g., a platinum-based agent, a taxane, an alkylating agent, an antimetabolite and/or a vinca alkaloid. In an embodiment, the cancer is a refractory, relapsed or resistant to one or more chemotherapeutic agents, e.g., a platinum-based agent, a taxane, an alkylating agent, an anthracycline (e.g., doxorubicin (e.g., liposomal doxorubicin)), an antimetabolite and/or a vinca alkaloid. In one embodiment, the cancer is, e.g., ovarian cancer, and the ovarian cancer is refractory, relapsed or resistant to a platinum-based agent (e.g., carboplatin, cisplatin, oxaliplatin), a taxane (e.g., paclitaxel, docetaxel, larotaxel, cabazitaxel) and/or an anthracycline (e.g., doxorubicin (e.g., liposomal doxorubicin)). In one embodiment, the cancer is, e.g., colorectal cancer, and the cancer is refractory, relapsed or resistant to an antimetabolite (e.g., an antifolate (e.g., pemetrexed, floxuridine, raltitrexed) and a pyrimidine analogue (e.g., capecitabine, cytrarabine, gemcitabine, 5FU)) and/or a platinum-based agent (e.g., carboplatin, cisplatin, oxaliplatin). In one embodiment, the cancer is, e.g., lung cancer, and the cancer is refractory, relapsed or resistant to a taxane (e.g., paclitaxel, docetaxel, larotaxel, cabazitaxel), a platinum-based agent (e.g., carboplatin, cisplatin, oxaliplatin), a vinca alkaloid (e.g., vinblastine, vincristine, vindesine, vinorelbine), a vascular endothelial growth factor (VEGF) pathway inhibitor, an epidermal growth factor (EGF) pathway inhibitor) and/or an antimetabolite (e.g., an antifolate (e.g., pemetrexed, floxuridine, raltitrexed) and a pyrimidine analogue (e.g., capecitabine, cytrarabine, gemcitabine, 5FU)). In one embodiment, the cancer is, e.g., breast cancer, and the cancer is refractory, relapsed or resistant to a taxane (e.g., paclitaxel, docetaxel, larotaxel, cabazitaxel), a vascular endothelial growth factor (VEGF) pathway inhibitor, an anthracycline (e.g., daunorubicin, doxorubicin (e.g., liposomal doxorubicin), epirubicin, valrubicin, idarubicin), a platinum-based agent (e.g., carboplatin, cisplatin, oxaliplatin), and/or an antimetabolite (e.g., an antifolate (e.g., pemetrexed, floxuridine, raltitrexed) and a pyrimidine analogue (e.g., capecitabine, cytrarabine, gemcitabine, 5FU)). In one embodiment, the cancer is, e.g., gastric cancer, and the cancer is refractory, relapsed or resistant to an antimetabolite (e.g., an antifolate (e.g., pemetrexed, floxuridine, raltitrexed) and a pyrimidine analogue (e.g., capecitabine, cytrarabine, gemcitabine, 5FU)) and/or a platinum-based agent (e.g., carboplatin, cisplatin, oxaliplatin).

In one embodiment, the subject has ovarian cancer that is refractory, relapsed or resistant to a platinum-based agent, and the subject is administered a CDP-topoisomerase inhibitor conjugate, particle or composition, e.g., a CDP-topoisomerase inhibitor conjugate, particle or composition described herein. In one embodiment, the CDP-topoisomerase inhibitor conjugate, particle or composition is administered in combination with doxorubicin (e.g., liposomal doxorubicin). In one embodiment, the doxorubicin (e.g., the liposomal doxorubicin) is administered at a dose of about 20 mg/m², about 30 mg/m² or about 40 mg/m², every 24, 25, 26, 27, 28, 29, 30 or 31 days, e.g., 28 days. In one embodiment, the CDP-topoisomerase inhibitor conjugate, particle or composition, e.g., a CDP-topoisomerase inhibitor conjugate, particle or composition described herein is administered at a dose and/or dosing regimen described herein and the doxorubicin (e.g., the liposomal doxorubicin) is administered at a dose of about 20 mg/m², about 30 mg/m² or about 40 mg/m², every 24, 25, 26, 27, 28, 29, 30 or 31 days, e.g., 28 days. In one embodiment, when the CDP-topoisomerase inhibitor conjugate, particle or composition is administered in combination with doxorubicin (e.g., liposomal doxorubicin), the dose at which the CDP-topoisomerase inhibitor conjugate, particle or composition is administered is 1%, 3%, 5%, 10%, 15%, 20%, 25%, 30% less than a dose described herein.

In an embodiment, the cancer has been sensitized to topoisomerase inhibitors, e.g., the subject has received radiation and/or the subject has received a phosphatase inhibitor (e.g., okadiac acid) prior to the administration of the CDP-topoisomerase inhibitor conjugate, particle or composition. In an embodiment, the cancer is sensitized to topoisomerase inhibitors, e.g., the subject receives radiation in combination with the administration of the CDP-topoisomerase inhibitor conjugate, particle or composition and/or the subject is administered a phosphatase inhibitor (e.g., okadiac acid) in combination with the administration of the CDP-topoisomerase inhibitor conjugate, particle or composition. In one embodiment, the cancer is sensitized or has been sensitized to topoisomerase inhibitors and the cancer is a glial cell cancer (e.g., glioblastoma multiforme) or head and neck cancer.

In one embodiment, the conjugate, particle or composition is administered in combination with one or more additional chemotherapeutic agent, e.g., a chemotherapeutic agent (such as an angiogenesis inhibitor) or combination of chemotherapeutic agents described herein. In one embodiment, the conjugate, particle or composition is administered in combination with one or more of: a platinum based agent (e.g., carboplatin, cisplatin, oxaliplatin), a taxane (e.g., paclitaxel, docetaxel, larotaxel, cabazitaxel), a vinca alkaloid (e.g., vinblastine, vincristine, vindesine, vinorelbine), an antimetabolite (e.g., an antifolate (e.g., floxuridine, premetrexed), a pyrimidine analogue (e.g., 5FU, capecitabine)), an alkylating agent (e.g., cyclophosphamide, decarbazine, melphalan, ifosfamide, temozolomide), a vascular endothelial growth factor (VEGF) pathway inhibitor, a poly ADP-ribose polymerase (PARP) inhibitor and an mTOR inhibitor. In one embodiment, when the CDP-topoisomerase inhibitor conjugate, particle or composition is administered in combination with an additional chemotherapeutic agent, the dose at which the CDP-topoisomerase inhibitor conjugate, particle or composition is administered is 1%, 3%, 5%, 10%, 15%, 20%, 25%, 30% less than the doses described herein.

In one aspect, the disclosure features, a method of treating a proliferative disorder, e.g., a cancer, in a subject. The method comprises:
providing an initial administration of a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, to said subject at a dosage of 3 mg/m², 4 mg/m², 5 mg/m², 6 mg/m², 7 mg/m², 8 mg/m², 9 mg/m², 10 mg/m², or 11 mg/m², (wherein said dosage is expressed in mg of drug, as opposed to mg of conjugate),
optionally, providing one or more subsequent administrations of said CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, at a dosage of 3 mg/m², 4 mg/m², 5 mg/m², 6 mg/m², 7 mg/m², 8 mg/m², 9 mg/m², 10 mg/m², or 11 mg/m², wherein each subsequent administration is provided, independently, between 5, 6, 7, 8, 9 days after the previous, e.g., the initial, administration, to thereby treat the proliferative disorder.

In an embodiment, the dosage of at least 2, 3, 4, 5, 6, 7, 8, 9, 10, 12, 15, or 20 administrations is the same.

In an embodiment, the time between at least 2, 3, 4, 5, 6, 7, 8, 9, 10, 12, 15, or 20 administrations is the same.

In an embodiment, each subsequent administration is administered 5-9, e.g., 7, days after the previous administration. In an embodiment, 3 administrations are given between 5, 6, 7, 8 or 9 days from the previous administration and the fourth administration is given between 10, 11, 12, 13, 14, 15 or 16 days from the previous administration. This dosing schedule can be repeated with 3 additional administrations given between 5, 6, 7, 8 or 9 days from the previous administration and the subsequent administration given between 10, 11, 12, 13, 14, 15 or 16 days from the previous administration.

In an embodiment, at least 2, 3, 4, 5, 6, 7, 8, 9, 10, 12, 15, 20, 50 or 100 administrations are administered to said subject.

In an embodiment, the drug is provided at 9 -33 mg/m² /month.

In an embodiment, the conjugate includes a topoisomerase I inhibitor and/or a topoisomerase II inhibitor. In an embodiment, the conjugate includes a topoisomerase I inhibitor or combination of topoisomerase I inhibitors, e.g., camptothecin, irinotecan, SN-38, topotecan, lamellarin D and derivatives thereof. In an embodiment, the conjugate includes a topoisomerase II inhibitor or a combination of topoisomerase II inhibitors, e.g., eptoposide, tenoposide, doxorubicin and derivatives thereof. In one embodiment, the conjugate includes a combination of one or more topoisomerase I inhibitors and one or more topoisomerase II inhibitors. In an embodiment, the CDP-topoisomerase inhibitor conjugate is a CDP-camptothecin or camptothecin derivate conjugate, e.g., a CDP-camptothecin or camptothecin derivative conjugate described herein, e.g., CRLX101.

In one embodiment, the CDP-topoisomerase inhibitor conjugate, particle or composition, e.g., a CDP-camptothecin or camptothecin derivative, a CDP-camptothecin or camptothecin derivative conjugate, particle or composition described herein, e.g., CRLX101, is administered by intravenous administration over a period equal to or less than about 30 minutes, 45 minutes, 60 minutes, 90 minutes, 120 minutes, 150 minutes, or 180 minutes. In one embodiment, the CDP-topoisomerase inhibitor conjugate, particle or composition, e.g., a CDP-camptothecin or camptothecin derivative conjugate, particle or composition, e.g., the CDP-camptothecin or camptothecin derivative conjugate, particle or composition described herein, e.g. CRLX101, is administered at a dosage of 3 mg/m², 4 mg/m², 5 mg/m², 6 mg/m², 7 mg/m², 8 mg/m², 9 mg/m², 10 mg/m², or 11 mg/m² by intravenous administration over a period equal to or less than about 30 minutes, 45 minutes, 60 minutes or 90 minutes, e.g., a period equal to or less than 30 minutes, 45 minutes or 60 minutes.

In an embodiment, the method includes an initial administration of CRLX101 to said subject at a dosage of 3 mg/m², 4 mg/m², 5 mg/m², 6 mg/m², 7 mg/m², 8 mg/m², 9 mg/m², 10 mg/m², or 11 mg/m², and
one or more subsequent administrations of CRLX101 to said subject, at a dosage of 3 mg/m², 4 mg/m², 5 mg/m², 6 mg/m², 7 mg/m², 8 mg/m², 9 mg/m², 10 mg/m², or 11 mg/m², e.g., at the same dosage as the initial dosage, wherein each subsequent administration is administered, independently, 5-9, e.g., 7, days after the previous, e.g., the initial, administration, and the cancer is, e.g., lung cancer, e.g., non-small cell lung cancer and/or small cell lung cancer (e.g., squamous cell non-small cell lung cancer or squamous cell small cell lung cancer). In one embodiment, the lung cancer is refractory, relapsed or resistant to a platinum based agent (e.g., carboplatin, cisplatin oxaliplatin) and/or a taxane (e.g., docetaxel, paclitaxel, larotaxel, cabazitaxel).

In an embodiment, the method includes an initial administration of CRLX101 to said subject at a dosage of 3 mg/m², 4 mg/m², 5 mg/m², 6 mg/m², 7 mg/m², 8 mg/m², 9 mg/m², 10 mg/m², or 11 mg/m², and
one or more subsequent administrations of CRLX101 to said subject, at a dosage of 3 mg/m², 4 mg/m², 5 mg/m², 6 mg/m², 7 mg/m², 8 mg/m², 9 mg/m², 10 mg/m², or 11 mg/m², e.g., at the same dosage as the initial dosage, wherein each subsequent administration is administered, independently, 5-9, e.g., 7, days after the previous, e.g., the initial, administration, and the cancer is, e.g., ovarian cancer. In one embodiment, the ovarian cancer is refractory, relapsed or resistant to a platinum-based agent (e.g., carboplatin, cisplatin, oxaliplatin). In one embodiment, the CRLX101 is administered by intraperitoneal administration.

In an embodiment, the method includes an initial administration of CRLX101 to said subject at a dosage of 3 mg/m², 4 mg/m², 5 mg/m², 6 mg/m², 7 mg/m², 8 mg/m², 9 mg/m², 10 mg/m², or 11 mg/m², and
one or more subsequent administrations of CRLX101 to said subject, at a dosage of 3 mg/m², 4 mg/m², 5 mg/m², 6 mg/m², 7 mg/m², 8 mg/m², 9 mg/m², 10 mg/m², or 11 mg/m², e.g., at the same dosage as the initial dosage, wherein each subsequent administration is administered, independently, 5-9, e.g., 7, days after the previous, e.g., the initial, administration, and the cancer is, e.g., gastric cancer, e.g., gastroesophageal, upper gastric or lower gastric cancer.

In an embodiment, the method includes an initial administration of CRLX101 to said subject at a dosage of 3 mg/m², 4 mg/m², 5 mg/m², 6 mg/m², 7 mg/m², 8 mg/m², 9 mg/m², 10 mg/m², or 11 mg/m², and
one or more subsequent administrations of CRLX101 to said subject, at a dosage of 3 mg/m², 4 mg/m², 5 mg/m², 6 mg/m², 7 mg/m², 8 mg/m², 9 mg/m², 10 mg/m², or 11 mg/m², e.g., at the same dosage as the initial dosage, wherein each subsequent administration is administered, independently, 5-9, e.g., 7, days after the previous, e.g., the initial, administration, and the cancer is, e.g., pancreatic cancer.

In an embodiment, the method includes an initial administration of CRLX101 to said subject at a dosage of 3 mg/m², 4 mg/m², 5 mg/m², 6 mg/m², 7 mg/m², 8 mg/m², 9 mg/m², 10 mg/m², or 11 mg/m², and
one or more subsequent administrations of CRLX101 to said subject, at a dosage of 3 mg/m², 4 mg/m², 5 mg/m², 6 mg/m², 7 mg/m², 8 mg/m², 9 mg/m², 10 mg/m², or 11 mg/m², e.g., at the same dosage as the initial dosage, wherein each subsequent administration is administered, independently, 5-9, e.g., 7, days after the previous, e.g., the initial, administration, and the cancer is, e.g., colorectal cancer.

In an embodiment, the method includes an initial administration of CRLX101 to said subject at a dosage of 3 mg/m², 4 mg/m², 5 mg/m², 6 mg/m², 7 mg/m², 8 mg/m², 9 mg/m², 10 mg/m², or 11 mg/m², and
one or more subsequent administrations of CRLX101 to said subject, at a dosage of 3 mg/m², 4 mg/m², 5 mg/m², 6 mg/m², 7 mg/m², 8 mg/m², 9 mg/m², 10 mg/m², or 11 mg/m², e.g., at the same dosage as the initial dosage, wherein each subsequent administration is administered, independently, 5-9, e.g., 7, days after the previous, e.g., the initial, administration, and the cancer is, e.g., breast cancer, e.g., estrogen receptor positive breast cancer, estrogen receptor negative breast cancer, HER-2 positive breast cancer, HER-2 negative breast cancer, triple negative breast cancer or inflammatory breast cancer.

In an embodiment, the cancer is a cancer described herein. For example, the cancer can be a cancer of the bladder (including accelerated and metastatic bladder cancer), breast (e.g., estrogen receptor positive breast cancer, estrogen receptor negative breast cancer, HER-2 positive breast cancer, HER-2 negative breast cancer, triple negative breast cancer, inflammatory breast cancer), colon (including colorectal cancer), kidney, liver, lung (including small cell lung cancer and non-small cell lung cancer (including adenocarcinoma, squamous cell carcinoma, bronchoalveolar carcinoma and large cell carcinoma)), genitourinary tract, e.g., ovary (including fallopian, endometrial and peritoneal cancers), cervix, prostate and testes, lymphatic system, rectum, larynx, pancreas (including exocrine pancreatic carcinoma), stomach (e.g., gastroesophageal, upper gastric or lower gastric cancer), gastrointestinal cancer (e.g., anal cancer), gall bladder, thyroid, lymphoma (e.g., Burkitt's, Hodgkin's or non-Hodgkin's lymphoma), leukemia (e.g., acute myeloid leukemia), Ewing's sarcoma, nasoesophageal cancer, nasopharyngeal cancer, neural and glial cell cancers (e.g., glioblastoma multiforme), and head and neck. Preferred cancers include breast cancer (e.g., metastatic or locally advanced breast cancer), prostate cancer (e.g., hormone refractory prostate cancer), renal cell carcinoma, lung cancer (e.g., small cell lung cancer and non-small cell lung cancer (including adenocarcinoma, squamous cell carcinoma, bronchoalveolar carcinoma and large cell carcinoma)), pancreatic cancer, gastric cancer (e.g., gastroesophageal, upper gastric or lower gastric cancer), colorectal cancer, squamous cell cancer of the head and neck, ovarian cancer (e.g., advanced ovarian cancer, platinum-based agent resistant or relapsed ovarian cancer), lymphoma (e.g., Burkitt's, Hodgkin's or non-Hodgkin's lymphoma), leukemia (e.g., acute myeloid leukemia) and gastrointestinal cancer.

In an embodiment, the cancer is ovarian, colorectal, breast, lung, lymphoma or gastric cancer. In an embodiment, the cancer is a cancer other than pancreatic cancer, renal cell carcinoma and/or lung cancer (e.g., non-small cell lung cancer). In an embodiment, the cancer is a cancer other than pancreatic cancer, renal cell carcinoma, lung cancer (e.g., non-small cell lung cancer) and/or ovarian cancer.

In one embodiment, the subject has not been administered a CDP-topoisomerase inhibitor conjugate, particle or composition, e.g., a CDP-camptothecin or camptothecin derivative conjugate, particle or composition, a CDP-camptothecin or camptothecin derivative conjugate, particle or composition described herein, e.g., CRLX101, prior to the initial administration.

In an embodiment, the CDP-topoisomerase inhibitor conjugate, particle or composition is administered as a first line treatment for the cancer.

In an embodiment, the CDP-topoisomerase inhibitor conjugate, particle or composition is administered as a second, third or fourth line treatment for the cancer. In an embodiment, the cancer is sensitive to one or more chemotherapeutic agents, e.g., a platinum-based agent, a taxane, an alkylating agent, an antimetabolite and/or a vinca alkaloid. In an embodiment, the cancer is a refractory, relapsed or resistant to one or more chemotherapeutic agents, e.g., a platinum-based agent, a taxane, an alkylating agent, an anthracycline (e.g., doxorubicin (e.g., liposomal doxorubicin)), and an antimetabolite and/or a vinca alkaloid. In one embodiment, the cancer is, e.g., ovarian cancer, and the ovarian cancer is refractory, relapsed or resistant to a platinum-based agent (e.g., carboplatin, cisplatin, oxaliplatin), a taxane (e.g., paclitaxel, docetaxel, larotaxel, cabazitaxel) and/or an anthracycline (e.g., doxorubicin (e.g., liposomal doxorubicin)). In one embodiment, the cancer is, e.g., colorectal cancer, and the cancer is refractory, relapsed or resistant to an antimetabolite (e.g., an antifolate (e.g., pemetrexed, floxuridine, raltitrexed) and a pyrimidine analogue (e.g., capecitabine, cytrarabine, gemcitabine, 5FU)) and/or a platinum-based agent (e.g., carboplatin, cisplatin, oxaliplatin). In one embodiment, the cancer is, e.g., lung cancer, and the cancer is refractory, relapsed or resistant to a taxane (e.g., paclitaxel, docetaxel, larotaxel, cabazitaxel), a platinum-based agent (e.g., carboplatin, cisplatin, oxaliplatin), a vinca alkaloid (e.g., vinblastine, vincristine, vindesine, vinorelbine), a vascular endothelial growth factor (VEGF) pathway inhibitor, an epidermal growth factor (EGF) pathway inhibitor) and/or an antimetabolite (e.g., an antifolate (e.g., pemetrexed, floxuridine, raltitrexed) and a pyrimidine analogue (e.g., capecitabine, cytrarabine, gemcitabine, 5FU)). In one embodiment, the cancer is, e.g., breast cancer, and the cancer is refractory, relapsed or resistant to a taxane (e.g., paclitaxel, docetaxel, larotaxel, cabazitaxel), a vascular endothelial growth factor (VEGF) pathway inhibitor, an anthracycline (e.g., daunorubicin, doxorubicin (e.g., liposomal doxorubicin), epirubicin, valrubicin, idarubicin), a platinum-based agent (e.g., carboplatin, cisplatin, oxaliplatin) and/or an antimetabolite (e.g., an antifolate (e.g., pemetrexed, floxuridine, raltitrexed) and a pyrimidine analogue (e.g., capecitabine, cytrarabine, gemcitabine, 5FU)). In one embodiment, the cancer is, e.g., gastric cancer, and the cancer is refractory, relapsed or resistant to an antimetabolite (e.g., an antifolate (e.g., pemetrexed, floxuridine, raltitrexed) and a pyrimidine analogue (e.g., capecitabine, cytrarabine, gemcitabine, 5FU)) and/or a platinum-based agent (e.g., carboplatin, cisplatin, oxaliplatin).

In one embodiment, the subject has ovarian cancer that is refractory, relapsed or resistant to a platinum-based agent, and the subject is administered a CDP-topoisomerase inhibitor conjugate, particle or composition, e.g., a CDP-topoisomerase inhibitor conjugate, particle or composition described herein. In one embodiment, the CDP-topoisomerase inhibitor conjugate, particle or composition is administered in combination with doxorubicin (e.g., liposomal doxorubicin). In one embodiment, the doxorubicin (e.g., the liposomal doxorubicin) is administered at a dose of about 20 mg/m², about 30 mg/m² or about 40 mg/m², every 24, 25, 26, 27, 28, 29, 30 or 31 days, e.g., 28 days. In one embodiment, when the CDP-topoisomerase inhibitor conjugate, particle or composition is administered in combination with doxorubicin (e.g., liposomal doxorubicin), the dose at which the CDP-topoisomerase inhibitor conjugate, particle or composition is administered is 1%, 3%, 5%, 10%, 15%, 20%, 25%, 30% less than a dose described herein.

In an embodiment, the cancer has been sensitized to a topoisomerase inhibitor, e.g., the subject has received radiation and/or the subject has received a phosphatase inhibitor (e.g., okadiac acid) prior to the administration of the CDP-topoisomerase inhibitor conjugate, particle or composition. In an embodiment, the cancer is sensitized to topoisomerase inhibitors, e.g., the subject receives radiation in combination with the administration of the CDP-topoisomerase inhibitor conjugate, particle or composition and/or the subject is administered a phosphatase inhibitor (e.g., okadiac acid) in combination with the administration of the CDP-topoisomerase inhibitor conjugate, particle or composition. In one embodiment, the cancer is sensitized or has been sensitized to topoisomerase inhibitors and the cancer is a glial cell cancer (e.g., glioblastoma multiforme) or head and neck cancer.

In one embodiment, the conjugate, particle or composition is administered in combination with one or more additional chemotherapeutic agent, e.g., a chemotherapeutic agent (such as an angiogenesis inhibitor), or combination of chemotherapeutic agents described herein. In one embodiment, the composition is administered in combination with one or more of: a platinum-based agent (e.g., carboplatin, cisplatin, oxaliplatin), a taxane (e.g., paclitaxel, docetaxel, larotaxel, cabazitaxel), a vinca alkaloid (e.g., vinblastine, vincristine, vindesine, vinorelbine), an antimetabolite (e.g., an antifolate (e.g., pemetrexed, floxuridine, raltitrexed) and a pyrimidine analogue (e.g., 5FU, capecitabine, cytrarabine, gemcitabine)), an alkylating agent (e.g., cyclophosphamide, decarbazine, melphalan, ifosfamide, temozolomide), a vascular endothelial growth factor (VEGF) pathway inhibitor, a poly ADP-ribose polymerase (PARP) inhibitor and an mTOR inhibitor. In one embodiment, when the CDP-topoisomerase inhibitor conjugate, particle or composition is administered in combination with an additional chemotherapeutic agent, the dose at which the CDP-topoisomerase inhibitor conjugate, particle or composition is administered is 1%, 3%, 5%, 10%, 15%, 20%, 25%, 30% less than a dose described herein.

In an embodiment, the method further comprises administering to said subject a treatment that reduces one or more side effect associated with administration of a CDP-topoisomerase inhibitor conjugate, particle or composition, e.g., a treatment described herein.

In one aspect, the disclosure features, a method of treating ovarian cancer (e.g., epithelial carcinoma, fallopian tube cancer, germ cell cancer (e.g., a teratoma), sex cord-stromal tumor (e.g., estrogen-producing granulose cell tumor, virilizing Sertoli-Leydig tumor, arrhenoblastoma)), e.g., locally advanced or metastatic ovarian cancer, in a subject, e.g., a human subject. The method comprises administering a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, in combination with a second chemotherapeutic agent.

In one embodiment, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered prior to surgery, after surgery or before and after surgery to remove the cancer, e.g., to remove a primary tumor and/or a metastases.

In one embodiment, the method comprises administering a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, in combination with a platinum-based agent (e.g., cisplatin, carboplatin, oxaliplatin). In one embodiment, the conjugate, particle or composition is further administered in combination with an anti-metabolite, e.g., an antifolate (e.g., pemetrexed, floxuridine, raltitrexed) or pyrimidine analogue (e.g., capecitabine, cytrarabine, gemcitabine, 5FU)). In one embodiment, the conjugate, particle or composition is further administered in combination with an anti-metabolite, e.g., an antifolate (e.g., pemetrexed, floxuridine, raltitrexed) or pyrimidine analogue (e.g., capecitabine, cytrarabine, gemcitabine, 5FU)) and folinic acid (leucovorin).

In one embodiment, the conjugate, particle or composition is administered in combination with an angiogenesis inhibitor (e.g., an angiogenesis inhibitor described herein such as an inhibitor of the VEGF pathway).In one embodiment, the method comprises administering a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, in combination with a taxane (e.g., paclitaxel, docetaxel, larotaxel, cabazitaxel). In one embodiment, the conjugate, particle or composition is further administered in combination with a platinum-based agent (e.g., cisplatin, carboplatin, oxaliplatin). In one embodiment, the conjugate, particle or composition is further administered in combination with an anti-metabolite, e.g., an antifolate (e.g., pemetrexed, floxuridine, raltitrexed) or pyrimidine analogue (e.g., capecitabine, cytrarabine, gemcitabine, 5FU)). In one embodiment, the conjugate, particle or composition is further administered in combination with an anti-metabolite, e.g., an antifolate (e.g., pemetrexed, floxuridine, raltitrexed) or pyrimidine analogue (e.g., capecitabine, cytrarabine, gemcitabine, 5FU)) and folinic acid (leucovorin).

In one embodiment, the method comprises administering a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, in combination with an anthracycline (e.g., doxorubicin (e.g., liposomal doxorubicin), daunorubicin, epirubicin, idarubicin, mitoxantrone, valrubicin). In one embodiment, the cancer is refractory, relapsed or resistant to a taxane and/or a platinum-based agent.

In one embodiment, the conjugate, particle or composition is administered in combination with one or more of: an anti-metabolite, e.g., an antifolate (e.g., pemetrexed, floxuridine, raltitrexed) or pyrimidine analogue (e.g., capecitabine, cytrarabine, gemcitabine, 5FU); an alkylating agent (e.g., cyclophosphamide, decarbazine, melphalan, ifosfamide, temozolomide); a platinum-based agent (carboplatin, cisplatin, oxaliplatin); a vinca alkaloid (e.g., vinblastine, vincristine, vindesine, vinorelbine). In one embodiment, the conjugate, particle or composition is administered in combination with one or more of: capecitabine, cyclophosphamide, gemcitabine, ifosfamide, melphalan, oxaliplatin, vinorelbine, vincristine and pemetrexed. In one embodiment, the cancer is refractory, relapsed or resistant to a taxane and/or a platinum-based agent.

In one embodiment, the conjugate, particle or composition is administered at a dose and/or dosing schedule described herein. In one embodiment, when the CDP-topoisomerase inhibitor conjugate, particle or composition is administered in combination with an additional chemotherapeutic agent, the dose at which the CDP-topoisomerase inhibitor conjugate, particle or composition is administered is 1%, 3%, 5%, 10%, 15%, 20%, 25%, 30% less than a dose described herein.

In one embodiment, the conjugate, particle or composition is administered in combination with a treatment that reduces one or more side effect associated with the administration of a CDP-topoisomerase inhibitor conjugate, particle or composition, e.g., a treatment described herein.

In another aspect the disclosure features a method of treating colorectal cancer (e.g., colon, small intestine, rectum and/or appendix cancer), e.g., locally advanced or metastatic colorectal cancer, in a subject, e.g., a human subject. The method comprises administering a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, in combination with a second chemotherapeutic agent.

In one embodiment, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered prior to surgery, after surgery or before and after surgery to remove the cancer, e.g., to remove the primary tumor and/or a metastases.

In one embodiment, the method comprises administering a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, in combination with an antimetabolite, e.g., an antifolate (e.g., pemetrexed, floxuridine, raltitrexed). In one embodiment, the conjugate, particle or composition is administered in combination with an antimetabolite, e.g., pyrimidine analogue (e.g., capecitabine, cytrarabine, gemcitabine, 5FU) and folinic acid (leucovorin). In one embodiment, the conjugate, particle or composition is further administered in combination with a platinum-based agent (e.g., cisplatin, carboplatin, oxaliplatin). For example, in one embodiment, the conjugate, particle or composition is administered in combination with an antimetabolite, e.g., 5FU, folinic acid (leucovorin), and a platinum-based agent, e.g., oxaliplatin. In another embodiment, the antimetabolite is a pyrimidine analogue, e.g., capecitabine.

In one embodiment, the conjugate, particle or composition is administered in combination with an angiogenesis inhibitor (e.g., an angiogenesis inhibitor described herein such as an inhibitor of the VEGF pathway).In one embodiment, the method includes administering a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, in combination with a platinum-based agent (e.g., cisplatin, carboplatin, oxaliplatin).

In one embodiment, the method comprises administering a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, in combination with a vascular endothelial growth factor (VEGF) pathway inhibitor, e.g., a VEGF inhibitor or VEGF receptor inhibitor. In one embodiment, the VEGF inhibitor is bevacizumab or AV-951. In one embodiment, the VEGF receptor inhibitor is selected from CP-547632 and AZD2171. In one embodiment, the conjugate, particle or composition is administered in combination with a VEGF pathway inhibitor, e.g., bevacizumab, and an antimetabolite, e.g., an antifolate (e.g., pemetrexed, floruridine, raltitrexed) or pyrimidine analogue (e.g., capecitabine, 5FU, cytrarabine, gemcitabine). In one embodiment, the conjugate, particle or composition is administered with a VEGF pathway inhibitor, e.g., bevacizumab, an antimetabolite, e.g., a pyrimidine analogue (e.g., 5FU), and folinic acid (leucovorin). In another embodiment, the conjugate, particle or composition is administered with a VEGF pathway inhibitor, e.g., bevacizumab, an antimetabolite, e.g., a pyrimidine analogue (e.g., 5FU), folinic acid (leucovorin), and a platinum-based agent (e.g., cisplatin, carboplatin, oxaliplatin). In one embodiment, the cancer is refractory, relapsed or resistant to an antimetabolite and/or a platinum-based agent.

In another embodiment, a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with a VEGF pathway inhibitor, e.g., bevacizumab, and an antimetabolite wherein the antimetabolite is a pyrimidine analogue, e.g., capecitabine. In one embodiment, the conjugate, particle or composition is further administered in combination with a platinum-based agent (e.g., cisplatin, carboplatin, oxaliplatin). For example, in one embodiment, the conjugate, particle or composition is administered with the following combination: a VEGF pathway inhibitor, e.g., a VEGF inhibitor (e.g., bevacizumab) or a VEGF receptor inhibitor, a pyrimidine analogue (e.g., capecitabine), and a platinum-based agent (e.g., oxaliplatin); or a VEGF pathway inhibitor (e.g., bevacizumab) and a pyrimidine analogue (e.g., capecitabine). In one embodiment, the cancer is refractory, relapsed or resistant to an antimetabolite and/or a platinum-based agent.

In one embodiment, a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with an epidermal growth factor (EGF) pathway inhibitor, e.g., an EGF inhibitor or EGF receptor inhibitor. The EGF receptor inhibitor can be, e.g., cetuximab, erlotinib, gefitinib, panitumumab. In one embodiment, the conjugate, particle or composition is administered in combination with an EGF pathway inhibitor, e.g., cetuximab or panitumumab, and a VEGF pathway inhibitor, e.g., bevacizumab. In one embodiment, the cancer is refractory, relapsed or resistant to an antimetabolite and/or a platinum-based agent.

In one embodiment, the conjugate, particle or composition is administered at a dose and/or dosing schedule described herein. In one embodiment, when the CDP-topoisomerase inhibitor conjugate, particle or composition is administered in combination with an additional chemotherapeutic agent, the dose at which the CDP-topoisomerase inhibitor conjugate, particle or composition is administered is 1%, 3%, 5%, 10%, 15%, 20%, 25%, 30% less than a dose described herein.

In one embodiment, the conjugate, particle or composition is administered in combination with a treatment that reduces one or more side effect associated with the administration of a CDP-topoisomerase inhibitor conjugate, particle or composition, e.g., a treatment described herein.

In one aspect, the disclosure features a method of treating lung cancer (e.g., small cell lung cancer or non-small cell lung cancer (e.g., adenocarcinoma, squamous cell carcinoma, bronchoalveolar carcinoma and large cell carcinoma)), e.g., locally advanced or metastatic lung cancer, in a subject, e.g., a human subject. The method comprises administering a CDP-topoisomerase inhibitor conjugate, particle or composition, e.g., a CDP-topoisomerase I or II inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101. In one embodiment, the method comprises selecting a subject that has squamous cell lung cancer for treatment with a CDP-topoisomerase inhibitor conjugate, particle or composition, e.g., a CDP-topoisomerase I or II inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101.

In one embodiment, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered prior to surgery, after surgery or before and after surgery to remove the cancer, e.g., to remove a primary tumor and/or a metastases.

In one embodiment, the method includes selecting a subject who has lung cancer and who has increased KRAS and/or ST expression levels, e.g., as compared to a reference standard, and/or has a mutation in a KRAS and/or ST gene; and
administering a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, to the subject in an amount effective to treat the cancer, to thereby treat the cancer.

In one embodiment, the subject has increased KRAS and/or ST expression levels, e.g., as compared to a reference standard, and/or has a mutation in a KRAS and/or ST gene. In one embodiment, the subject has a mutation at one or more of: codon 12 of the KRAS gene (e.g., a G to T transversion), codon 13 of the KRAS gene, codon 61 of the KRAS gene. In one embodiment, the subject has non small cell lung cancer associated with mucinous broncholoalveolar cells or goblet cells.

In one embodiment, the method includes selecting a subject who has lung cancer and who has a mutation in an EGFR gene; and
administering a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, to the subject in an amount effective to treat the cancer, to thereby treat the cancer.

In one embodiment, the subject has lung cancer that is resistant, relapsed or refractory to an EGF pathway inhibitor, e.g., an EGF receptor inhibitor (e.g., erlotinib)

In one embodiment, the subject has one or more of the following mutations: codon 719 of the EGFR gene (e.g., a missense mutation that results in a glycine to cysteine, alanine or serine substitution at codon 719 of the EGFR gene), codon 746 of the EGFR gene (e.g., a deletion of one or more nucleic acids of codon 746 of the EGFR gene), codon 747 of the EGFR gene (e.g., a deletion of one or more nucleic acids of codon 747 of the EGFR gene), codon 748 of the EGFR gene (e.g., a deletion of one or more nucleic acids of codon 748 of the EGFR gene), codon 749 of the EGFR gene (e.g., a deletion of one or more nucleic acids of codon 749 of the EGFR gene), codon 750 of the EGFR gene (e.g., a deletion of one or more nucleic acids of codon 750 of the EGFR gene), codon 858 of the EGFR gene (e.g., a missense mutation that results in a leucine to arginine substitution at codon 858 of the EGFR gene), a deletion in exon 19 of the EGFR gene, and an insert mutation at exon 20 of the EGFR gene.

In one embodiment, the subject has a mutation in the EGFR gene and has a mutation in the KRAS gene and/or overexpresses KRAS, e.g., as compared to a reference standard.

In one embodiment, the method includes selecting a subject who has lung cancer and who does not have a mutation in an EGFR gene; and
administering a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, to the subject in an amount effective to treat the cancer, to thereby treat the cancer.

In one embodiment, the method includes selecting a subject who has squamous cell lung cancer; and
administering a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, to the subject in an amount effective to treat the cancer, to thereby treat the cancer.

In one embodiment, the subject does not have one or more of the following mutations: codon 719 of the EGFR gene (e.g., a missense mutation that results in a glycine to cysteine, alanine or serine substitution at codon 719 of the EGFR gene), codon 746 of the EGFR gene (e.g., a deletion of one or more nucleic acids of codon 746 of the EGFR gene), codon 747 of the EGFR gene (e.g., a deletion of one or more nucleic acids of codon 747 of the EGFR gene), codon 748 of the EGFR gene (e.g., a deletion of one or more nucleic acids of codon 748 of the EGFR gene), codon 749 of the EGFR gene (e.g., a deletion of one or more nucleic acids of codon 749 of the EGFR gene), codon 750 of the EGFR gene (e.g., a deletion of one or more nucleic acids of codon 750 of the EGFR gene), codon 858 of the EGFR gene (e.g., a missense mutation that results in a leucine to arginine substitution at codon 858 of the EGFR gene), a deletion in exon 19 of the EGFR gene, and an insert mutation at exon 20 of the EGFR gene.

In one embodiment, the subject has a mutation in the KRAS gene and/or overexpresses KRAS, e.g., as compared to a reference standard, and does not have a mutation in the EGFR gene.

In one embodiment, the subject is refractory, relapsed or resistant to one or more chemotherapeutic agents, e.g., a platinum-based agent (e.g., carboplatin, cisplatin, oxaliplatin) and/or an EGF pathway inhibitor, e.g., an EGF inhibitor or and EGFR inhibitor, e.g., erlotinib.

In one embodiment, the conjugate, particle or composition is administered at a dose and/or dosing schedule described herein.

In one embodiment, the conjugate, particle or composition is administered in combination with a treatment that reduces one or more side effect associated with the administration of a CDP-topoisomerase inhibitor conjugate, particle or composition, e.g., a treatment described herein.

In one aspect, the disclosure features a method of treating lung cancer (e.g., small cell lung cancer and non-small cell lung cancer (e.g., adenocarcinoma, squamous cell carcinoma, bronchoalveolar carcinoma and large cell carcinoma)), e.g., locally advanced or metastatic lung cancer, in a subject, e.g., a human subject. The method comprises administering a CDP-topoisomerase inhibitor conjugate, particle or composition, e.g., a CDP-topoisomerase I or II inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, in combination with a second chemotherapeutic agent.

In one embodiment, the method includes selecting a subject who has squamous cell lung cancer; and
administering a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, to the subject in an amount effective to treat the cancer, to thereby treat the cancer.

In one embodiment, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered prior to surgery, after surgery or before and after surgery to remove the cancer, e.g., to remove a primary tumor and/or a metastases.

In one embodiment, the method comprises administering a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, in combination with an epidermal growth factor (EGF) pathway inhibitor, e.g., an EGF inhibitor or EGF receptor inhibitor. The EGF receptor inhibitor can be, e.g., cetuximab, erlotinib, gefitinib, panitumumab. In an embodiment, the subject has a mutation at codon 858 of the gene encoding the EGF receptor, e.g., that results in a substitution of a leucine to an arginine in the EGF receptor. In one embodiment, the conjugate, particle or composition is administered in combination with an EGF pathway inhibitor, e.g., cetuximab, erlotinib, gefitinib, panitumumab, and radiation. In one embodiment, the conjugate, particle or composition is administered in combination with an EGF pathway inhibitor, e.g., cetuximab, erlotinib, gefitinib, panitumumab, and one or more additional chemotherapeutic agents. For example, the chemotherapeutic agent can be a platinum-based agent (e.g., cisplatin, carboplatin, oxaliplatin), a taxane (e.g., paclitaxel, docetaxel, larotaxel, cabazitaxel), a vinca alkaloid (e.g., vinblastine, vincristine, vindesine, vinorelbine), an anti-metabolite, e.g., an antifolate (e.g., pemetrexed, floxuridine, raltitrexed) or pyrimidine analogue (e.g., capecitabine, cytrarabine, gemcitabine, 5FU), and combinations thereof.

In one embodiment, a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with a vascular endothelial growth factor (VEGF) pathway inhibitor, e.g., a VEGF inhibitor or VEGF receptor inhibitor. In one embodiment, the VEGF inhibitor is bevacizumab or AV-951. In one embodiment, the VEGF receptor inhibitor is selected from CP-547632 and AZD2171. In one embodiment, the conjugate, particle or composition is administered in combination with a VEGF pathway inhibitor, e.g., bevacizumab, and radiation. In one embodiment, the conjugate, particle or composition is administered in combination with a VEGF pathway inhibitor, e.g., bevacizumab, and one or more additional chemotherapeutic agents. For example, the chemotherapeutic agent can be a platinum-based agent (e.g., cisplatin, carboplatin, oxaliplatin), a taxane (e.g., paclitaxel, docetaxel, larotaxel, cabazitaxel), a vinca alkaloid (e.g., vinblastine, vincristine, vindesine, vinorelbine), an anti-metabolite, e.g., an antifolate (e.g., pemetrexed, floxuridine, raltitrexed) or pyrimidine analogue (e.g., capecitabine, cytrarabine, gemcitabine, 5FU), and combinations thereof. In one embodiment, the cancer is refractory, relapsed or resistant to one or more chemotherapeutic agents, e.g., an EGF pathway inhibitor, e.g., erlotinib.

In one embodiment, the conjugate, particle or composition is administered in combination with an angiogenesis inhibitor (e.g., an angiogenesis inhibitor described herein such as an inhibitor of the VEGF pathway).In one embodiment, a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with a platinum-based agent (e.g., cisplatin, carboplatin, oxaliplatin). In one embodiment, the conjugate, particle or composition is further administered in combination with a taxane (e.g., paclitaxel, docetaxel, larotaxel, cabazitaxel), a vinca alkaloid (e.g., vinblastine, vincristine, vindesine, vinorelbine) and/or an anti-metabolite, e.g., an antifolate (e.g., pemetrexed, floxuridine, raltitrexed) or pyrimidine analogue (e.g., capecitabine, cytrarabine, gemcitabine, 5FU). In one embodiment, the method further includes administering radiation to the subject. In one embodiment, the cancer is refractory, relapsed or resistant to one or more chemotherapeutic agents, e.g., an EGF pathway inhibitor (e.g., erlonitib), a VEGF pathway inhibitor and/or a taxane.

In one embodiment, a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with a taxane (e.g., paclitaxel, docetaxel, larotaxel, cabazitaxel). In one embodiment, the method further includes administering radiation to the subject. In one embodiment, the cancer is refractory, relapsed or resistant to one or more chemotherapeutic agents, e.g., an EGF pathway inhibitor (e.g., erlotinib), a VEGF pathway inhibitor and/or a platinum-based agent.

In one embodiment, a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with an anti-metabolite, e.g., an antifolate (e.g., pemetrexed, floxuridine, raltitrexed). In one embodiment, the method further includes administering radiation to the subject. In one embodiment, the cancer is refractory, relapsed or resistant to one or more chemotherapeutic agents, e.g., an EGF pathway inhibitor (e.g., erlotinib), a VEGF pathway inhibitor, a taxane and/or a platinum-based agent.

In one embodiment, the conjugate, particle or composition is administered at a dose and/or dosing schedule described herein. In one embodiment, when the CDP-topoisomerase inhibitor conjugate, particle or composition is administered in combination with an additional chemotherapeutic agent, the dose at which the CDP-topoisomerase inhibitor conjugate, particle or composition is administered is 1%, 3%, 5%, 10%, 15%, 20%, 25%, 30% less than a dose described herein.

In one embodiment, the conjugate, particle or composition is administered in combination with a treatment that reduces one or more side effect associated with the administration of a CDP-topoisomerase inhibitor conjugate, particle or composition, e.g., a treatment described herein.

In one aspect, the disclosure features a method of treating breast cancer (e.g., estrogen receptor positive breast cancer; estrogen receptor negative breast cancer; HER-2 positive breast cancer; HER-2 negative breast cancer; progesterone receptor positive breast cancer; progesterone receptor negative breast cancer; estrogen receptor negative, HER-2 negative and progesterone receptor negative breast cancer (i.e., triple negative breast cancer)), e.g., locally advanced or metastatic breast cancer, in a subject, e.g., a human subject. The method comprises administering a CDP-topoisomerase inhibitor conjugate, particle or composition, e.g., a topoisomerase I or II inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, in combination with a second chemotherapeutic agent.

In one embodiment, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered prior to surgery, after surgery or before and after surgery to remove the cancer, e.g., to remove a primary tumor and/or a metastases.

In one embodiment, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with a HER-2 pathway inhibitor, e.g., a HER-2 inhibitor or a HER-2 receptor inhibitor. For example, the CDP-topoisomerase inhibitor conjugate, particle or composition is administered with trastuzumab.

In some embodiments, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with a vascular endothelial growth factor (VEGF) pathway inhibitor, e.g., a VEGF inhibitor (e.g., bevacizumab) or VEGF receptor inhibitor (e.g., CP-547632 and AZD2171). In one embodiment, the CDP-topoisomerase inhibitor conjugate, particle or composition is administered in combination with bevacizumab. In some embodiments, the method further comprises administering a taxane (e.g., paclitaxel, docetaxel, larotaxel, cabazitaxel). In one embodiment, the method further comprises administering a poly ADP-ribose polymerase (PARP) inhibitor (e.g., BSI 201, Olaparib (AZD-2281), ABT-888, AG014699, CEP 9722, MK 4827, KU-0059436 (AZD2281), LT-673, 3-aminobenzamide).

In some embodiments, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with a platinum-based agent (e.g., cisplatin, carboplatin, oxaliplatin). In some embodiments, the method further comprises administering a taxane (e.g., paclitaxel, docetaxel, larotaxel, cabazitaxel). In some embodiments, the method further comprises administering an mTOR inhibitor. Non-limiting examples of mTOR inhibitors include rapamycin, everolimus, AP23573, CCI-779 and SDZ-RAD. In one embodiment, the method further comprises administering a PARP inhibitor (e.g., BSI 201, Olaparib (AZD-2281), ABT-888, AG014699, CEP 9722, MK 4827, KU-0059436 (AZD2281), LT-673, 3-aminobenzamide). In some embodiments, the method further comprises administering a VEGF pathway inhibitor, e.g., a VEGF inhibitor (e.g., bevacizumab) or VEGF receptor inhibitor (e.g., CP-547632 and AZD2171).

In one embodiment, the conjugate, particle or composition is administered in combination with an angiogenesis inhibitor (e.g., an angiogenesis inhibitor described herein such as an inhibitor of the VEGF pathway).In some embodiments, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with a taxane (e.g., paclitaxel, docetaxel, larotaxel, cabazitaxel). In some embodiments, the method further comprises administering an mTOR inhibitor. Non-limiting examples of mTOR inhibitors include rapamycin, everolimus, AP23573, CCI-779 and SDZ-RAD. In one embodiment, the method further comprises administering a PARP inhibitor (e.g., BSI 201, Olaparib (AZD-2281), ABT-888, AG014699, CEP 9722, MK 4827, KU-0059436 (AZD2281), LT-673, 3-aminobenzamide).

In some embodiments, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with an epothilone (e.g., ixabelipone, epothilone B, epothilone D, BMS310705, dehydelone, ZK-EPO). In some embodiments, the method further comprises administering an mTOR inhibitor. Non-limiting examples of mTOR inhibitors include rapamycin, everolimus, AP23573, CCI-779 and SDZ-RAD. In one embodiment, the method further comprises administering a PARP inhibitor (e.g., BSI 201, Olaparib (AZD-2281), ABT-888, AG014699, CEP 9722, MK 4827, KU-0059436 (AZD2281), LT-673, 3-aminobenzamide). In some embodiments, the method further comprises administering a VEGF pathway inhibitor, e.g., a VEGF inhibitor (e.g., bevacizumab) or VEGF receptor inhibitor (e.g., CP-547632 and AZD2171). In some embodiments, the method further includes administering one or more of an anthracycline (e.g., daunorubicin, doxorubicin (liposomal doxorubicin), epirubicin, valrubicin and idarubicin) and/or an anti-metabolite (e.g., floxuridine, pemetrexed, 5FU).

In some embodiments, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with an anthracycline (e.g., daunorubicin, doxorubicin (liposomal doxorubicin), epirubicin, valrubicin and idarubicin). In one embodiment, the cancer is refractory, relapsed or resistant to one or more chemotherapeutic agents, e.g., a HER-2 pathway inhibitor, a VEGF pathway inhibitor, a taxane, an antimetabolite and/or a platinum-based agent.

In some embodiments, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with an anti-metabolite, e.g., an antifolate (e.g., floxuridine, pemetrexed) or pyrimidine analogue (e.g., 5FU)). In one embodiment, the cancer is refractory, relapsed or resistant to one or more chemotherapeutic agents, e.g., a HER-2 pathway inhibitor, a VEGF pathway inhibitor, a taxane, an anthracycline and/or a platinum-based agent.

In some embodiments, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with an anthracycline (e.g., daunorubicin, doxorubicin (liposomal doxorubicin), epirubicin, valrubicin and idarubicin) and an anti-metabolite (e.g., floxuridine, pemetrexed, 5FU). In one embodiment, the cancer is refractory, relapsed or resistant to one or more chemotherapeutic agents, e.g., a HER-2 pathway inhibitor, a VEGF pathway inhibitor, and/or a platinum-based agent.

In some embodiments, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with an mTOR inhibitor. Non-limiting examples of mTOR inhibitors include rapamycin, everolimus, AP23573, CCI-779 and SDZ-RAD. In some embodiments, the method further comprises administering a PARP inhibitor (e.g., BSI 201, Olaparib (AZD-2281), ABT-888, AG014699, CEP 9722, MK 4827, KU-0059436 (AZD2281), LT-673, 3-aminobenzamide).

In some embodiments, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with a PARP inhibitor (e.g., BSI 201, Olaparib (AZD-2281), ABT-888, AG014699, CEP 9722, MK 4827, KU-0059436 (AZD2281), LT-673, 3-aminobenzamide).

In some embodiments, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with a pyrimidine analogue, e.g., a pyrimidine analogue described herein (e.g., capecitabine). In some embodiments, the method further comprises administering a taxane (e.g., docetaxel, paclitaxel, larotaxel, cabazitaxel). In some embodiments, the method further comprises administering an epothilone (e.g., ixabelipone, epothilone B, epothilone D, BMS310705, dehydelone, ZK-EPO).

In one embodiment, the conjugate, particle or composition is administered at a dose and/or dosing schedule described herein. In one embodiment, when the CDP-topoisomerase inhibitor conjugate, particle or composition is administered in combination with an additional chemotherapeutic agent, the dose at which the CDP-topoisomerase inhibitor conjugate, particle or composition is administered is 1%, 3%, 5%, 10%, 15%, 20%, 25%, 30% less than a dose described herein.

In one embodiment, the conjugate, particle or composition is administered in combination with a treatment that reduces one or more side effect associated with the administration of a CDP-topoisomerase inhibitor conjugate, particle or composition, e.g., a treatment described herein.

In one aspect, the disclosure features a method of treating gastric cancer (e.g., gastric adenocarcinoma (e.g., intestinal or diffuse), gastric lymphoma (e.g., MALT lymphoma), carcinoid stromal tumor), e.g., locally advanced or metastatic gastric cancer, in a subject, e.g., a human subject. The method comprises administering a CDP-topoisomerase inhibitor conjugate, particle or composition, e.g., a CDP-topoisomerase I or II inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, in combination with a second chemotherapeutic agent.

In one embodiment, the gastric cancer is gastroesophageal junction adenocarcinoma.

In one embodiment, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered prior to surgery, after surgery or before and after surgery to remove the cancer, e.g., to remove a primary tumor and/or a metastases.

In one embodiment, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with one or more of an anthracycline (e.g., daunorubicin, doxorubicin (e.g., liposomal doxorubicin), epirubicin, valrubicin, mitoxatrone, and idarubicin), a platinum-based agent (e.g., cisplatin, carboplatin, oxaliplatin) and an anti-metabolite, e.g., an antifolate (e.g., floxuridine, pemetrexed, raltitrexed) or pyrimidine analogue (e.g., 5FU, capecitabine, cytrarabine, gemcitabine)). For example, in one embodiment, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with an anthracycline (e.g., daunorubicin, doxorubicin (e.g., liposomal doxorubicin), epirubicin, valrubicin, mitoxatrone and idarubicin), a platinum-based agent (e.g., cisplatin, carboplatin, oxaliplatin) and an anti-metabolite, e.g., an antifolate (e.g., floxuridine, pemetrexed, raltitrexed) or pyrimidine analogue (e.g., 5FU, capecitabine, cytrarabine, gemcitabine). In one embodiment, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with an anthracycline (e.g., daunorubicin, doxorubicin (e.g., liposomal doxorubicin), epirubicin, valrubicin, mitoxatrone and idarubicin). In one embodiment, the cancer is refractory, relapsed or resistant to one or more chemotherapeutic agents, e.g., a platinum-based agent (e.g., cisplatin, carboplatin, oxaliplatin).

In another embodiment, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with a platinum-based agent (e.g., cisplatin, carboplatin, oxaliplatin) and an anti-metabolite, e.g., an antifolate (e.g., floxuridine, pemetrexed, raltitrexed) or pyrimidine analogue (e.g., 5FU, capecitabine, cytrarabine, gemcitabine).

In some embodiments, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with an anti-metabolite, e.g., an antifolate (e.g., floxuridine, pemetrexed, raltitrexed) or pyrimidine analogue (e.g., capecitabine, 5FU, cytrarabine, gemcitabine). In one embodiment, the method further includes administering a taxane (e.g., paclitaxel, docetaxel, larotaxel, cabazitaxel). For example, in one embodiment, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with an anti-metabolite, e.g., an antifolate (e.g., floxuridine, pemetrexed, raltitrexed) or pyrimidine analogue (e.g., capecitabine, 5FU, cytrarabine, gemcitabine) and a taxane (e.g., paclitaxel, docetaxel, larotaxel, cabazitaxel).

In one embodiment, the conjugate, particle or composition is administered in combination with an angiogenesis inhibitor (e.g., an angiogenesis inhibitor described herein such as an inhibitor of the VEGF pathway).

In one embodiment, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with radiation.

In some embodiments, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with a vascular endothelial growth factor (VEGF) pathway inhibitor, e.g., a VEGF inhibitor (e.g., bevacizumab) or VEGF receptor inhibitor (e.g., CP-547632 and AZD2171). In one embodiment, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with bevacizumab. In one embodiment, the cancer is refractory, relapsed or resistant to one or more chemotherapeutic agents, e.g., an antimetabolite, a platinum-based agent and/or an anthracycline.

In some embodiments, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with an mTOR inhibitor. Non-limiting examples of mTOR inhibitors include rapamycin, everolimus, AP23573, CCI-779 and SDZ-RAD. In one embodiment, the cancer is refractory, relapsed or resistant to one or more chemotherapeutic agents, e.g., an antimetabolite, a platinum-based agent and/or an anthracycline.

In some embodiments, the CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, is administered in combination with a poly ADP-ribose polymerase (PARP) inhibitor (e.g., BSI 201, Olaparib (AZD-2281), ABT-888, AG014699, CEP 9722, MK 4827, KU-0059436 (AZD2281), LT-673, 3-aminobenzamide). In one embodiment, the cancer is refractory, relapsed or resistant to one or more chemotherapeutic agents, e.g., an antimetabolite, a platinum-based agent and/or an anthracycline.

In one embodiment, the conjugate, particle or composition is administered at a dose and/or dosing schedule described herein. In one embodiment, when the CDP-topoisomerase inhibitor conjugate, particle or composition is administered in combination with an additional chemotherapeutic agent, the dose at which the CDP-topoisomerase inhibitor conjugate, particle or composition is administered is 1%, 3%, 5%, 10%, 15%, 20%, 25%, 30% less than a dose described herein.

In one embodiment, the conjugate, particle or composition is administered in combination with a treatment that reduces one or more side effect associated with the administration of a CDP-topoisomerase inhibitor conjugate, particle or composition, e.g., a treatment described herein.

In one aspect, the disclosure features, a method of treating pancreatic cancer in a subject, the method comprising, administering a CDP-topoisomerase inhibitor conjugate, particle or composition to the subject in combination with a pyrimidine analogue (e.g., capecitabine, 5FU, cytrarabine, gemcitabine). In an embodiment, the pyrimidine analogue is gemcitabine.

In one aspect, the disclosure features, a method of treating a proliferative disorder, e.g., a cancer, in a subject, e.g., a human subject. The method comprises:
providing a subject who has a proliferative disorder, e.g., cancer; and
administering a composition that comprises a CDP-topoisomerase inhibitor conjugate, particle or composition, e.g., a CDP-topoisomerase I or II inhibitor conjugate, particle or composition, e.g., a CDP-camptothecin or camptothecin derivative conjugate, particle or composition, e.g., a CDP-camptothecin derivative conjugate, particle or composition described herein, e.g., CRLX101, to said subject in combination with an angiogenesis inhibitor.

In one embodiment, the cancer is renal cancer.

In one embodiment, the conjugate, particle or composition is administered in combination with an angiogenesis inhibitor described herein such as a VEGF pathway inhibitor (e.g., a VEGF pathway inhibitor described herein). Exemplary angiogenesis inhibitors include the following: A6 (Angstrom Pharmacueticals), ABT-510 (Abbott Laboratories), ABT-627 (Atrasentan) (Abbott Laboratories/Xinlay), ABT-869 (Abbott Laboratories), Actimid (CC4047, Pomalidomide) (Celgene Corporation), AdGVPEDF.11D (GenVec), ADH-1 (Exherin) (Adherex Technologies), AEE788 (Novartis), AG-013736 (Axitinib) (Pfizer), AG3340 (Prinomastat) (Agouron Pharmaceuticals), AGX1053 (AngioGenex), AGX51 (AngioGenex), ALN-VSP (ALN-VSP O2) (Alnylam Pharmaceuticals), AMG 386 (Amgen), AMG706 (Amgen), Apatinib (YN968D1) (Jiangsu Hengrui Medicine), AP23573 (Ridaforolimus/MK8669) (Ariad Pharmaceuticals), AQ4N (Novavea), ARQ 197 (ArQule), ASA404 (Novartis/Antisoma), Atiprimod (Callisto Pharmaceuticals), ATN-161 (Attenuon), AV-412 (Aveo Pharmaceuticals), AV-951 (Aveo Pharmaceuticals), Avastin (Bevacizumab) (Genentech), AZD2171 (Cediranib/Recentin) (AstraZeneca), BAY 57-9352 (Telatinib) (Bayer), BEZ235 (Novartis), BIBF1120 (Boehringer Ingelheim Pharmaceuticals), BIBW 2992 (Boehringer Ingelheim Pharmaceuticals), BMS-275291 (Bristol-Myers Squibb), BMS-582664 (Brivanib) (Bristol-Myers Squibb), BMS-690514 (Bristol-Myers Squibb), Calcitriol, CCI-779 (Torisel) (Wyeth), CDP-791 (ImClone Systems), Ceflatonin (Homoharringtonine/HHT) (ChemGenex Therapeutics), Celebrex (Celecoxib) (Pfizer), CEP-7055 (Cephalon/Sanofi), CHIR-265 (Chiron Corporation), NGR-TNF, COL-3 (Metastat) (Collagenex Pharaceuticals), Combretastatin (Oxigene), CP-751,871(Figitumumab) (Pfizer), CP-547,632 (Pfizer), CS-7017 (Daiichi Sankyo Pharma), CT-322 (Angiocept) (Adnexus), Curcumin, Dalteparin (Fragmin) (Pfizer), Disulfiram (Antabuse), E7820 (Eisai Limited), E7080 (Eisai Limited), EMD 121974(Cilengitide) (EMD Pharmaceuticals), ENMD-1198 (EntreMed), ENMD-2076 (EntreMed), Endostar (Simcere), Erbitux (ImClone/Bristol-Myers Squibb), EZN-2208 (Enzon Pharmaceuticals), EZN-2968 (Enzon Pharmaceuticals), GC1008 (Genzyme), Genistein, GSK1363089(Foretinib) (GlaxoSmithKline), GW786034 (Pazopanib) (GlaxoSmithKline), GT-111 (Vascular Biogenics Ltd.), IMC--1121B (Ramucirumab) (ImClone Systems), IMC-18F1 (ImClone Systems), IMC-3G3 (ImClone LLC), INCB007839 (Incyte Corporation), INGN 241 (Introgen Therapeutics), Iressa (ZD1839/Gefitinib), LBH589 (Faridak/Panobinostst) (Novartis), Lucentis (Ranibizumab) (Genentech/Novartis), LY317615 (Enzastaurin) (Eli Lilly and Company), Macugen (Pegaptanib) (Pfizer), MEDI522 (Abegrin) (MedImmune), MLN518(Tandutinib) (Millennium), Neovastat (AE941/Benefin) (Aeterna Zentaris), Nexavar (Bayer/Onyx), NM-3 (Genzyme Corporation), Noscapine (Cougar Biotechnology), NPI-2358 (Nereus Pharmaceuticals), OSI-930 (OSI), Palomid 529 (Paloma Pharmaceuticals, Inc.), Panzem Capsules (2ME2) (EntreMed), Panzem NCD (2ME2) (EntreMed), PF-02341066 (Pfizer), PF-04554878 (Pfizer), PI-88 (Progen Industries/Medigen Biotechnology), PKC412 (Novartis), Polyphenon E (Green Tea Extract) (Polypheno E International, Inc), PPI-2458 (Praecis Pharmaceuticals), PTC299 (PTC Therapeutics), PTK787 (Vatalanib) (Novartis), PXD101 (Belinostat) (CuraGen Corporation), RAD001 (Everolimus) (Novartis), RAF265 (Novartis), Regorafenib (BAY73-4506) (Bayer), Revlimid (Celgene), Retaane (Alcon Research), SN38 (Liposomal) (Neopharm), SNS-032 (BMS-387032) (Sunesis), SOM230(Pasireotide) (Novartis), Squalamine (Genaera), Suramin, Sutent (Pfizer), Tarceva (Genentech), TB-403 (Thrombogenics), Tempostatin (Collard Biopharmaceuticals), Tetrathiomolybdate (Sigma-Aldrich), TG100801 (TargeGen), Thalidomide (Celgene Corporation), Tinzaparin Sodium, TKI258 (Novartis), TRC093 (Tracon Pharmaceuticals Inc.), VEGF Trap (Aflibercept) (Regeneron Pharmaceuticals), VEGF Trap-Eye (Regeneron Pharmaceuticals), Veglin (VasGene Therapeutics), Bortezomib (Millennium), XL184 (Exelixis), XL647 (Exelixis), XL784 (Exelixis), XL820 (Exelixis), XL999 (Exelixis), ZD6474 (AstraZeneca), Vorinostat (Merck), and ZSTK474.

In one embodiment, the conjugate, particle or composition is administered in combination with a treatment that reduces one or more side effect associated with the administration of a CDP-topoisomerase inhibitor conjugate, particle or composition, e.g., a treatment described herein.

In one aspect, the disclosure features, a method of treating a proliferative disorder, e.g., a cancer, in a subject, e.g., a human subject. The method comprises:
providing a subject who has a proliferative disorder, e.g., cancer;
administering a polysaccharide to said subject; and
administering a composition that comprises a CDP-topoisomerase inhibitor conjugate, particle or composition, e.g., a CDP-topoisomerase I or II inhibitor conjugate, particle or composition, e.g., a CDP-camptothecin or camptothecin derivative conjugate, particle or composition, e.g., a CDP-camptothecin derivative conjugate, particle or composition described herein, e.g., CRLX101, to said subject.

In one embodiment, the polysaccharide is a linear, branched or cyclic polysaccharide. In one embodiment, the polysaccharide is a linear polysaccharide that includes glucose molecules. In one embodiment, the polysaccharide is dextran, a cyclodextrin or a cyclodextrin derivative, e.g., an α-, β- and/or γ-cyclodextrin, e.g., CDP.

In one embodiment, the polysaccharide is administered prior to, currently with or after administration of the composition. In one embodiment, the polysaccharide is administered at a dose of 100 mg to 10 g.

In an embodiment, the conjugate includes a topoisomerase I inhibitor and/or a topoisomerase II inhibitor. In an embodiment, the conjugate includes a topoisomerase I inhibitor or combination of topoisomerase I inhibitors, e.g., camptothecin, irinotecan, SN-38, topotecan, lamellarin D and derivatives thereof. In an embodiment, the conjugate includes a topoisomerase II inhibitor or a combination of topoisomerase II inhibitors, e.g., eptoposide, tenoposide, doxorubicin and derivatives thereof. In one embodiment, the conjugate includes a combination of one or more topoisomerase I inhibitors and one or more topoisomerase II inhibitors. In an embodiment, the CDP-topoisomerase inhibitor conjugate is a CDP-camptothecin or camptothecin derivate conjugate, e.g., a CDP-camptothecin or camptothecin derivative conjugate described herein, e.g., CRLX101.

In one embodiment, the proliferative disorder is cancer, e.g., a cancer described herein.

In one embodiment, the CDP-topoisomerase inhibitor conjugate, particle or composition is administered in combination with one or more additional chemotherapeutic agent, e.g., as described herein. In one embodiment, the CDP-topoisomerase inhibitor conjugate, particle or composition is administered at a dose and/or dosing schedule described herein. In one embodiment, the subject is administered more than one dose of the CDP-topoisomerase inhibitor conjugate, particle or composition, e.g., as described herein, and the polysaccharide is administered prior to, currently with, or after one or more dose of the CDP-topoisomerase inhibitor conjugate, particle or composition.

In one aspect, the disclosure features, a method of treating a proliferative disorder, e.g., a cancer, in a subject, e.g., a human subject. The method comprises:
providing a subject who has a proliferative disorder, e.g., cancer;
administering an agent which ameliorates bladder toxicity associated with therapy, e.g., an agent which increases urinary excretion and/or neutralizes one or more urinary metabolite; and
administering a composition that comprises a camptothecin or camptothecin derivative, e.g., a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, to said subject.

In one embodiment, the agent which ameliorates bladder toxicity associated with therapy, e.g., the agent which increases urinary excretion and/or neutralizes one or more urinary metabolite, is administered prior to, concurrently with and/or after administration with the camptothecin or camptothecin derivative.

In one embodiment, the agent which ameliorates bladder toxicity associated with therapy is saline, e.g., intravenous saline, D5 half normal saline or D5 water. In one embodiment, the agent which increases urinary excretion and/or neutralizes one or more urinary metabolite is 2-mercaptoethane sulfonate sodium (MESNA). In one embodiment, the agent which ameliorates bladder toxicity associated with therapy is 2-mercaptoethane sulfonate sodium (MESNA) and the MESNA is administered intravenously at a dose of about 10%, 20%, 30% the dose of the camptothecin or camptothecin derivative and/or the MESNA is administered orally at a dose of about 20%, 30%, 40%, 50% the dose of the camptothecin or camptothecin derivative.

In an embodiment, the conjugate includes a topoisomerase I inhibitor and/or a topoisomerase II inhibitor. In an embodiment, the conjugate includes a topoisomerase I inhibitor or combination of topoisomerase I inhibitors, e.g., camptothecin, irinotecan, SN-38, topotecan, lamellarin D and derivatives thereof. In an embodiment, the conjugate includes a topoisomerase II inhibitor or a combination of topoisomerase II inhibitors, e.g., eptoposide, tenoposide, doxorubicin and derivatives thereof. In one embodiment, the conjugate includes a combination of one or more topoisomerase I inhibitors and one or more topoisomerase II inhibitors. In an embodiment, the CDP-topoisomerase inhibitor conjugate is a CDP-camptothecin or camptothecin derivate conjugate, e.g., a CDP-camptothecin or camptothecin derivative conjugate described herein, e.g., CRLX101.

In one embodiment, the proliferative disorder is cancer, e.g., a cancer described herein.

In one embodiment, the CDP-topoisomerase inhibitor conjugate, particle or composition, e.g., a CDP-topoisomerase inhibitor conjugate, particle or composition, described herein, e.g., a CDP-camptothecin or camptothecin derivative conjugate, particle or composition, e.g., a CDP-camptothecin or camptothecin derivative conjugate, particle or composition described herein, is administered in combination with one or more additional chemotherapeutic agent, e.g., as described herein. In one embodiment, the CDP-topoisomerase inhibitor conjugate, particle or composition is administered at a dose and/or dosing schedule described herein. In one embodiment, the subject is administered more than one dose of the CDP-topoisomerase inhibitor conjugate, particle or composition, e.g., as described herein, and the agent which ameliorates bladder toxicity associated with therapy is administered prior to one or more dose of the CDP-topoisomerase inhibitor conjugate, particle or composition.

In one embodiment, the CDP-topoisomerase inhibitor conjugate, particle or composition is further administered in combination with one or more of the agents described herein. For example, the CDP-topoisomerase inhibitor conjugate, particle or composition can be administered in combination with an agent which reduces or inhibits one or more symptom of hypersensitivity.

In one embodiment, the method includes selecting a subject who has a proliferative disorder, e.g., cancer, and has experienced cystitis, e.g., has experienced cystitis as a result of a previous chemotherapeutic treatment, for administration of an agent which ameliorates bladder toxicity associated with therapy and a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101.

In one aspect, the disclosure features, a method of treating a proliferative disorder, e.g., a cancer, in a subject, e.g., a human subject. The method comprises:
providing a subject who has a proliferative disorder, e.g., cancer, and has been administered an agent which reduces or inhibits one or more symptom of hypersensitivity; and
administering a composition that comprises a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, to said subject.

In one embodiment, the method further comprises administering the agent which reduces or inhibits one or more symptom of hypersensitivity to the subject.

In one embodiment, the agent which reduces or inhibits one or more symptoms of hypersensitivity can be one or more of a corticosteroid (e.g., dexamethasone), an antihistamine (e.g., diphenhydramine), an H1 antagonist and an H2 antagonist (e.g., ranitidine or famotidine). In one embodiment, the agent is a corticosteroid (e.g., dexamethasone) and the corticosteroid is administered at 5, 10, 15, 20, 25 or 30 mg. In one embodiment, the corticosteroid is administered about 12, 11, 10, 9, 8, 7, 6, 5, 4, and/or 3 hours before administration of the CDP-topoisomerase inhibitor conjugate, particle or composition, or the corticosteroid is administered intravenously about 40, 30, 20 minutes before the CDP-topoisomerase inhibitor conjugate, particle or composition. In one embodiment, the agent is an antihistamine (e.g., diphenhydramine) and the antihistamine is administered at 15, 20, 25, 30, 35, 40, 45, 50, 55, 60, 65 or 70 mg. In one embodiment, the antihistamine is administered intravenously about 40, 30, 20, 10 minutes before the CDP-topoisomerase inhibitor conjugate, particle or composition. In one embodiment, the agent is an H2 antagonist (e.g., ranitidine or famotidine) and the H2 antagonist is administered at 10, 15, 20, 25, 30, 35, 40, 45, 50, 55, 60, 65 or 70 mg. In one embodiment the H2 antagonist is administered intravenously about 70, 60, 50, 40, 30, 20, 10 minutes before the CDP-topoisomerase inhibitor conjugate, particle or composition.

In an embodiment, the conjugate includes a topoisomerase I inhibitor and/or a topoisomerase II inhibitor. In an embodiment, the conjugate includes a topoisomerase I inhibitor or combination of topoisomerase I inhibitors, e.g., camptothecin, irinotecan, SN-38, topotecan, lamellarin D and derivatives thereof. In an embodiment, the conjugate includes a topoisomerase II inhibitor or a combination of topoisomerase II inhibitors, e.g., eptoposide, tenoposide, doxorubicin and derivatives thereof. In one embodiment, the conjugate includes a combination of one or more topoisomerase I inhibitors and one or more topoisomerase II inhibitors. In an embodiment, the CDP-topoisomerase inhibitor conjugate is a CDP-camptothecin or camptothecin derivate conjugate, e.g., a CDP-camptothecin or camptothecin derivative conjugate described herein, e.g., CRLX101.

In one embodiment, the proliferative disorder is cancer, e.g., a cancer described herein.

In one embodiment, the CDP-topoisomerase inhibitor conjugate, particle or composition is administered in combination with one or more additional chemotherapeutic agent, e.g., as described herein. In one embodiment, the CDP-topoisomerase inhibitor conjugate, particle or composition is administered at a dose and/or dosing schedule described herein. In one embodiment, the subject is administered more than one dose of the CDP-topoisomerase inhibitor conjugate, particle or composition, e.g., as described herein, and the agent which reduces or inhibits one or more symptom of hypersensitivity is administered prior to each dose of the CDP-topoisomerase inhibitor conjugate, particle or composition.

In one embodiment, the method includes selecting a subject who has a proliferative disorder, e.g., cancer, and has experienced one or more symptom of hypersensitivity, e.g., has experienced one or more symptom of hypersensitivity to a previous chemotherapeutic treatment, for administration of an agent which reduces or inhibits one or more symptom of hypersensitivity and a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101. Symptoms of hypersensitivity include: injection site reaction, dyspnea, hypotension, angioedema, urticaria, bronchospasm and erythema.

In yet another aspect, the disclosure features a method of treating a subject, e.g., a human subject, with a proliferative disorder, e.g., cancer, comprising:
selecting a subject who has a proliferative disorder, e.g., cancer, that has increased KRAS and/or ST expression levels, e.g., as compared to a reference standard and/or a mutation in a KRAS gene and/or ST gene; and
administering a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, to the subject in an amount effective to treat the cancer, to thereby treat the cancer.

In an embodiment, the conjugate includes a topoisomerase I inhibitor and/or a topoisomerase II inhibitor. In an embodiment, the conjugate includes a topoisomerase I inhibitor or combination of topoisomerase I inhibitors, e.g., camptothecin, irinotecan, SN-38, topotecan, lamellarin D and derivatives thereof. In an embodiment, the conjugate includes a topoisomerase II inhibitor or a combination of topoisomerase II inhibitors, e.g., eptoposide, tenoposide, doxorubicin and derivatives thereof. In one embodiment, the conjugate includes a combination of one or more topoisomerase I inhibitors and one or more topoisomerase II inhibitors. In an embodiment, the CDP-topoisomerase inhibitor conjugate is a CDP-camptothecin or camptothecin derivate conjugate, e.g., a CDP-camptothecin or camptothecin derivative conjugate described herein, e.g., CRLX101.

In one embodiment, the subject has increased KRAS and/or ST expression levels, e.g., as compared to a reference standard, and/or has a mutation in a KRAS and/or ST gene. In one embodiment, the subject has a mutation at one or more of: codon 12 of the KRAS gene (e.g., a G to T transversion), codon 13 of the KRAS gene, codon 61 of the KRAS gene. In one embodiment, the subject has lung cancer (e.g., small cell lung cancer and/or non-small cell lung cancer), pancreatic cancer or colorectal cancer.

In one embodiment, the cancer is a cancer described herein.

In one embodiment, the CDP-topoisomerase inhibitor conjugate, particle or composition is administered in combination with one or more additional chemotherapeutic agent, e.g., as described herein. In one embodiment, the CDP-topoisomerase inhibitor conjugate, particle or composition is administered at a dose and/or dosing schedule described herein.

In one embodiment, the CDP-topoisomerase inhibitor conjugate, particle or composition is administered in combination with one or more of the agents described herein. For example, the CDP-topoisomerase inhibitor conjugate, particle or composition can be administered in combination with an agent which reduces or inhibits one or more symptom of hypersensitivity and/or an agent which increases urinary excretion and/or neutralizes one or more urinary metabolite.

In another aspect, the disclosure features, a unit dosage of a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101.

In one aspect, the disclosure features a CDP-topoisomerase inhibitor conjugate, particle or composition, e.g., a CDP- camptothecin derivative conjugate, particle or composition, e.g., a CDP- camptothecin derivative conjugate, particle or composition described herein, and methods of making the CDP-topoisomerase inhibitor conjugates, particles and compositions, e.g., a CDP- camptothecin derivative conjugate, particle or composition, e.g., a CDP-camptothecin derivative conjugate, particle or composition described herein.

In an embodiment, the conjugate includes a topoisomerase I inhibitor and/or a topoisomerase II inhibitor. In an embodiment, the conjugate includes a topoisomerase I inhibitor or combination of topoisomerase I inhibitors, e.g., irinotecan, SN-38, topotecan, lamellarin D and derivatives thereof. In an embodiment, the conjugate includes a topoisomerase II inhibitor or a combination of topoisomerase II inhibitors, e.g., eptoposide, tenoposide, doxorubicin and derivatives thereof. In one embodiment, the conjugate includes a combination of one or more topoisomerase I inhibitors and one or more topoisomerase II inhibitors.

In one embodiment, CDP is not biodegradable.

In one embodiment, CDP is biocompatible.

In one embodiment, the CDP-topoisomerase inhibitor conjugate, particle or composition, e.g., a CDP-camptothecin derivative conjugate particle or composition, e.g., a CDP-camptothecin derivative described herein, includes an inclusion complex between a topoisomerase inhibitor, e.g., a camptothecin derivative, attached or conjugated to the CDP, e.g., via a covalent linkage, and another molecule in the CDP. In one embodiment, the CDP-topoisomerase inhibitor conjugate forms a nanoparticle. In one embodiment, the CDP-topoisomerase inhibitor conjugate including an inclusion complex forms a nanoparticle. The nanoparticle ranges in size from 10 to 300 nm in diameter, e.g., 20 to 280, 30 to 250, 30 to 200, 20 to 150, 30 to 100, 20 to 80, 30 to 70, 30 to 60 or 30 to 50 nm diameter. In one embodiment, the nanoparticle is 30 to 60 nm in diameter. In one embodiment, the composition comprises a population or a plurality of nanoparticles with an average diameter from 10 to 300 nm, e.g., 20 to 280, 30 to 250, 30 to 200, 20 to 150, 30 to 100, 20 to 80, 30 to 70, 30 to 60 or 30 to 50 nm. In one embodiment, the average nanoparticle diameter is from 30 to 60 nm. In one embodiment, the surface charge of the molecule is neutral, or slightly negative. In some embodiments, the zeta potential of the particle surface is from about -80 mV to about 50 mV, about -20 mV to about 20 mV, about -20 mV to about -10 mV, or about -10 mV to about 0.

In one embodiment, the topoisomerase inhibitor (e.g., a camptothecin derivative, e.g., a camptothecin derivative described herein), conjugated to the CDP is more soluble when conjugated to the CDP, than when not conjugated to the CDP.

In one embodiment, the composition comprises a population, mixture or plurality of CDP-topoisomerase inhibitor conjugates. In one embodiment, the population, mixture or plurality of CDP-topoisomerase inhibitor conjugates comprises a plurality of different topoisomerase inhibitors conjugated to a CDP (e.g., two different topoisomerase inhibitors are in the composition such that two different topoisomerase inhibitors are attached to a single CDP; or a first topoisomerase inhibitor is attached to a first CDP and a second topoisomerase inhibitor is attached to a second CDP and both CDP-topoisomerase inhibitor conjugates are present in the composition).

The details of one or more embodiments of the disclosure, including embodiments of the invention, are set forth in the description below. Other features, objects, and advantages will be apparent from the description and the drawings, and from the claims.

### Brief Description of the Drawings

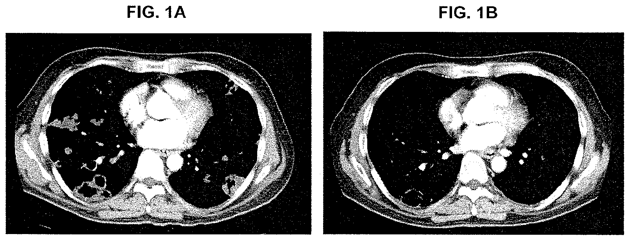

FIGs. 1A and 1B are CT (computed tomography) scans from a patient with metastatic pancreatic cancer pre-treatment (FIG. 1A), and after six months of treatment with CRLX101 (FIG. IB). The patient received 6 mg/m² CRLX101 on schedule Ia.

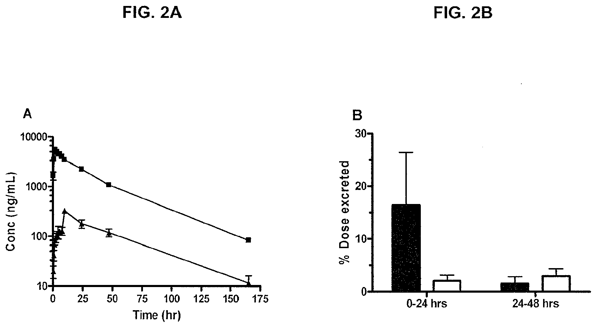

FIGs. 2A and 2B are graphs depicting pharmacokinetic and toxicokinetic analysis of CRLX101 delivered by intravenous administration. FIG. 2A shows mean plasma concentration-time profile for polymer conjugated (squares) and unconjugated (triangles) CPT for cohort 1b-1 (12 mg/m²). FIG. 2B shows average urinary excretion of polymer conjugated (black bars) and unconjugated CPT (white bars) in the first 48 hours following CRLX101 administration. Plasma concentrations for conjugated and unconjugated CPT were below the limit of quantitation at 336 hrs (before the second dose) and therefore are not plotted in FIG. 2A.

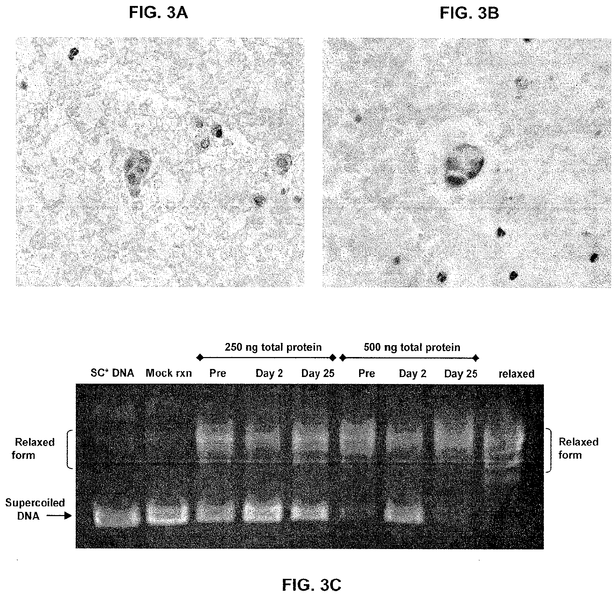

FIGs. 3A-3C depict immunohistochemistry and topoisomerase I activity of ovarian cancer cells from a patient treated with CRLX101, showing post-treatment reduction of topoisomerase I protein. FIG. 3A is an immunohistochemical stain of ascites cells collected before CRLX101 was given. FIG.3B is an immunohistochemical stain of ascites cells collected 2 days following CRLX101 treatment. FIG. 3C is a gel depicting the results of topoisomerase I enzymatic activity assay in whole cell lysates.

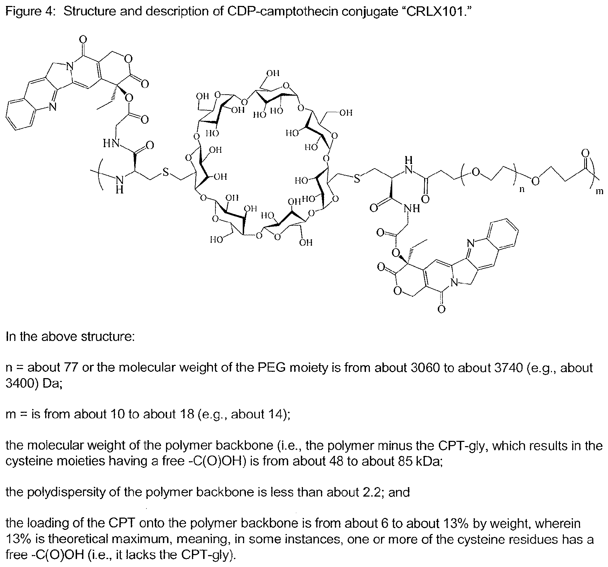

FIG. 4 depicts the structure and description of an exemplary CDP-camptothecin conjugate referred to as "CRLX101" throughout this application. CRLX101 is used interchangeably with the term CRLX101 (e.g., as in Example 1).

### Detailed Description of the Invention

The present disclosure relates to compositions of therapeutic cyclodextrin-containing polymers (CDP) designed for drug delivery of a topoisomerase inhibitor such as camptothecin or a camptothecin derivative. In certain embodiments, these cyclodextrin-containing polymers improve drug stability and/or solubility, and/or reduce toxicity, and/or improve efficacy of the topoisomerase inhibitor when used *in vivo.*

Furthermore, by selecting from a variety of linker groups that link or couple CDP to a topoisomerase inhibitor such as camptothecin or a camptothecin derivative, and/or targeting ligands, the rate of drug release from the polymers can be attenuated for controlled delivery. The disclosure also relates to methods of treating subjects with compositions described herein. The disclosure further relates to methods for conducting a pharmaceutical business comprising manufacturing, licensing, or distributing kits containing or relating to the CDP-topoisomerase inhibitor conjugates, particles and compositions described herein.

More generally, the present disclosure provides water-soluble, biocompatible polymer conjugates comprising a water-soluble, biocompatible polymer covalently attached to the topoisomerase inhibitor through attachments that are cleaved under biological conditions to release the topoisomerase inhibitor.

Polymeric conjugates featured in the methods described herein may be useful to improve solubility and/or stability of a bioactive/therapeutic agent, such as camptothecin, reduce drug-drug interactions, reduce interactions with blood elements including plasma proteins, reduce or eliminate immunogenicity, protect the agent from metabolism, modulate drug-release kinetics, improve circulation time, improve drug half-life (e.g., in the serum, or in selected tissues, such as tumors), attenuate toxicity, improve efficacy, normalize drug metabolism across subjects of different species, ethnicities, and/or races, and/or provide for targeted delivery into specific cells or tissues.

In preferred embodiments, the topoisomerase inhibitor in the CDP-topoisomerase inhibitor conjugate, particle or composition is camptothecin or a camptothecin derivative. The term "camptothecin derivative", as used herein, includes camptothecin analogues and metabolites of camptothecin. For example, camptothecin derivatives can have the following structure: 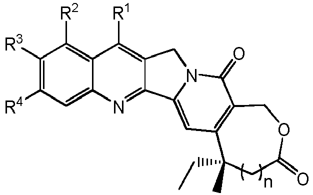 wherein
R¹ is H, OH, optionally substituted alkyl (e.g., optionally substituted with NR^{a}₂ or ORₐ, or SiR^{a}₃), or SiR^{a}₃; or R¹ and R² may be taken together to form an optionally substituted 5- to 8-membered ring (e.g., optionally substituted with NR^{a}₂ or OR^{a});
R² is H, OH, NH₂, halo, nitro, optionally substituted alkyl (e.g., optionally substituted with NR^{a}₂ or OR^{a}, NR^{a}₂, OC(=O)NR^{a}₂, or OC(=O)OR^{a});
R³ is H, OH, NH₂, halo, nitro, NR^{a}₂, OC(=O)NR^{a}₂, or OC(=O)OR^{a}
R⁴ is H, OH, NH₂, halo, CN, or NR^{a}₂; or R³ and R⁴ taken together with the atoms to which they are attached form a 5- or 6-membered ring (e.g. forming a ring including -OCH₂O- or -OCH₂CH₂O-);
each R^{a} is independently H or alkyl; or two R^{a}s, taken together with the atom to which they are attached, form a 4- to 8-membered ring (e.g., optionally containing an O or NR^{b})
R_{b} is H or optionally substituted alkyl (e.g., optionally substituted with OR^{c} or NR^{c}₂);
R^{c} is H or alkyl; or, two R^{c}s, taken together with the atom to which they are attached, form a 4- to 8-membered ring; and
n = 0 or 1.

In some embodiments, the camptothecin or camptothecin derivative is the compound as provided below.

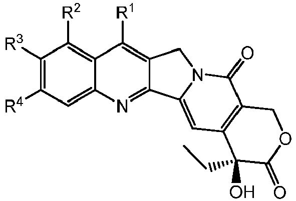

In one embodiment, R¹, R², R³ and R⁴ of the camptothecin derivative are each H, and n is 0.

In one embodiment, R¹, R², R³ and R⁴ of the camptothecin derivative are each H, and n is 1.

In one embodiment, R¹ of the camptothecin derivative is H, R² is-CH₂N(CH₃)₂, R³ is -OH, R⁴ is H; and n is 0.

In one embodiment, R¹ of the camptothecin derivative is -CH₂CH₃, R² is H, R³ is:

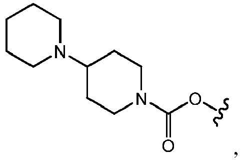

R⁴ is H, and n is 0.

In one embodiment, R¹ of the camptothecin derivative is -CH₂CH₃, R² is H, R³ is -OH, R⁴ is H, and n is 0.

In one embodiment, R¹ of the camptothecin derivative is *tert-*butyldimethylsilyl, R² is H, R³ is -OH and R⁴ is H, and n is 0.

In one embodiment, R¹ of the camptothecin derivative is *tert-*butyldimethylsilyl, R² is hydrogen, R³ is -OH and R⁴ is hydrogen, and n is 1.

In one embodiment, R¹ of the camptothecin derivative is *tert-*butyldimethylsilyl, R², R³ and R⁴ are each H, and n is 0.

In one embodiment, R¹ of the camptothecin derivative is *tert-*butyldimethylsilyl, R², R³ and R⁴ are each H, and n is 1.

In one embodiment, R¹ of the camptothecin derivative is -CH₂CH₂Si(CH₃)₃ and R², R³ and R⁴ are each H.

In one embodiment, R¹ and R² of the camptothecin derivative are taken together with the carbons to which they are attached to form an optionally substituted ring. In one embodiment, R¹ and R² of the camptothecin derivative are taken together with the carbons to which they are attached to form a substituted 6-membered ring. In one embodiment, the camptothecin derivative has the following formula: 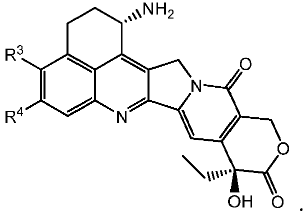 In one embodiment, R³ is methyl and R⁴ is fluoro.

In one embodiment, R³ and R⁴ are taken together with the carbons to which they are attached to form an optionally substituted ring. In one embodiment, R³ and R⁴ are taken together with the carbons to which they are attached to form a 6-membered heterocyclic ring. In one embodiment, the camptothecin derivative has the following formula: 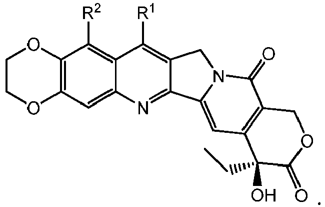 In one embodiment, R¹ is: 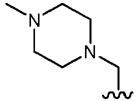 and R² is hydrogen.

In one embodiment, the camptothecin derivative has the following formula: 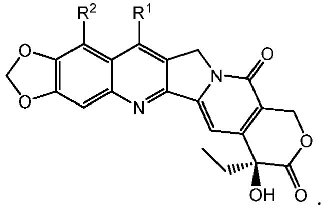 In one embodiment, R¹ is: 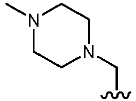 and R² is hydrogen.

In one embodiment, R¹ is: 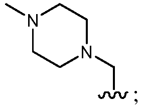 R² is H, R³ is methyl, R⁴ is chloro; and n is 1.

In one embodiment, R¹ is -CH=NOC(CH₃)₃, R², R³ and R⁴ are each H, and n is 0.

In one embodiment, R¹ is -CH₂CH₂NHCH(CH₃)₂, R², R³ and R⁴ are each H; and n is 0.

In one embodiment, R¹ and R² are H, R³ and R⁴ are fluoro, and n is 1.

In one embodiment, each of R¹, R³, and R⁴ is H, R² is NH₂, and n is 0.

In one embodiment, each of R¹, R³, and R⁴ is H, R² is NO₂, and n is 0.

An "effective amount" or "an amount effective" refers to an amount of the CDP-topoisomerase inhibitor conjugate, particle or composition which is effective, upon single or multiple dose administrations to a subject, in treating a cell, or curing, alleviating, relieving or improving a symptom of a disorder. An effective amount of the conjugate, particle or composition may vary according to factors such as the disease state, age, sex, and weight of the individual, and the ability of the compound to elicit a desired response in the individual. An effective amount is also one in which any toxic or detrimental effects of the conjugate, particle or composition is outweighed by the therapeutically beneficial effects.

As used herein, the term "subject" is intended to include human and non-human animals. Exemplary human subjects include a human patient having a disorder, e.g., a disorder described herein, or a normal subject. The term "non-human animals" includes all vertebrates, e.g., non-mammals (such as chickens, amphibians, reptiles) and mammals, such as non-human primates, domesticated and/or agriculturally useful animals, e.g., sheep, dog, cat, cow, pig, etc.

As used herein, the term "treat" or "treating" a subject having a disorder refers to subjecting the subject to a regimen, e.g., the administration of a CDP-topoisomerase inhibitor conjugate, particle or composition, such that at least one symptom of the disorder is cured, healed, alleviated, relieved, altered, remedied, ameliorated, or improved. Treating includes administering an amount effective to alleviate, relieve, alter, remedy, ameliorate, improve or affect the disorder or the symptoms of the disorder. The treatment may inhibit deterioration or worsening of a symptom of a disorder.

An amount of a CDP-topoisomerase inhibitor conjugate, particle or composition effective to prevent a disorder, or "a prophylactically effective amount" of the conjugate, particle or composition as used in the context of the administration of an agent to a subject, refers to subjecting the subject to a regimen, e.g., the administration of a CDP-topoisomerase inhibitor conjugate, particle or composition such that the onset of at least one symptom of the disorder is delayed as compared to what would be seen in the absence of the regimen.

### CDP-Topoisomerase inhibitor conjugates, particles and compositions

Described herein are cyclodextrin containing polymer ("CDP")-topoisomerase inhibitor conjugates, wherein one or more topoisomerase inhibitors are covalently attached to the CDP (e.g., either directly or through a linker). The CDP-topoisomerase inhibitor conjugates include linear or branched cyclodextrin-containing polymers and polymers grafted with cyclodextrin. Exemplary cyclodextrin-containing polymers that may be modified as described herein are taught in U.S. Patent Nos. 7,270,808, 6,509,323, 7,091,192, 6,884,789, U.S. Publication Nos. 20040087024, 20040109888 and 20070025952.

Accordingly, in one embodiment the CDP-topoisomerase inhibitor conjugate is represented by Formula I: 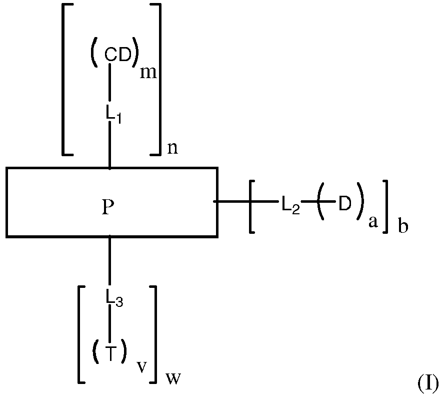 wherein
P represents a linear or branched polymer chain;
CD represents a cyclic moiety such as a cyclodextrin moiety;
L₁, L₂ and L₃, independently for each occurrence, may be absent or represent a linker group;
D, independently for each occurrence, represents a topoisomerase inhibitor or a prodrug thereof (e.g., a camptothecin or camptothecin derivative);
T, independently for each occurrence, represents a targeting ligand or precursor thereof;
a, m, and v, independently for each occurrence, represent integers in the range of 1 to 10 (preferably 1 to 8, 1 to 5, or even 1 to 3);
n and w, independently for each occurrence, represent an integer in the range of 0 to about 30,000 (preferably <25,000, <20,000, <15,000, <10,000, <5,000, <1,000, <500, <100, <50, <25, <10, or even <5); and
b represents an integer in the range of 1 to about 30,000 (preferably <25,000, <20,000, <15,000, <10,000, <5,000, <1,000, <500, <100, <50, <25, <10, or even <5),
wherein either P comprises cyclodextrin moieties or n is at least 1.

In some embodiments, one or more of the topoisomerase inhibitor moieties in the CDP-topoisomerase inhibitor conjugate can be replaced with another therapeutic agent, e.g., another anticancer agent or anti-inflammatory agent. Examples of other anticancer agents are described herein. Examples of anti-inflammatory agents include a steroid, e.g., prednisone, and a NSAID.

In certain embodiments, P contains a plurality of cyclodextrin moieties within the polymer chain as opposed to the cyclodextrin moieties being grafted on to pendant groups off of the polymeric chain. Thus, in certain embodiments, the polymer chain of formula I further comprises n' units of U, wherein n' represents an integer in the range of 1 to about 30,000, e.g., from 4-100, 4-50, 4-25, 4-15, 6-100, 6-50, 6-25, and 6-15 (preferably <25,000, <20,000, <15,000, <10,000, <5,000, <1,000, <500, <100, <50, <25, <20, <15, <10, or even <5); and U is represented by one of the general formulae below: 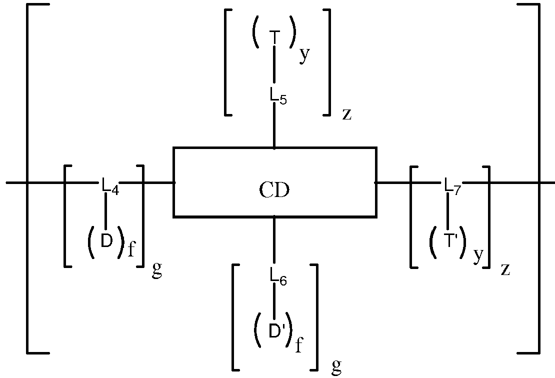 or 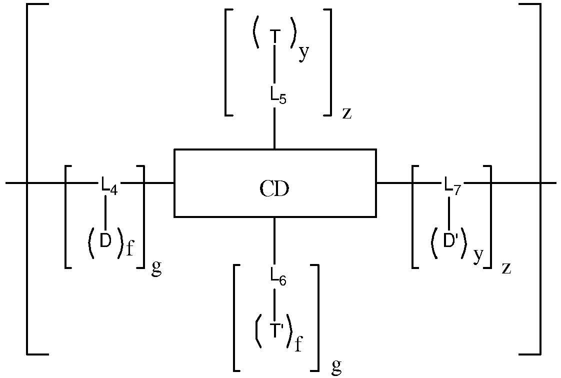 wherein
CD represents a cyclic moiety, such as a cyclodextrin moiety, or derivative thereof;
L₄, L₅, L₆, and L₇, independently for each occurrence, may be absent or represent a linker group;
D and D', independently for each occurrence, represent the same or different topoisomerase inhibitor or prodrug forms thereof (e.g., a camptothecin or camptothecin derivative);
T and T', independently for each occurrence, represent the same or different targeting ligand or precursor thereof;
f and y, independently for each occurrence, represent an integer in the range of 1 and 10; and
g and z, independently for each occurrence, represent an integer in the range of 0 and 10.

Preferably the polymer has a plurality of D or D' moieties. In some embodiments, at least 50% of the U units have at least one D or D'. In some embodiments, one or more of the topoisomerase inhibitor moieties in the CDP-topoisomerase conjugate can be replaced with another therapeutic agent, e.g., another anticancer agent or anti-inflammatory agent.

In preferred embodiments, L₄ and L₇ represent linker groups.

The CDP may include a polycation, polyanion, or non-ionic polymer. A polycationic or polyanionic polymer has at least one site that bears a positive or negative charge, respectively. In certain such embodiments, at least one of the linker moiety and the cyclic moiety comprises such a charged site, so that every occurrence of that moiety includes a charged site. In some embodiments, the CDP is biocompatible.

In certain embodiments, the CDP may include polysaccharides, and other non-protein biocompatible polymers, and combinations thereof, that contain at least one terminal hydroxyl group, such as polyvinylpyrrollidone, poly(oxyethylene)glycol (PEG), polysuccinic anhydride, polysebacic acid, PEG-phosphate, polyglutamate, polyethylenimine, maleic anhydride divinylether (DIVMA), cellulose, pullulans, inulin, polyvinyl alcohol (PVA), N-(2-hydroxypropyl)methacrylamide (HPMA), dextran and hydroxyethyl starch (HES), and have optional pendant groups for grafting therapeutic agents, targeting ligands and/or cyclodextrin moieties. In certain embodiments, the polymer may be biodegradable such as poly(lactic acid), poly(glycolic acid), poly(alkyl 2-cyanoacrylates), polyanhydrides, and polyorthoesters, or bioerodible such as polylactide-glycolide copolymers, and derivatives thereof, non-peptide polyaminoacids, polyiminocarbonates, poly alpha-amino acids, polyalkyl-cyano-acrylate, polyphosphazenes or acyloxymethyl poly aspartate and polyglutamate copolymers and mixtures thereof.

In another embodiment the CDP-topoisomerase inhibitor conjugate is represented by Formula II: 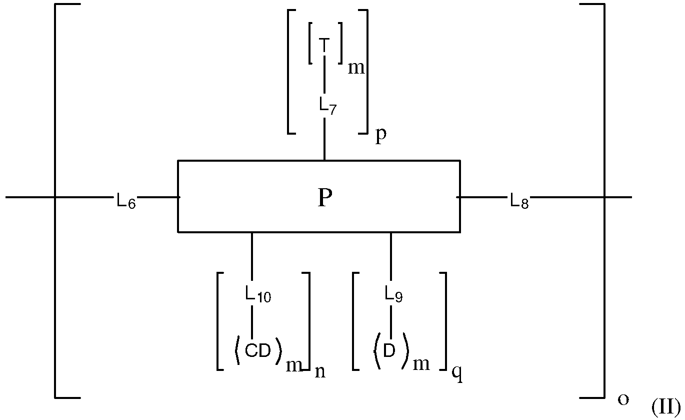 wherein
P represents a monomer unit of a polymer that comprises cyclodextrin moieties;
T, independently for each occurrence, represents a targeting ligand or a precursor thereof;
L₆, L₇, L₈, L₉, and L₁₀, independently for each occurrence, may be absent or represent a linker group;
CD, independently for each occurrence, represents a cyclodextrin moiety or a derivative thereof;
D, independently for each occurrence, represents a topoisomerase inhibitor or a prodrug form thereof (e.g., a camptothecin or camptothecin derivative);
m, independently for each occurrence, represents an integer in the range of 1 to 10 (preferably 1 to 8, 1 to 5, or even 1 to 3);
o represents an integer in the range of 1 to about 30,000 (preferably <25,000, <20,000, <15,000, <10,000, <5,000, <1,000, <500, <100, <50, <25, <10, or even <5); and
p, n, and q, independently for each occurrence, represent an integer in the range of 0 to 10 (preferably 0 to 8, 0 to 5, 0 to 3, or even 0 to about 2),
wherein CD and D are preferably each present at least 1 location (preferably at least 5, 10, 25, or even 50 or 100 locations) in the compound.

In some embodiments, one or more of the topoisomerase inhibitor moieties in the CDP-topoisomerase inhibitor conjugate can be replaced with another therapeutic agent, e.g., another anticancer agent or anti-inflammatory agent. Examples of an anticancer agent are described herein. Examples of anti-inflammatory agents include a steroid, e.g., prednisone, or a NSAID.

In another embodiment the CDP-topoisomerase inhibitor conjugate is represented either of the formulae below: 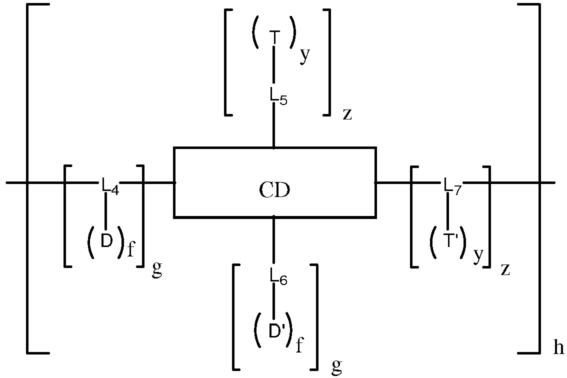 or 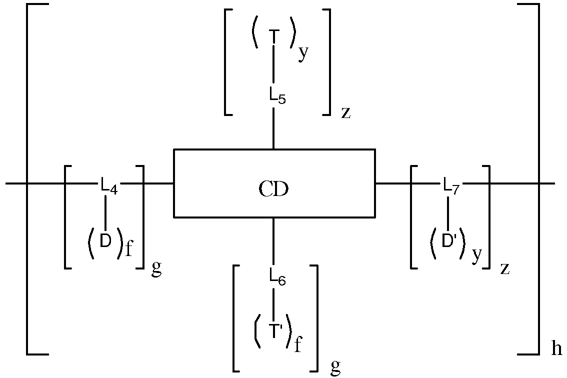 wherein
CD represents a cyclic moiety, such as a cyclodextrin moiety, or derivative thereof;
L₄, L₅, L₆, and L₇, independently for each occurrence, may be absent or represent a linker group;
D and D', independently for each occurrence, represent the same or different topoisomerase inhibitor or prodrug thereof (e.g., a camptothecin or camptothecin derivative);
T and T', independently for each occurrence, represent the same or different targeting ligand or precursor thereof;
f and y, independently for each occurrence, represent an integer in the range of 1 and 10 (preferably 1 to 8, 1 to 5, or even 1 to 3);
g and z, independently for each occurrence, represent an integer in the range of 0 and 10 (preferably 0 to 8, 0 to 5, 0 to 3, or even 0 to about 2); and
h represents an integer in the range of 1 and 30,000 , e.g., from 4-100, 4-50, 4-25, 4-15, 6-100, 6-50, 6-25, and 6-15 (preferably <25,000, <20,000, <15,000, <10,000, <5,000, <1,000, <500, <100, <50, <25, <20, <15, <10, or even <5),
wherein at least one occurrence (and preferably at least 5, 10, or even at least 20, 50, or 100 occurrences) of g represents an integer greater than 0.

Preferably the polymer has a plurality of D or D' moieties. In some embodiments, at least 50% of the polymer repeating units have at least one D or D'. In some embodiments, one or more of the topoisomerase inhibitor moieties in the CDP-topoisomerase inhibitor conjugate can be replaced with another therapeutic agent, e.g., another anticancer agent or anti-inflammatory agent.

In preferred embodiments, L4 and L7 represent linker groups.

In certain such embodiments, the CDP comprises cyclic moieties alternating with linker moieties that connect the cyclic structures, e.g., into linear or branched polymers, preferably linear polymers. The cyclic moieties may be any suitable cyclic structures, such as cyclodextrins, crown ethers (e.g., 18-crown-6, 15-crown-5, 12-crown-4, etc.), cyclic oligopeptides (e.g., comprising from 5 to 10 amino acid residues), cryptands or cryptates (e.g., cryptand [2.2.2], cryptand-2,1,1, and complexes thereof), calixarenes, or cavitands, or any combination thereof. Preferably, the cyclic structure is (or is modified to be) water-soluble. In certain embodiments, e.g., for the preparation of a linear polymer, the cyclic structure is selected such that under polymerization conditions, exactly two moieties of each cyclic structure are reactive with the linker moieties, such that the resulting polymer comprises (or consists essentially of) an alternating series of cyclic moieties and linker moieties, such as at least four of each type of moiety. Suitable difunctionalized cyclic moieties include many that are commercially available and/or amenable to preparation using published protocols. In certain embodiments, conjugates are soluble in water to a concentration of at least 0.1 g/mL, preferably at least 0.25 g/mL.

Thus, in certain embodiments, the disclosure relates to novel compositions of therapeutic cyclodextrin-containing polymeric compounds designed for drug delivery of a topoisomerase inhibitor. In certain embodiments, these CDPs improve drug stability and/or solubility, and/or reduce toxicity, and/or improve efficacy of the topoisomerase inhibitor when used *in vivo.* Furthermore, by selecting from a variety of linker groups, and/or targeting ligands, the rate of topoisomerase inhibitor release from the CDP can be attenuated for controlled delivery.

In certain embodiments, the CDP comprises a linear cyclodextrin-containing polymer, e.g., the polymer backbone includes cyclodextrin moieties. For example, the polymer may be a water-soluble, linear cyclodextrin polymer produced by providing at least one cyclodextrin derivative modified to bear one reactive site at each of exactly two positions, and reacting the cyclodextrin derivative with a linker having exactly two reactive moieties capable of forming a covalent bond with the reactive sites under polymerization conditions that promote reaction of the reactive sites with the reactive moieties to form covalent bonds between the linker and the cyclodextrin derivative, whereby a linear polymer comprising alternating units of cyclodextrin derivatives and linkers is produced. Alternatively the polymer may be a water-soluble, linear cyclodextrin polymer having a linear polymer backbone, which polymer comprises a plurality of substituted or unsubstituted cyclodextrin moieties and linker moieties in the linear polymer backbone, wherein each of the cyclodextrin moieties, other than a cyclodextrin moiety at the terminus of a polymer chain, is attached to two of said linker moieties, each linker moiety covalently linking two cyclodextrin moieties. In yet another embodiment, the polymer is a water-soluble, linear cyclodextrin polymer comprising a plurality of cyclodextrin moieties covalently linked together by a plurality of linker moieties, wherein each cyclodextrin moiety, other than a cyclodextrin moiety at the terminus of a polymer chain, is attached to two linker moieties to form a linear cyclodextrin polymer.

In some embodiments, the CDP-topoisomerase inhibitor conjugate comprises a water soluble linear polymer conjugate comprising: cyclodextrin moieties; comonomers which do not contain cyclodextrin moieties (comonomers); and a plurality of topoisomerase inhibitor; wherein the CDP-topoisomerase inhibitor conjugate comprises at least four, five six, seven, eight, etc., cyclodextrin moieties and at least four, five six, seven, eight, etc., comonomers. In some embodiments, the topoisomerase inhibitor is a topoisomerase inhibitor described herein, for example, the topoisomerase inhibitor is a camptothecin or camptothecin derivative described herein. The topoisomerase inhibitor can be attached to the CDP via a functional group such as a hydroxyl group, or where appropriate, an amino group.

In some embodiments, one or more of the topoisomerase inhibitor moieties in the CDP-topoisomerase inhibitor conjugate can be replaced with another therapeutic agent, e.g., another anticancer agent or anti-inflammatory agent.

In some embodiments, the least four cyclodextrin moieties and at least four comonomers alternate in the CDP-topoisomerase inhibitor conjugate. In some embodiments, the topoisomerase inhibitors are cleaved from said CDP-topoisomerase inhibitor conjugate under biological conditions to release the topoisomerase inhibitor. In some embodiments, the cyclodextrin moieties comprise linkers to which topoisomerase inhibitors are linked. In some embodiments, the topoisomerase inhibitors are attached via linkers.

In some embodiments, the comonomer comprises residues of at least two functional groups through which reaction and linkage of the cyclodextrin monomers was achieved. In some embodiments, the functional groups, which may be the same or different, terminal or internal, of each comonomer comprise an amino, acid, imidazole, hydroxyl, thio, acyl halide, -HC=CH-, -C≡C- group, or derivative thereof. In some embodiments, the two functional groups are the same and are located at termini of the comonomer precursor. In some embodiments, a comonomer contains one or more pendant groups with at least one functional group through which reaction and thus linkage of a topoisomerase inhibitor was achieved. In some embodiments, the functional groups, which may be the same or different, terminal or internal, of each comonomer pendant group comprise an amino, acid, imidazole, hydroxyl, thiol, acyl halide, ethylene, ethyne group, or derivative thereof. In some embodiments, the pendant group is a substituted or unsubstituted branched, cyclic or straight chain C1-C10 alkyl, or arylalkyl optionally containing one or more heteroatoms within the chain or ring. In some embodiments, the cyclodextrin moiety comprises an alpha, beta, or gamma cyclodextrin moiety. In some embodiments, the topoisomerase inhibitor is at least 5%, 10%, 15%, 20%, 25%, 30%, or 35% by weight of CDP-topoisomerase inhibitor conjugate.

In some embodiments, the comonomer comprises polyethylene glycol of molecular weight 3,400 Da, the cyclodextrin moiety comprises beta-cyclodextrin, the theoretical maximum loading of the topoisomerase inhibitor on the CDP-topoisomerase inhibitor conjugate is 13% by weight, and the topoisomerase inhibitor is 6-10% by weight of CDP-topoisomerase inhibitor conjugate. In some embodiments, the topoisomerase inhibitor is poorly soluble in water. In some embodiments, the solubility of the topoisomerase inhibitor is <5 mg/ml at physiological pH. In some embodiments, the topoisomerase inhibitor is a hydrophobic compound with a log P>0.4, >0.6, >0.8, >1, >2, >3, >4, or >5.

In some embodiments, the topoisomerase inhibitor is attached to the CDP via a second compound.

In some embodiments, administration of the CDP-topoisomerase inhibitor conjugate to a subject results in release of the topoisomerase inhibitor over a period of at least 6 hours. In some embodiments, administration of the CDP-topoisomerase inhibitor conjugate to a subject results in release of the topoisomerase inhibitor over a period of 2 hours, 3 hours, 5 hours, 6 hours, 8 hours, 10 hours, 15 hours, 20 hours, 1 day, 2 days, 3 days, 4 days, 7 days, 10 days, 14 days, 17 days, 20 days, 24 days, 27 days up to a month. In some embodiments, upon administration of the CDP-topoisomerase inhibitor conjugate to a subject, the rate of topoisomerase inhibitor release is dependent primarily upon the rate of hydrolysis as opposed to enzymatic cleavage.

In some embodiments, the CDP-topoisomerase inhibitor conjugate has a molecular weight of 10,000-500,000. In some embodiments, the cyclodextrin moieties make up at least about 2%, 5%, 10%, 20%, 30%, 50% or 80% of the CDP-topoisomerase inhibitor conjugate by weight.

In some embodiments, the CDP-topoisomerase inhibitor conjugate is made by a method comprising providing cyclodextrin moiety precursors modified to bear one reactive site at each of exactly two positions, and reacting the cyclodextrin moiety precursors with comonomer precursors having exactly two reactive moieties capable of forming a covalent bond with the reactive sites under polymerization conditions that promote reaction of the reactive sites with the reactive moieties to form covalent bonds between the comonomers and the cyclodextrin moieties, whereby a CDP comprising alternating units of a cyclodextrin moiety and a comonomer is produced. In some embodiments, the cyclodextrin moiety precursors are in a composition, the composition being substantially free of cyclodextrin moieties having other than two positions modified to bear a reactive site (e.g., cyclodextrin moieties having 1, 3, 4, 5, 6, or 7 positions modified to bear a reactive site).

In some embodiments, a comonomer of the CDP-topoisomerase inhibitor conjugate comprises a moiety selected from the group consisting of: an alkylene chain, polysuccinic anhydride, poly-L-glutamic acid, poly(ethyleneimine), an oligosaccharide, and an amino acid chain. In some embodiments, a CDP-topoisomerase inhibitor conjugate comonomer comprises a polyethylene glycol chain. In some embodiments, a comonomer comprises a moiety selected from: polyglycolic acid and polylactic acid chain. In some embodiments, a comonomer comprises a hydrocarbylene group wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR₁, O or S), -OC(O)-, -C(=O)O, -NR₁-, -NR₁CO-, -C(O)NR₁-, -S(O)ₙ- (wherein n is 0, 1, or 2), -OC(O)-NR₁, -NR₁-C(O)-NR₁-, -NR₁1-C(NR₁)-NR₁-, and -B(OR₁)-; and R₁, independently for each occurrence, represents H or a lower alkyl.

In some embodiments, the CDP-topoisomerase inhibitor conjugate is a polymer having attached thereto a plurality of D moieties of the following formula: 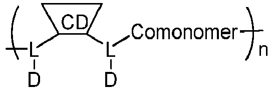 wherein each L is independently a linker, and each D is independently a topoisomerase inhibitor, a prodrug derivative thereof, e.g., a camptothecin or camptothecin derivative, or absent; and each comonomer is independently a comonomer described herein, and n is at least 4, 5, 6, 7, 8, 9, 10, 11, 12, 13, 14, 15, 16, 17, 18, 19 or 20, provided that the polymer comprises at least one topoisomerase inhibitor and in some embodiments, at least two topoisomerase inhibitor moieties. In some embodiments, the molecular weight of the comonomer is from about 2000 to about 5000 Da (e.g., from about 3000 to about 4000 Da (e.g., about 3400 Da).

In some embodiments, the topoisomerase inhibitor is a topoisomerase inhibitor described herein, for example, the topoisomerase inhibitor is a camptothecin or camptothecin derivative described herein. The topoisomerase inhibitor can be attached to the CDP via a functional group such as a hydroxyl group, or where appropriate, an amino group. In some embodiments, one or more of the topoisomerase inhibitor moieties in the CDP-topoisomerase inhibitor conjugate can be replaced with another therapeutic agent, e.g., another anticancer agent or anti-inflammatory agent.

In some embodiments, the CDP-topoisomerase inhibitor conjugate is a polymer having attached thereto a plurality of D moieties of the following formula: 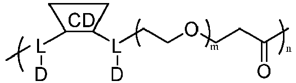 wherein each L is independently a linker, and each D is independently a topoisomerase, a prodrug derivative thereof, e.g., a camptothecin or camptothecin derivative, or absent, provided that the polymer comprises at least one topoisomerase inhibitor and in some embodiments, at least two topoisomerase inhibitor moieties; and
wherein the group 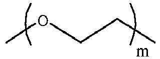 has a Mw of 3.4 kDa or less and n is at least 4, 5, 6, 7, 8, 9, 10, 11, 12, 13, 14, 15, 16, 17, 18, 19 or 20.

In some embodiments, the topoisomerase inhibitor is a topoisomerase inhibitor described herein, for example, the topoisomerase is a camptothecin or camptothecin derivative described herein. The topoisomerase inhibitor can be attached to the CDP via a functional group such as a hydroxyl group, or where appropriate, an amino group. In some embodiments, one or more of the topoisomerase inhibitor moieties in the CDP-topoisomerase inhibitor conjugate can be replaced with another therapeutic agent, e.g., another anticancer agent or anti-inflammatory agent.

In some embodiments, less than all of the L moieties are attached to D moieties, meaning in some embodiments, at least one D is absent. In some embodiments, the loading of the D moieties on the CDP-topoisomerase inhibitor conjugate is from about 1 to about 50% (e.g., from about 1 to about 25%, from about 5 to about 20% or from about 5 to about 15%). In some embodiments, each L independently comprises an amino acid or a derivative thereof. In some embodiments, each L independently comprises a plurality of amino acids or derivatives thereof. In some embodiments, each L is independently a dipeptide or derivative thereof. In one embodiment, L is one ore more of: alanine, arginine, histidine, lysine, aspartic acid, glutamic acid, serine, threonine, asparganine, glutamine, cysteine, glycine, proline, isoleucine, leucine, methionine, phenylalanine, tryptophan, tyrosine and valine.

In some embodiments, the CDP-topoisomerase inhibitor conjugate is a polymer having attached thereto a plurality of L-D moieties of the following formula: 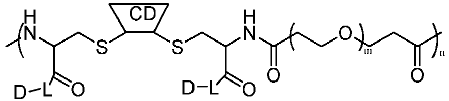 wherein each L is independently a linker or absent and each D is independently a topoisomerase inhibitor, a prodrug derivative thereof, e.g., a camptothecin or camptothecin derivative, or absent and wherein the group 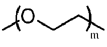 has a Mw of 3.4kDa or less and n is at least 4, 5, 6, 7, 8, 9, 10, 11, 12, 13, 14, 15, 16, 17, 18, 19 or 20, provided that the polymer comprises at least one topoisomerase inhibitor and in some embodiments, at least two topoisomerase inhibitor moieties.

In some embodiments, less than all of the C(=O) moieties are attached to L-D moieties, meaning in some embodiments, at least one L and/or D is absent. In some embodiments, the loading of the L, D and/or L-D moieties on the CDP-topoisomerase inhibitor conjugate is from about 1 to about 50% (e.g., from about 1 to about 25%, from about 5 to about 20% or from about 5 to about 15%). In some embodiments, each L is independently an amino acid or derivative thereof. In some embodiments, each L is glycine or a derivative thereof.

In some embodiments, one or more of the topoisomerase inhibitor moieties in the CDP-topoisomerase inhibitor conjugate can be replaced with another therapeutic agent, e.g., another anticancer agent or anti-inflammatory agent.

In some embodiments, the CDP-topoisomerase inhibitor conjugate is a polymer having the following formula:

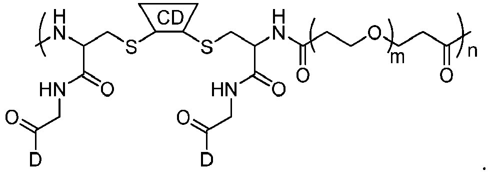

In some embodiments, less than all of the C(=O) moieties are attached to 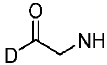 moieties, meaning in some embodiments, 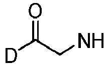 is absent, provided that the polymer comprises at least one topoisomerase inhibitor and in some embodiments, at least two topoisomerase inhibitor moieties. In some embodiments, the loading of the 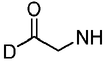 moieties on the CDP-topoisomerase inhibitor conjugate is from about 1 to about 50% (e.g., from about 1 to about 25%, from about 5 to about 20% or from about 5 to about 15%).

In some embodiments, one or more of the topoisomerase inhibitor moieties in the CDP-topoisomerase inhibitor conjugate can be replaced with another therapeutic agent, e.g., another anticancer agent or anti-inflammatory agent.

In some embodiments, the CDP-topoisomerase inhibitor conjugate will contain an topoisomerase inhibitor and at least one additional therapeutic agent. For instance, a topoisomerase inhibitor and one more different cancer drugs, an immunosuppressant, an antibiotic or an anti-inflammatory agent may be grafted on to the polymer via optional linkers. By selecting different linkers for different drugs, the release of each drug may be attenuated to achieve maximal dosage and efficacy.

### Cyclodextrins

In certain embodiments, the cyclodextrin moieties make up at least about 2%, 5% or 10% by weight, up to 20%, 30%, 50% or even 80% of the CDP by weight. In certain embodiments, the topoisomerase inhibitors, or targeting ligands make up at least about 1%, 5%, 10% or 15%, 20%, 25%, 30% or even 35% of the CDP by weight. Number-average molecular weight (Mₙ) may also vary widely, but generally fall in the range of about 1,000 to about 500,000 daltons, preferably from about 5000 to about 200,000 daltons and, even more preferably, from about 10,000 to about 100,000. Most preferably, Mₙ varies between about 12,000 and 65,000 daltons. In certain other embodiments, Mₙ varies between about 3000 and 150,000 daltons. Within a given sample of a subject polymer, a wide range of molecular weights may be present. For example, molecules within the sample may have molecular weights that differ by a factor of 2, 5, 10, 20, 50, 100, or more, or that differ from the average molecular weight by a factor of 2, 5, 10, 20, 50, 100, or more. Exemplary cyclodextrin moieties include cyclic structures consisting essentially of from 7 to 9 saccharide moieties, such as cyclodextrin and oxidized cyclodextrin. A cyclodextrin moiety optionally comprises a linker moiety that forms a covalent linkage between the cyclic structure and the polymer backbone, preferably having from 1 to 20 atoms in the chain, such as alkyl chains, including dicarboxylic acid derivatives (such as glutaric acid derivatives, succinic acid derivatives, and the like), and heteroalkyl chains, such as oligoethylene glycol chains.

Cyclodextrins are cyclic polysaccharides containing naturally occurring D-(+)-glucopyranose units in an α-(1,4) linkage. The most common cyclodextrins are alpha ((α)-cyclodextrins, beta (β)-cyclodextrins and gamma (γ)-cyclodextrins which contain, respectively six, seven, or eight glucopyranose units. Structurally, the cyclic nature of a cyclodextrin forms a torus or donut-like shape having an inner apolar or hydrophobic cavity, the secondary hydroxyl groups situated on one side of the cyclodextrin torus and the primary hydroxyl groups situated on the other. Thus, using (β)-cyclodextrin as an example, a cyclodextrin is often represented schematically as follows.

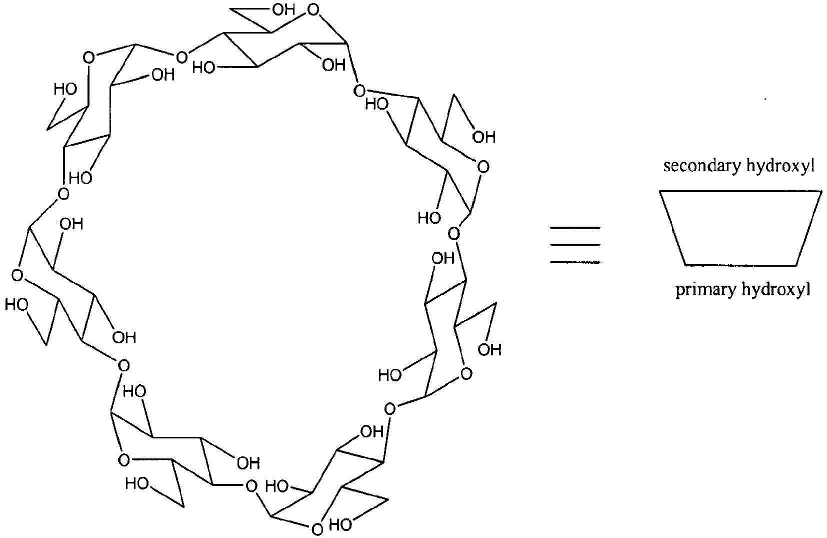

The side on which the secondary hydroxyl groups are located has a wider diameter than the side on which the primary hydroxyl groups are located. The present disclosure contemplates covalent linkages to cyclodextrin moieties on the primary and/or secondary hydroxyl groups. The hydrophobic nature of the cyclodextrin inner cavity allows for host-guest inclusion complexes of a variety of compounds, e.g., adamantane. (Comprehensive Supramolecular Chemistry, Volume 3, J.L. Atwood et al., eds., Pergamon Press (1996); T. Cserhati, Analytical Biochemistry, 225:328-332(1995); Husain et al., Applied Spectroscopy, 46:652-658 (1992); FR 2 665 169). Additional methods for modifying polymers are disclosed in Suh, J. and Noh, Y., Bioorg. Med. Chem. Lett. 1998, 8, 1327-1330.

In certain embodiments, the compounds comprise cyclodextrin moieties and wherein at least one or a plurality of the cyclodextrin moieties of the CDP-topoisomerase inhibitor conjugate is oxidized. In certain embodiments, the cyclodextrin moieties of P alternate with linker moieties in the polymer chain.

### Comonomers

In addition to a cyclodextrin moiety, the CDP can also include a comonomer, for example, a comonomer described herein. In some embodiments, a comonomer of the CDP-topoisomerase inhibitor conjugate comprises a moiety selected from the group consisting of: an alkylene chain, polysuccinic anhydride, poly-L-glutamic acid, poly(ethyleneimine), an oligosaccharide, and an amino acid chain. In some embodiments, a CDP-topoisomerase inhibitor conjugate comonomer comprises a polyethylene glycol chain. In some embodiments, a comonomer comprises a moiety selected from: polyglycolic acid and polylactic acid chain. In some embodiments, a comonomer comprises a hydrocarbylene group wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR₁, O or S), -OC(O)-, -C(=O)O,-NR₁-, -NR₁CO-, -C(O)NR₁-, -S(O)ₙ- (wherein n is 0, 1, or 2), -OC(O)-NR₁, -NR₁-C(O)-NR₁-, -NR₁1-C(NR₁)-NR₁-, and -B(OR₁)-; and R₁, independently for each occurrence, represents H or a lower alkyl.

In some embodiments, a comonomer can be and/or can comprise a linker such as a linker described herein.

### Exemplary CDP-topoisomerase inhibitor conjugates, particles and compositions

In one embodiment, the CDP-topoisomerase inhibitor conjugate forms a particle, e.g., a nanoparticle. The particle can comprise a CDP-topoisomerase inhibitor conjugate, e.g., a plurality of CDP-topoisomerase inhibitor conjugates, e.g., CDP-topoisomerase inhibitor conjugates having the same topoisomerase inhibitor or different topoisomerase inhibitors. The compositions described herein comprise a CDP-topoisomerase inhibitor conjugate or a plurality of CDP-topoisomerase inhibitor conjugates. The composition can also comprise a particle or a plurality of particles described herein.

In one embodiment, the CDP-topoisomerase inhibitor conjugate containing the inclusion complex forms a particle, e.g., a nanoparticle. The nanoparticle ranges in size from 10 to 300 nm in diameter, e.g., 20 to 280, 30 to 250, 40 to 200, 20 to 150, 30 to 100, 20 to 80, 30 to 70, 40 to 60 or 40 to 50 nm diameter. In one embodiment, the particle is 50 to 60 nm, 20 to 60 nm, 30 to 60 nm, 35 to 55 nm, 35 to 50 nm or 35 to 45 nm in diameter.

In one embodiment, the surface charge of the molecule is neutral, or slightly negative. In some embodiments, the zeta potential of the particle surface is from about -80 mV to about 50 mV, about -20 mV to about 20 mV, about -20 mV to about -10 mV, or about -10 mV to about 0.

In some embodiments, the CDP-topoisomerase inhibitor conjugate is a polymer having the following formula C: 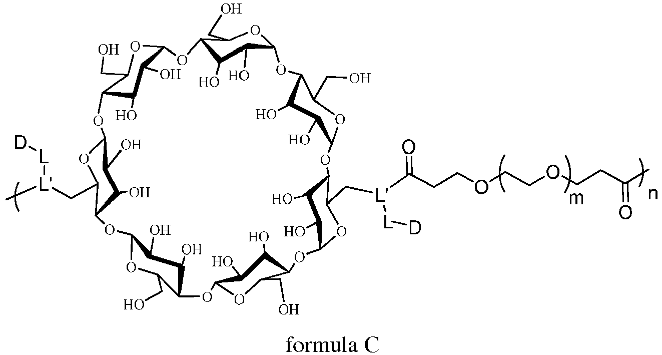 wherein L and L' independently for each occurrence, is a linker, a bond, or -OH and D, independently for each occurrence, is a topoisomerase inhibitor such as camptothecin ("CPT"), a camptothecin derivative or absent, and
wherein the group 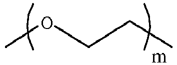 has a Mw of 3.4kDa or less and n is at least 4, provided that at least one D is CPT or a camptothecin derivative. In some embodiments, at least 2 D moieties are CPT and/or a camptothecin derivative.

In some embodiments, each L', for each occurrence, is a cysteine. In some embodiments, the cysteine is attached to the cyclodextrin via a sulfide bond. In some embodiments, the cysteine is attached to the PEG containing portion of the polymer via an amide bond.

In some embodiments, the L is a linker (e.g., an amino acid such as glycine). In some embodiments, L is absent. In some embodiments, D-L together form

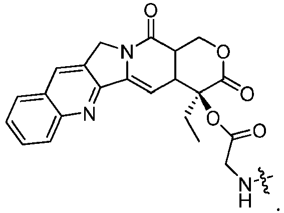

In some embodiments, a plurality of D moieties are absent and at the same position on the polymer, the corresponding L is -OH.

In some embodiments, less than all of the C(=O) moieties of the cysteine residue in the polymer backbone are attached to 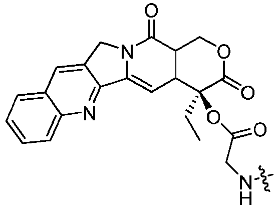 moieties, meaning in some embodiments, 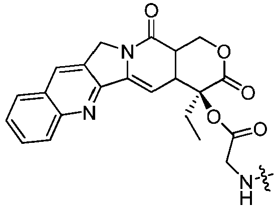 is absent in one or more positions of the polymer backbone, provided that the polymer comprises at least one 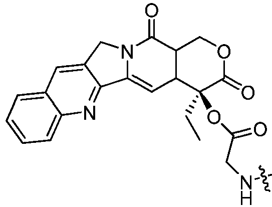 and in some embodiments, at least two 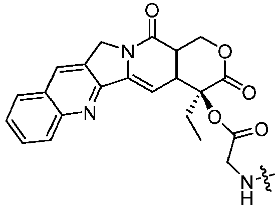 moieties. In some embodiments, the loading of the 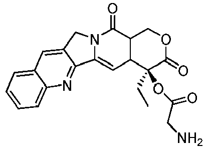 moieties on the CDP-topoisomerase inhibitor conjugate is from about 1 to about 50% (e.g., from about 1 to about 25%, from about 5 to about 20% or from about 5 to about 15%, e.g., from about 6 to about 10%). In some embodiments, the loading of 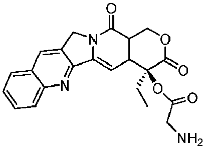 on the CDP is from about 6% to about 10% by weight of the total polymer.

In some embodiments, the CDP-topoisomerase inhibitor conjugate of formula C is a polymer having the following formula: 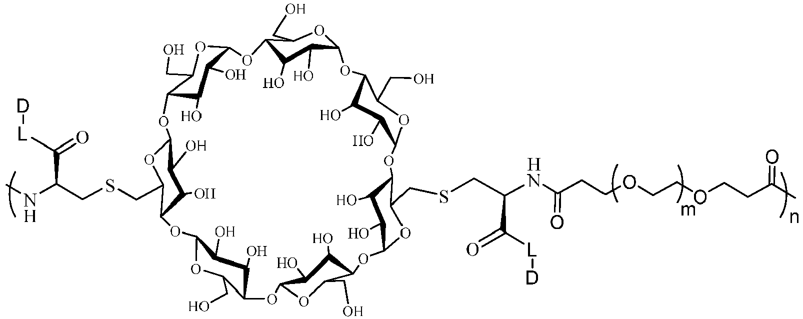 wherein L, independently for each occurrence, is a linker, a bond, or -OH and D, independently for each occurrence, is camptothecin ("CPT"), a camptothecin derivative or absent, and
wherein the group 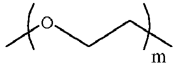 has a Mw of 3.4kDa or less and n is at least 4, provided that at least one D is CPT or a camptothecin derivative. In some embodiments, at least 2 D moieties are CPT and/or a camptothecin derivative.

In some embodiments, the CDP-camptothecin conjugate of formula C is a polymer of the following formula: 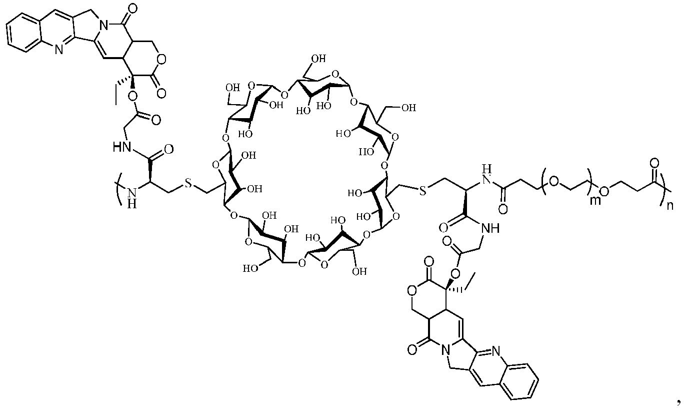 wherein m and n are as defined above, and wherein less than all of the C(=O) sites of the cysteine of the polymer backbone are occupied as indicated above with the CPT-Gly, but instead are free acids, meaning, the theoretical loading of the polymer is less than 100%.

In some embodiments, the CDP-camptothecin conjugate is as provided in FIG. 4, and shown below, which is referred to herein as "CRLX101." 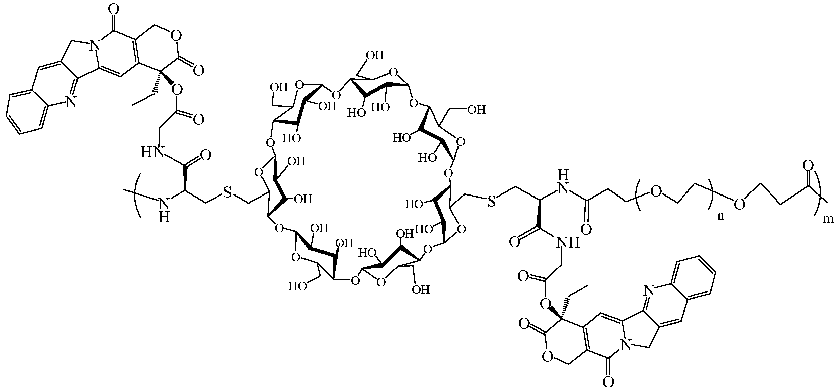 In the above structure:
n = about 77 or the molecular weight of the PEG moiety is from about 3060 to about 3740 (e.g., about 3400) Da;
m = is from about 10 to about 18 (e.g., about 14);
the molecular weight of the polymer backbone (i.e., the polymer minus the CPT-gly, which results in the cysteine moieties having a free -C(O)OH) is from about 48 to about 85 kDa;
the polydispersity of the polymer backbone is less than about 2.2; and
the loading of the CPT onto the polymer backbone is from about 6 to about 13% by weight, wherein 13% is theoretical maximum, meaning, in some instances, one or more of the cysteine residues has a free -C(O)OH (i.e., it lacks the CPT-gly).

In some embodiments, the polydispersity of the PEG component in the above structure is less than about 1.1.

In some embodiments, a CDP-camptothecin conjugate described herein has a terminal amine and/or a terminal carboxylic acid.

### Linkers/tethers

The CDPs described herein can include on or more linkers. In some embodiments, a linker can link a topoisomerase inhibitor to a CDP. In some embodiments, a linker can link camptothecin or a camptothecin derivative to a CDP. In some embodiments, for example, when referring to a linker that links a topoisomerase inhibitor to the CDP, the linker can be referred to as a tether.

In certain embodiments, a plurality of the linker moieties are attached to a topoisomerase inhibitor or prodrug thereof and are cleaved under biological conditions.

Described herein are CDP-topoisomerase inhibitor conjugates comprising a CDP covalently attached to a topoisomerase inhibitor through attachments that are cleaved under biological conditions to release the topoisomerase inhibitor. In certain embodiments, a CDP-topoisomerase inhibitor conjugate comprises a topoisomerase inhibitor covalently attached to a polymer, preferably a biocompatible polymer, through a tether, e.g., a linker, wherein the tether comprises a selectivity-determining moiety and a self-cyclizing moiety which are covalently attached to one another in the tether, e.g., between the polymer and the topoisomerase inhibitor.

In some embodiments, such topoisomerase inhibitors are covalently attached to CDPs through functional groups comprising one or more heteroatoms, for example, hydroxy, thiol, carboxy, amino, and amide groups. Such groups may be covalently attached to the subject polymers through linker groups as described herein, for example, biocleavable linker groups, and/or through tethers, such as a tether comprising a selectivity-determining moiety and a self-cyclizing moiety which are covalently attached to one another.

In certain embodiments, the CDP-topoisomerase inhibitor conjugate comprises a topoisomerase inhibitor covalently attached to the CDP through a tether, wherein the tether comprises a self-cyclizing moiety. In some embodiments, the tether further comprises a selectivity-determining moiety. Thus, one aspect of the disclosure relates to a polymer conjugate comprising a topoisomerase inhibitor covalently attached to a polymer, preferably a biocompatible polymer, through a tether, wherein the tether comprises a selectivity-determining moiety and a self-cyclizing moiety which are covalently attached to one another.

In some embodiments, the selectivity-determining moiety is bonded to the self-cyclizing moiety between the self-cyclizing moiety and the CDP.

In certain embodiments, the selectivity-determining moiety is a moiety that promotes selectivity in the cleavage of the bond between the selectivity-determining moiety and the self-cyclizing moiety. Such a moiety may, for example, promote enzymatic cleavage between the selectivity-determining moiety and the self-cyclizing moiety. Alternatively, such a moiety may promote cleavage between the selectivity-determining moiety and the self-cyclizing moiety under acidic conditions or basic conditions.

In certain embodiments, the disclosure contemplates any combination of the foregoing. Those skilled in the art will recognize that, for example, any topoisomerase inhibitor of the disclosure in combination with any linker (e.g., self-cyclizing moiety, any selectivity-determining moiety, and/or any topoisomerase inhibitor) are within the scope of the disclosure.

In certain embodiments, the selectivity-determining moiety is selected such that the bond is cleaved under acidic conditions.

In certain embodiments, where the selectivity-determining moiety is selected such that the bond is cleaved under basic conditions, the selectivity-determining moiety is an aminoalkylcarbonyloxyalkyl moiety. In certain embodiments, the selectivity-determining moiety has a structure

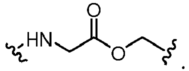

In certain embodiments where the selectivity-determining moiety is selected such that the bond is cleaved enzymatically, it may be selected such that a particular enzyme or class of enzymes cleaves the bond. In certain preferred such embodiments, the selectivity-determining moiety may be selected such that the bond is cleaved by a cathepsin, preferably cathepsin B.

In certain embodiments the selectivity-determining moiety comprises a peptide, preferably a dipeptide, tripeptide, or tetrapeptide. In certain such embodiments, the peptide is a dipeptide is selected from KF and FK, In certain embodiments, the peptide is a tripeptide is selected from GFA, GLA, AVA, GVA, GIA, GVL, GVF, and AVF. In certain embodiments, the peptide is a tetrapeptide selected from GFYA and GFLG, preferably GFLG.

In certain such embodiments, a peptide, such as GFLG, is selected such that the bond between the selectivity-determining moiety and the self-cyclizing moiety is cleaved by a cathepsin, preferably cathepsin B.

In certain embodiments, the selectivity-determining moiety is represented by Formula A: 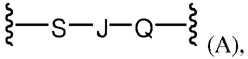 wherein
S a sulfur atom that is part of a disulfide bond;
J is optionally substituted hydrocarbyl; and
Q is O or NR¹³, wherein R¹³ is hydrogen or alkyl.

In certain embodiments, J may be polyethylene glycol, polyethylene, polyester, alkenyl, or alkyl. In certain embodiments, J may represent a hydrocarbylene group comprising one or more methylene groups, wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR³⁰, O or S), -OC(O)-, -C(=O)O, -NR³⁰-, -NR₁CO-, -C(O)NR³⁰-, -S(O)ₙ- (wherein n is 0, 1, or 2), -OC(O)-NR³⁰, -NR³⁰-C(O)-NR³⁰-, -NR³⁰-C(NR³⁰)-NR³⁰-, and -B(OR³⁰)-; and R³⁰, independently for each occurrence, represents H or a lower alkyl. In certain embodiments, J may be substituted or unsubstituted lower alkylene, such as ethylene. For example, the selectivity-determining moiety may be

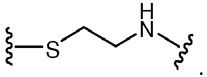

In certain embodiments, the selectivity-determining moiety is represented by Formula B: 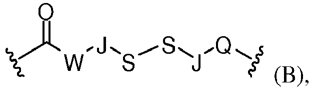 wherein
W is either a direct bond or selected from lower alkyl, NR¹⁴, S, O;
S is sulfur;
J, independently and for each occurrence, is hydrocarbyl or polyethylene glycol;
Q is O or NR¹³, wherein R¹³ is hydrogen or alkyl; and
R¹⁴ is selected from hydrogen and alkyl.

In certain such embodiments, J may be substituted or unsubstituted lower alkyl, such as methylene. In certain such embodiments, J may be an aryl ring. In certain embodiments, the aryl ring is a benzo ring. In certain embodiments W and S are in a 1,2-relationship on the aryl ring. In certain embodiments, the aryl ring may be optionally substituted with alkyl, alkenyl, alkoxy, aralkyl, aryl, heteroaryl, halogen, -CN, azido, -NR^{x}R^{x}, -CO₂OR^{x}, -C(O)-NR^{x}R^{x}, -C(O)-R^{x}, -NR^{x}-C(O)-R^{x}, -NR^{x}SO₂R^{x}, -SR^{x}, -S(O)R^{x}, -SO₂R^{x}, -SO₂NR^{x}R^{x}, -(C(R^{x})₂)ₙ-OR^{x}, -(C(R^{x})₂)ₙ-NR^{x}R^{x}, and -(C(R^{x})₂)ₙ-SO₂R^{x}; wherein R^{x} is, independently for each occurrence, H or lower alkyl; and n is, independently for each occurrence, an integer from 0 to 2.

In certain embodiments, the aryl ring is optionally substituted with alkyl, alkenyl, alkoxy, aralkyl, aryl, heteroaryl, halogen, -CN, azido, -NR^{x}R^{x}, -CO₂OR^{x}, -C(O)-NR^{x}R^{x}, -C(O)-R^{x}, -NR^{x}-C(O)-R^{x}, -NR^{x}SO₂R^{x}, -SR^{x}, -S(O)R^{x}, -SO₂R^{x}, -SO₂NR^{x}R^{x}, -(C(R^{x})₂)ₙ-OR^{x}, -(C(R^{x})₂)ₙ-NR^{x}R^{x}, and -(C(R^{x})₂)ₙ-SO₂R^{x}; wherein R^{x} is, independently for each occurrence, H or lower alkyl; and n is, independently for each occurrence, an integer from 0 to 2.

In certain embodiments, J, independently and for each occurrence, is polyethylene glycol, polyethylene, polyester, alkenyl, or alkyl.

In certain embodiments, independently and for each occurrence, the linker comprises a hydrocarbylene group comprising one or more methylene groups, wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR³⁰, O or S), -OC(O)-,-C(=O)O, -NR³⁰-, -NR₁CO-, -C(O)NR³⁰-, -S(O)ₙ- (wherein n is 0, 1, or 2), -OC(O)-NR³⁰, -NR³⁰-C(O)-NR³⁰-, -NR³⁰-C(NR³⁰)-NR³⁰-, and -B(OR³⁰)-; and R³⁰, independently for each occurrence, represents H or a lower alkyl.

In certain embodiments, J, independently and for each occurrence, is substituted or unsubstituted lower alkylene. In certain embodiments, J, independently and for each occurrence, is substituted or unsubstituted ethylene.

In certain embodiments, the selectivity-determining moiety is selected from 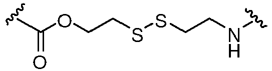 and 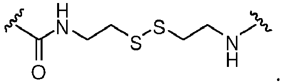 The selectivity-determining moiety may include groups with bonds that are cleavable under certain conditions, such as disulfide groups. In certain embodiments, the selectivity-determining moiety comprises a disulfide-containing moiety, for example, comprising aryl and/or alkyl group(s) bonded to a disulfide group. In certain embodiments, the selectivity-determining moiety has a structure 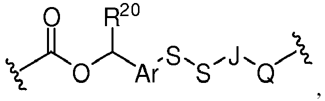 wherein
Ar is a substituted or unsubstituted benzo ring;
J is optionally substituted hydrocarbyl; and
Q is O or NR¹³,
wherein R¹³ is hydrogen or alkyl.

In certain embodiments, Ar is unsubstituted. In certain embodiments, Ar is a 1,2-benzo ring. For example, suitable moieties within Formula B include:

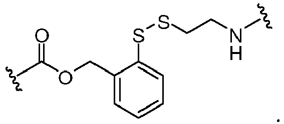

In certain embodiments, the self-cyclizing moiety is selected such that upon cleavage of the bond between the selectivity-determining moiety and the self-cyclizing moiety, cyclization occurs thereby releasing the therapeutic agent. Such a cleavage-cyclization-release cascade may occur sequentially in discrete steps or substantially simultaneously. Thus, in certain embodiments, there may be a temporal and/or spatial difference between the cleavage and the self-cyclization. The rate of the self-cyclization cascade may depend on pH, e.g., a basic pH may increase the rate of self-cyclization after cleavage. Self-cyclization may have a half-life after introduction *in vivo* of 24 hours, 18 hours, 14 hours, 10 hours, 6 hours, 3 hours, 2 hours, 1 hour, 30 minutes, 10 minutes, 5 minutes, or 1 minute.

In certain such embodiments, the self-cyclizing moiety may be selected such that, upon cyclization, a five- or six-membered ring is formed, preferably a five-membered ring. In certain such embodiments, the five- or six-membered ring comprises at least one heteroatom selected from oxygen, nitrogen, or sulfur, preferably at least two, wherein the heteroatoms may be the same or different. In certain such embodiments, the heterocyclic ring contains at least one nitrogen, preferably two. In certain such embodiments, the self-cyclizing moiety cyclizes to form an imidazolidone.

In certain embodiments, the self-cyclizing moiety has a structure 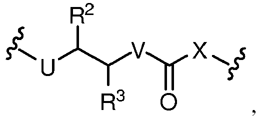 wherein
U is selected from NR¹ and S;
X is selected from O, NR⁵, and S, preferably O or S;
V is selected from O, S and NR⁴, preferably O or NR⁴;
R² and R³ are independently selected from hydrogen, alkyl, and alkoxy; or R² and R³ together with the carbon atoms to which they are attached form a ring; and R¹, R⁴, and R⁵ are independently selected from hydrogen and alkyl.

In certain embodiments, U is NR¹ and/or V is NR⁴, and R¹ and R⁴ are independently selected from methyl, ethyl, propyl, and isopropyl. In certain embodiments, both R¹ and R⁴ are methyl. On certain embodiments, both R² and R³ are hydrogen. In certain embodiments R² and R³ are independently alkyl, preferably lower alkyl. In certain embodiments, R² and R³ together are -(CH₂)ₙ- wherein n is 3 or 4, thereby forming a cyclopentyl or cyclohexyl ring. In certain embodiments, the nature of R² and R³ may affect the rate of cyclization of the self-cyclizing moiety. In certain such embodiments, it would be expected that the rate of cyclization would be greater when R² and R³ together with the carbon atoms to which they are attached form a ring than the rate when R² and R³ are independently selected from hydrogen, alkyl, and alkoxy. In certain embodiments, U is bonded to the self-cyclizing moiety.

In certain embodiments, the self-cyclizing moiety is selected from 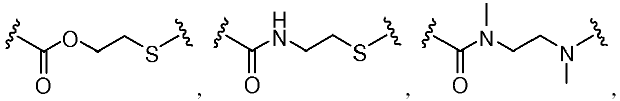 and

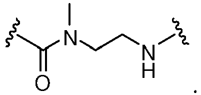

In certain embodiments, the selectivity-determining moiety may connect to the self-cyclizing moiety through carbonyl-heteroatom bonds, e.g., amide, carbamate, carbonate, ester, thioester, and urea bonds.

In certain embodiments, a topoisomerase inhibitor is covalently attached to a polymer through a tether, wherein the tether comprises a selectivity-determining moiety and a self-cyclizing moiety which are covalently attached to one another. In certain embodiments, the self-cyclizing moiety is selected such that after cleavage of the bond between the selectivity-determining moiety and the self-cyclizing moiety, cyclization of the self-cyclizing moiety occurs, thereby releasing the therapeutic agent. As an illustration, ABC may be a selectivity-determining moiety, and DEFGH maybe be a self-cyclizing moiety, and ABC may be selected such that enzyme Y cleaves between C and D. Once cleavage of the bond between C and D progresses to a certain point, D will cyclize onto H, thereby releasing topoisomerase inhibitor X, or a prodrug thereof.

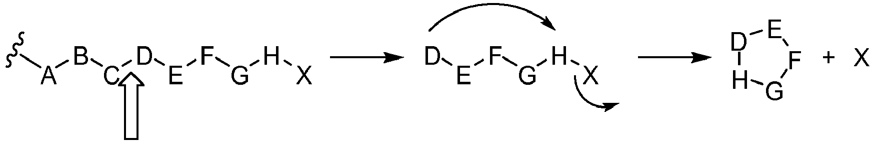

In certain embodiments, topoisomerase inhibitor X may further comprise additional intervening components, including, but not limited to another self-cyclizing moiety or a leaving group linker, such as CO₂ or methoxymethyl, that spontaneously dissociates from the remainder of the molecule after cleavage occurs.

In some embodiments, a linker may be and/or comprise an alkylene chain, a polyethylene glycol (PEG) chain, polysuccinic anhydride, poly-L-glutamic acid, poly(ethyleneimine), an oligosaccharide, an amino acid (e.g., glycine or cysteine), an amino acid chain, or any other suitable linkage. In certain embodiments, the linker group itself can be stable under physiological conditions, such as an alkylene chain, or it can be cleavable under physiological conditions, such as by an enzyme (e.g., the linkage contains a peptide sequence that is a substrate for a peptidase), or by hydrolysis (e.g., the linkage contains a hydrolyzable group, such as an ester or thioester). The linker groups can be biologically inactive, such as a PEG, polyglycolic acid, or polylactic acid chain, or can be biologically active, such as an oligo- or polypeptide that, when cleaved from the moieties, binds a receptor, deactivates an enzyme, etc. Various oligomeric linker groups that are biologically compatible and/or bioerodible are known in the art, and the selection of the linkage may influence the ultimate properties of the material, such as whether it is durable when implanted, whether it gradually deforms or shrinks after implantation, or whether it gradually degrades and is absorbed by the body. The linker group may be attached to the moieties by any suitable bond or functional group, including carbon-carbon bonds, esters, ethers, amides, amines, carbonates, carbamates, sulfonamides, etc.

In certain embodiments, the linker group(s) represent a hydrocarbylene group wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR₁, O or S), -OC(O)-, -C(=O)O, -NR₁-, -NR₁CO-, -C(O)NR₁-, -S(O)ₙ- (wherein n is 0, 1, or 2), -OC(O)-NR₁, -NR₁-C(O)-NR₁-, -NR₁-C(NR₁)-NR₁-, and -B(OR₁)-; and R₁, independently for each occurrence, represents H or a lower alkyl.

In certain embodiments, the linker group represents a derivatized or non-derivatized amino acid (e.g., glycine or cysteine). In certain embodiments, linker groups with one or more terminal carboxyl groups may be conjugated to the polymer. In certain embodiments, one or more of these terminal carboxyl groups may be capped by covalently attaching them to a therapeutic agent, a targeting moiety, or a cyclodextrin moiety via an (thio)ester or amide bond. In still other embodiments, linker groups with one or more terminal hydroxyl, thiol, or amino groups may be incorporated into the polymer. In preferred embodiments, one or more of these terminal hydroxyl groups may be capped by covalently attaching them to a therapeutic agent, a targeting moiety, or a cyclodextrin moiety via an (thio)ester, amide, carbonate, carbamate, thiocarbonate, or thiocarbamate bond. In certain embodiments, these (thio)ester, amide, (thio)carbonate or (thio)carbamates bonds may be biohydrolyzable, i.e., capable of being hydrolyzed under biological conditions.

In certain embodiments, a linker group represents a hydrocarbylene group wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR₁, O or S), -OC(O)-,-C(=O)O, -NR₁-, -NR₁CO-, -C(O)NR₁-, -S(O)ₙ- (wherein n is 0, 1, or 2), -OC(O)-NR₁, -NR₁-C(O)-NR₁-, -NR₁-C(NR₁)-NR₁-, and -B(OR₁)-; and R₁, independently for each occurrence, represents H or a lower alkyl.

In certain embodiments, a linker group, e.g., between a topoisomerase inhibitor and the CDP, comprises a self-cyclizing moiety. In certain embodiments, a linker group, e.g., between a topoisomerase inhibitor and the CDP, comprises a selectivity-determining moiety.

In certain embodiments as disclosed herein, a linker group, e.g., between a topoisomerase inhibitor and the CDP, comprises a self-cyclizing moiety and a selectivity-determining moiety.

In certain embodiments as disclosed herein, the topoisomerase inhibitor or targeting ligand is covalently bonded to the linker group via a biohydrolyzable bond (e.g., an ester, amide, carbonate, carbamate, or a phosphate).

In certain embodiments as disclosed herein, the CDP comprises cyclodextrin moieties that alternate with linker moieties in the polymer chain.

In certain embodiments, the linker moieties are attached to topoisomerase inhibitors or prodrugs thereof that are cleaved under biological conditions.

In certain embodiments, at least one linker that connects the topoisomerase inhibitor or prodrug thereof to the polymer comprises a group represented by the formula 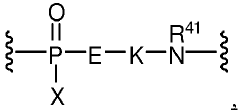 wherein
P is phosphorus;
O is oxygen;
E represents oxygen or NR⁴⁰;
K represents hydrocarbyl;
X is selected from OR⁴² or NR⁴³R⁴⁴; and
R⁴⁰, R⁴¹, R⁴², R⁴³, and R⁴⁴ independently represent hydrogen or optionally substituted alkyl.

In certain embodiments, E is NR⁴⁰ and R⁴⁰ is hydrogen.

In certain embodiments, K is lower alkylene (e.g., ethylene).

In certain embodiments, at least one linker comprises a group selected from 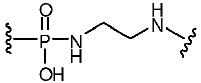 and

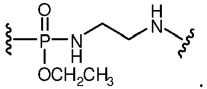

In certain embodiments, X is OR⁴².

In certain embodiments, the linker group comprises an amino acid or peptide, or derivative thereof (e.g., a glycine or cysteine).

In certain embodiments as disclosed herein, the linker is connected to the topoisomerase inhibitor through a hydroxyl group. In certain embodiments as disclosed herein, the linker is connected to the topoisomerase inhibitor through an amino group.

In certain embodiments, the linker group that connects to the topoisomerase inhibitor may comprise a self-cyclizing moiety, or a selectivity-determining moiety, or both. In certain embodiments, the selectivity-determining moiety is a moiety that promotes selectivity in the cleavage of the bond between the selectivity-determining moiety and the self-cyclizing moiety. Such a moiety may, for example, promote enzymatic cleavage between the selectivity-determining moiety and the self-cyclizing moiety. Alternatively, such a moiety may promote cleavage between the selectivity-determining moiety and the self-cyclizing moiety under acidic conditions or basic conditions.

In certain embodiments, any of the linker groups may comprise a self-cyclizing moiety or a selectivity-determining moiety, or both. In certain embodiments, the selectivity-determining moiety may be bonded to the self-cyclizing moiety between the self-cyclizing moiety and the polymer.

In certain embodiments, any of the linker groups may independently be or include an alkyl chain, a polyethylene glycol (PEG) chain, polysuccinic anhydride, poly-L-glutamic acid, poly(ethyleneimine), an oligosaccharide, an amino acid chain, or any other suitable linkage. In certain embodiments, the linker group itself can be stable under physiological conditions, such as an alkyl chain, or it can be cleavable under physiological conditions, such as by an enzyme (e.g., the linkage contains a peptide sequence that is a substrate for a peptidase), or by hydrolysis (e.g., the linkage contains a hydrolyzable group, such as an ester or thioester). The linker groups can be biologically inactive, such as a PEG, polyglycolic acid, or polylactic acid chain, or can be biologically active, such as an oligo- or polypeptide that, when cleaved from the moieties, binds a receptor, deactivates an enzyme, etc. Various oligomeric linker groups that are biologically compatible and/or bioerodible are known in the art, and the selection of the linkage may influence the ultimate properties of the material, such as whether it is durable when implanted, whether it gradually deforms or shrinks after implantation, or whether it gradually degrades and is absorbed by the body. The linker group may be attached to the moieties by any suitable bond or functional group, including carbon-carbon bonds, esters, ethers, amides, amines, carbonates, carbamates, sulfonamides, etc.

In certain embodiments, any of the linker groups may independently be an alkyl group wherein one or more methylene groups is optionally replaced by a group Y (provided that none of the Y groups are adjacent to each other), wherein each Y, independently for each occurrence, is selected from aryl, heteroaryl, carbocyclyl, heterocyclyl, or -O-, C(=X) (wherein X is NR¹, O or S), -OC(O)-, -C(=O)O-, -NR¹-, -NR¹CO-, -C(O)NR¹-, -S(O)ₙ-(wherein n is 0, 1, or 2), -OC(O)-NR¹-, -NR¹-C(O)-NR¹-, -NR¹-C(NR¹)-NR¹-, and -B(OR¹)-; and R¹, independently for each occurrence, is H or lower alkyl.

In certain embodiments, the present disclosure contemplates a CDP, wherein a plurality of topoisomerase inhibitors are covalently attached to the polymer through attachments that are cleaved under biological conditions to release the therapeutic agents as discussed above, wherein administration of the polymer to a subject results in release of the therapeutic agent over a period of at least 2, 3, 5, 6, 8, 10, 15, 20, 24, 36, 48 or even 72 hours.

In some embodiments, the conjugation of the topoisomerase inhibitor to the CDP improves the aqueous solubility of the topoisomerase inhibitor and hence the bioavailability. Accordingly, in one embodiment of the disclosure, the topoisomerase inhibitor has a log P >0.4, >0.6, >0.8, >1, >2, >3, >4, or even >5.

The CDP- topoisomerase inhibitor conjugate of the present disclosure preferably has a molecular weight in the range of 10,000 to 500,000; 30,000 to 200,000; or even 70,000 to 150,000 amu.

In certain embodiments, the present disclosure contemplates attenuating the rate of release of the topoisomerase inhibitor by introducing various tether and/or linking groups between the therapeutic agent and the polymer. Thus, in certain embodiments, the CDP-topoisomerase inhibitor conjugates of the present disclosure are compositions for controlled delivery of the topoisomerase inhibitor.

### CDP- topoisomerase inhibitor conjugate characteristics

In some embodiments, the CDP and/or CDP- topoisomerase inhibitor conjugate, particle or composition as described herein have polydispersities less than about 3, or even less than about 2.

One embodiment of the present disclosure provides an improved delivery of certain topoisomerase inhibitor by covalently attaching one or more topoisomerase inhibitors to a CDP. Such conjugation can improve the aqueous solubility and hence the bioavailability of the topoisomerase inhibitor.

The CDP- topoisomerase inhibitor conjugates, particles and compositions described herein preferably have molecular weights in the range of 10,000 to 500,000; 30,000 to 200,000; or even 70,000 to 150,000 amu. In certain embodiments as disclosed herein, the compound has a number average (Mₙ) molecular weight between 1,000 to 500,000 amu, or between 5,000 to 200,000 amu, or between 10,000 to 100,000 amu. One method to determine molecular weight is by gel permeation chromatography ("GPC"), e.g., mixed bed columns, CH₂Cl₂ solvent, light scattering detector, and off-line dn/dc. Other methods are known in the art.

In certain embodiments as disclosed herein, the CDP-topoisomerase inhibitor conjugate, particle or composition is biodegradable or bioerodable.

In certain embodiments as disclosed herein, the topoisomerase inhibitor, e.g., the camptothecin, camptothecin derivative, or prodrug thereof makes up at least 3% (e.g., at least about 5%) by weight of the polymer. In certain embodiments, the topoisomerase inhibitor, e.g., the camptothecin, camptothecin derivative or prodrug thereof makes up at least 20% by weight of the compound. In certain embodiments, the topoisomerase inhibitor, e.g., the camptothecin, camptothecin derivative or prodrug thereof makes up at least 5%, 10%, 15%, or at least 20% by weight of the compound.

CDP-topoisomerase inhibitor conjugates, particles and compositions of the present disclosure may be useful to improve solubility and/or stability of the topoisomerase inhibitor, reduce drug-drug interactions, reduce interactions with blood elements including plasma proteins, reduce or eliminate immunogenicity, protect the topoisomerase inhibitor from metabolism, modulate drug-release kinetics, improve circulation time, improve topoisomerase inhibitor half-life (e.g., in the serum, or in selected tissues, such as tumors), attenuate toxicity, improve efficacy, normalize topoisomerase inhibitor metabolism across subjects of different species, ethnicities, and/or races, and/or provide for targeted delivery into specific cells or tissues.

In other embodiments, the CDP-topoisomerase inhibitor conjugate, particle or composition may be a flexible or flowable material. When the CDP used is itself flowable, the CDP composition of the disclosure, even when viscous, need not include a biocompatible solvent to be flowable, although trace or residual amounts of biocompatible solvents may still be present.

While it is possible that the biodegradable polymer or the biologically active agent may be dissolved in a small quantity of a solvent that is non-toxic to more efficiently produce an amorphous, monolithic distribution or a fine dispersion of the biologically active agent in the flexible or flowable composition, it is an advantage of the disclosure that, in a preferred embodiment, no solvent is needed to form a flowable composition. Moreover, the use of solvents is preferably avoided because, once a polymer composition containing solvent is placed totally or partially within the body, the solvent dissipates or diffuses away from the polymer and must be processed and eliminated by the body, placing an extra burden on the body's clearance ability at a time when the illness (and/or other treatments for the illness) may have already deleteriously affected it.

However, when a solvent is used to facilitate mixing or to maintain the flowability of the CDP-topoisomerase inhibitor conjugate, particle or composition, it should be non-toxic, otherwise biocompatible, and should be used in relatively small amounts. Solvents that are toxic should not be used in any material to be placed even partially within a living body. Such a solvent also must not cause substantial tissue irritation or necrosis at the site of administration.

Examples of suitable biocompatible solvents, when used, include N-methyl-2-pyrrolidone, 2-pyrrolidone, ethanol, propylene glycol, acetone, methyl acetate, ethyl acetate, methyl ethyl ketone, dimethylformamide, dimethylsulfoxide, tetrahydrofuran, caprolactam, oleic acid, or 1-dodecylazacylcoheptanone. Preferred solvents include N-methylpyrrolidone, 2-pyrrolidone, dimethylsulfoxide, and acetone because of their solvating ability and their biocompatibility.

In certain embodiments, the CDP-topoisomerase inhibitor conjugates, particles and compositions are soluble in one or more common organic solvents for ease of fabrication and processing. Common organic solvents include such solvents as chloroform, dichloromethane, dichloroethane, 2-butanone, butyl acetate, ethyl butyrate, acetone, ethyl acetate, dimethylacetamide, N-methylpyrrolidone, dimethylformamide, and dimethylsulfoxide.

In certain embodiments, the CDP-topoisomerase inhibitor conjugates, particles and compositions described herein, upon contact with body fluids, undergo gradual degradation. The life of a biodegradable polymer in vivo depends upon, among other things, its molecular weight, crystallinity, biostability, and the degree of crosslinking. In general, the greater the molecular weight, the higher the degree of crystallinity, and the greater the biostability, the slower biodegradation will be.

If a subject composition is formulated with a topoisomerase inhibitor or other material, release of the topoisomerase inhibitor or other material for a sustained or extended period as compared to the release from an isotonic saline solution generally results. Such release profile may result in prolonged delivery (over, say 1 to about 2,000 hours, or alternatively about 2 to about 800 hours) of effective amounts (e.g., about 0.0001 mg/kg/hour to about 10 mg/kg/hour, e.g., 0.001 mg/kg/hour, 0.01 mg/kg/hour, 0.1 mg/kg/hour, 1.0 mg/kg/hour) of the topoisomerase inhibitor or any other material associated with the polymer.

A variety of factors may affect the desired rate of hydrolysis of CDP-topoisomerase inhibitor conjugates, particles and compositions, the desired softness and flexibility of the resulting solid matrix, rate and extent of bioactive material release. Some of such factors include the selection/identity of the various subunits, the enantiomeric or diastereomeric purity of the monomeric subunits, homogeneity of subunits found in the polymer, and the length of the polymer. For instance, the present disclosure contemplates heteropolymers with varying linkages, and/or the inclusion of other monomeric elements in the polymer, in order to control, for example, the rate of biodegradation of the matrix.

To illustrate further, a wide range of degradation rates may be obtained by adjusting the hydrophobicities of the backbones or side chains of the polymers while still maintaining sufficient biodegradability for the use intended for any such polymer. Such a result may be achieved by varying the various functional groups of the polymer. For example, the combination of a hydrophobic backbone and a hydrophilic linkage produces heterogeneous degradation because cleavage is encouraged whereas water penetration is resisted.

One protocol generally accepted in the field that may be used to determine the release rate of a therapeutic agent such as a topoisomerase inhibitor or other material loaded in the CDP-topoisomerase inhibitor conjugates, particles or compositions of the present disclosure involves degradation of any such matrix in a 0.1 M PBS solution (pH 7.4) at 37 °C, an assay known in the art. For purposes of the present disclosure, the term "PBS protocol" is used herein to refer to such protocol.

In certain instances, the release rates of different CDP-topoisomerase inhibitor conjugates, particles and compositions of the present disclosure may be compared by subjecting them to such a protocol. In certain instances, it may be necessary to process polymeric systems in the same fashion to allow direct and relatively accurate comparisons of different systems to be made. For example, the present disclosure teaches several different methods of formulating the CDP-topoisomerase inhibitor conjugates, particles and compositions. Such comparisons may indicate that any one CDP-topoisomerase inhibitor conjugate, particle or composition releases incorporated material at a rate from about 2 or less to about 1000 or more times faster than another polymeric system.

Alternatively, a comparison may reveal a rate difference of about 3, 5, 7, 10, 25, 50, 100, 250, 500 or 750 times. Even higher rate differences are contemplated by the present disclosure and release rate protocols.

In certain embodiments, when formulated in a certain manner, the release rate for CDP-topoisomerase inhibitor conjugates, particles and compositions of the present disclosure may present as mono- or bi-phasic.

Release of any material incorporated into the polymer matrix, which is often provided as a microsphere, may be characterized in certain instances by an initial increased release rate, which may release from about 5 to about 50% or more of any incorporated material, or alternatively about 10, 15, 20, 25, 30 or 40%, followed by a release rate of lesser magnitude.

The release rate of any incorporated material may also be characterized by the amount of such material released per day per mg of polymer matrix. For example, in certain embodiments, the release rate may vary from about 1 ng or less of any incorporated material per day per mg of polymeric system to about 500 or more ng/day/mg. Alternatively, the release rate may be about 0.05, 0.5, 5, 10, 25, 50, 75, 100, 125, 150, 175, 200, 250, 300, 350, 400, 450, or 500 ng/day/mg. In still other embodiments, the release rate of any incorporated material maybe 10,000 ng/day/mg, or even higher. In certain instances, materials incorporated and characterized by such release rate protocols may include therapeutic agents, fillers, and other substances.

In another aspect, the rate of release of any material from any CDP- topoisomerase inhibitor conjugate, particle or composition of the present disclosure may be presented as the half-life of such material in the matrix.

In addition to the embodiment involving protocols for in vitro determination of release rates, in vivo protocols, whereby in certain instances release rates for polymeric systems may be determined in vivo, are also contemplated by the present disclosure. Other assays useful for determining the release of any material from the polymers of the present system are known in the art.

### Physical Structures of the CDP-topoisomerase inhibitor conjugates, particles and compositions

The CDP-topoisomerase inhibitor conjugates, particles and compositions may be formed in a variety of shapes. For example, in certain embodiments, CDP-topoisomerase inhibitor conjugates may be presented in the form of microparticles or nanoparticles. Microspheres typically comprise a biodegradable polymer matrix incorporating a drug. Microspheres can be formed by a wide variety of techniques known to those of skill in the art. Examples of microsphere forming techniques include, but are not limited to, (a) phase separation by emulsification and subsequent organic solvent evaporation (including complex emulsion methods such as oil in water emulsions, water in oil emulsions and water-oil-water emulsions); (b) coacervation-phase separation; (c) melt dispersion; (d) interfacial deposition; (e) in situ polymerization; (f) spray drying and spray congealing; (g) air suspension coating; and (h) pan and spray coating. These methods, as well as properties and characteristics of microspheres are disclosed in, for example, U.S. Patent No. 4,438,253; U.S. Patent No. 4,652,441; U.S. Patent No. 5,100,669; U.S. Patent No. 5,330,768; U.S. Patent No. 4,526,938; U.S. Patent No. 5,889,110; U.S. Patent No. 6,034,175; and European Patent 0258780.

To prepare microspheres, several methods can be employed depending upon the desired application of the delivery vehicles. Suitable methods include, but are not limited to, spray drying, freeze drying, air drying, vacuum drying, fluidized-bed drying, milling, co-precipitation and critical fluid extraction. In the case of spray drying, freeze drying, air drying, vacuum drying, fluidized-bed drying and critical fluid extraction; the components (stabilizing polyol, bioactive material, buffers, etc.) are first dissolved or suspended in aqueous conditions. In the case of milling, the components are mixed in the dried form and milled by any method known in the art. In the case of co-precipitation, the components are mixed in organic conditions and processed as described below. Spray drying can be used to load the stabilizing polyol with the bioactive material. The components are mixed under aqueous conditions and dried using precision nozzles to produce extremely uniform droplets in a drying chamber. Suitable spray drying machines include, but are not limited to, Buchi, NIRO, APV and Lab-plant spray driers used according to the manufacturer's instructions.

The shape of microparticles and nanoparticles may be determined by scanning electron microscopy. Spherically shaped nanoparticles are used in certain embodiments, for circulation through the bloodstream. If desired, the particles may be fabricated using known techniques into other shapes that are more useful for a specific application.

In addition to intracellular delivery of a topoisomerase inhibitor, it also possible that particles of the CDP-topoisomerase inhibitor conjugates, such as microparticles or nanoparticles, may undergo endocytosis, thereby obtaining access to the cell. The frequency of such an endocytosis process will likely depend on the size of any particle.
In one embodiment, the surface charge of the molecule is neutral, or slightly negative. In some embodiments, the zeta potential of the particle surface is from about -80 mV to about 50 mV.

### CDPs, methods of making same, and methods of conjugating CDPs to Topoisomerase inhibitors

Generally, the CDP-topoisomerase inhibitor conjugates, particles and compositions described herein can be prepared in one of two ways: monomers bearing topoisomerase inhibitors, targeting ligands, and/or cyclodextrin moieties can be polymerized, or polymer backbones can be derivatized with topoisomerase inhibitors, targeting ligands, and/or cyclodextrin moieties. Exemplary methods of making CDPs and CDP-topoisomerase inhibitor conjugates, particles and compositions are described, for example, in U.S. Patent No.: 7,270,808.

The CDPs described herein can be made using a variety of methods including those described herein. In some embodiments, a CDP can be made by: providing cyclodextrin moiety precursors; providing comonomer precursors which do not contain cyclodextrin moieties (comonomer precursors); and copolymerizing the said cyclodextrin moiety precursors and comonomer precursors to thereby make a CDP wherein CDP comprises at least four cyclodextrin moieties and at least four comonomers.

In some embodiments, the at least four cyclodextrin moieties and at least four comonomers alternate in the water soluble linear polymer. In some embodiments, the method includes providing cyclodextrin moiety precursors modified to bear one reactive site at each of exactly two positions, and reacting the cyclodextrin moiety precursors with comonomer precursors having exactly two reactive moieties capable of forming a covalent bond with the reactive sites under polymerization conditions that promote reaction of the reactive sites with the reactive moieties to form covalent bonds between the comonomers and the cyclodextrin moieties, whereby a CDP comprising alternating units of a cyclodextrin moiety and a comonomer is produced.

In some embodiments, the cyclodextrin momomers comprise linkers to which the topoisomerase inhibitor maybe further linked.

In some embodiments, the comonomer precursor is a compound containing at least two functional groups through which reaction and thus linkage of the cyclodextrin moieties is achieved. In some embodiments, the functional groups, which maybe the same or different, terminal or internal, of each comonomer precursor comprise an amino, acid, imidazole, hydroxyl, thio, acyl halide, -HC=CH-, -C≡C- group, or derivative thereof. In some embodiments, the two functional groups are the same and are located at termini of the comonomer precursor. In some embodiments, a comonomer contains one or more pendant groups with at least one functional group through which reaction and thus linkage of a therapeutic agent can be achieved. In some embodiments, the functional groups, which may be the same or different, terminal or internal, of each comonomer pendant group comprise an amino, acid, imidazole, hydroxyl, thiol, acyl halide, ethylene, ethyne group, or derivative thereof. In some embodiments, the pendant group is a substituted or unsubstituted branched, cyclic or straight chain C1-C10 alkyl, or arylalkyl optionally containing one or more heteroatoms within the chain or ring.

In some embodiments, the cyclodextrin moiety comprises an alpha, beta, or gamma cyclodextrin moiety.

In some embodiments, the CDP is suitable for the attachment of sufficient topoisomerase inhibitor such that up to at least 3%, 5%, 10%, 11%, 12%, 13%, 14%, 15%, 20%, 25%, 30%, or even 35% by weight of the CDP, when conjugated, is topoisomerase inhibitor.

In some embodiments, the CDP has a molecular weight of 10,000-500,000 amu. In some embodiments, the cyclodextrin moieties make up at least about 2%, 5%, 10%, 20%, 30%, 50% or 80% of the CDP by weight.

In some embodiments, a CDP of the following formula can be made by the scheme below: 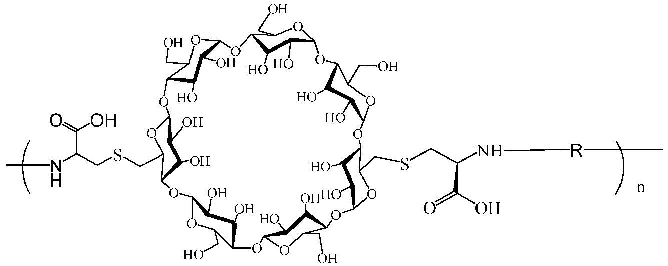 wherein R is of the form: 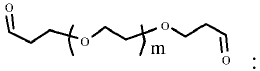 comprising the steps of:
reacting a compound of the formula below: 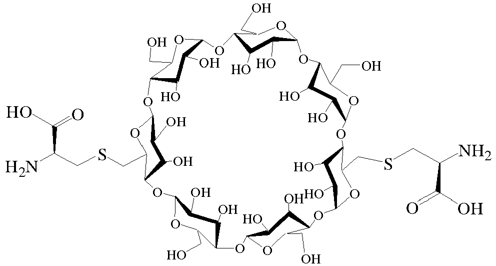 with a compound of the formula below: 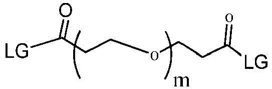 wherein the group 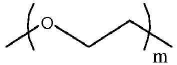 has a Mw of 3.4kDa or less and n is at least four,
in the presence of a non-nucleophilic organic base in a solvent.

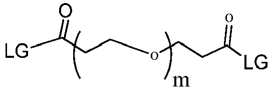

In some embodiments, is

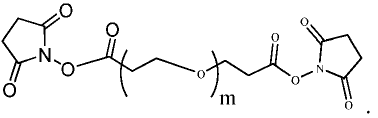

In some embodiments, the solvent is a polar aprotic solvent. In some embodiments, the solvent is DMSO.

In some embodiments, the method also includes the steps of dialysis; and lyophylization.

In some embodiments, a CDP provided below can be made by the following scheme: 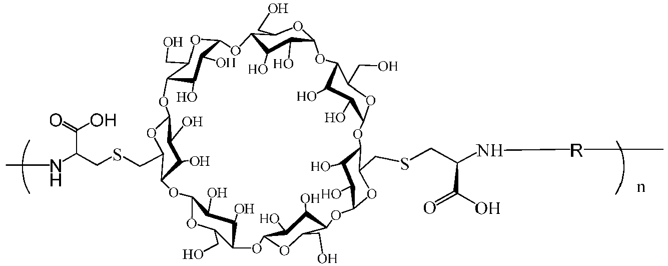 wherein R is of the form:
with a compound provided below: 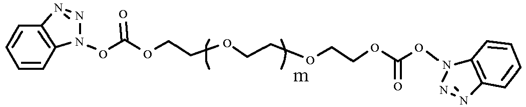 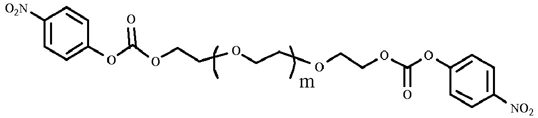 wherein the group 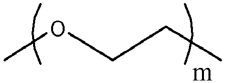 has a Mw of 3.4kDa;
in the presence of a non-nucleophilic organic base in DMSO;
and dialyzing and lyophilizing the following polymer

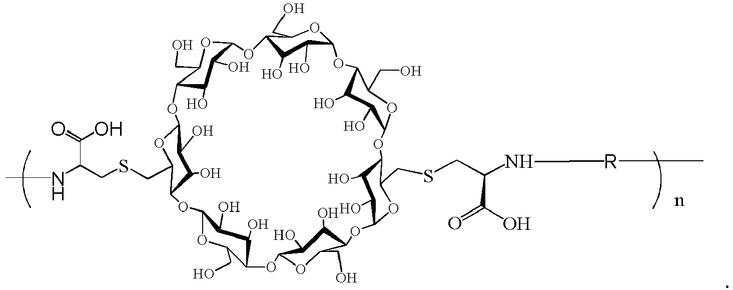

The present disclosure further contemplates CDPs and CDP-conjugates synthesized using CD-biscysteine monomer and a di-NHS ester such as PEG-DiSPA or PEG-BTC as shown in Scheme I. 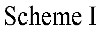 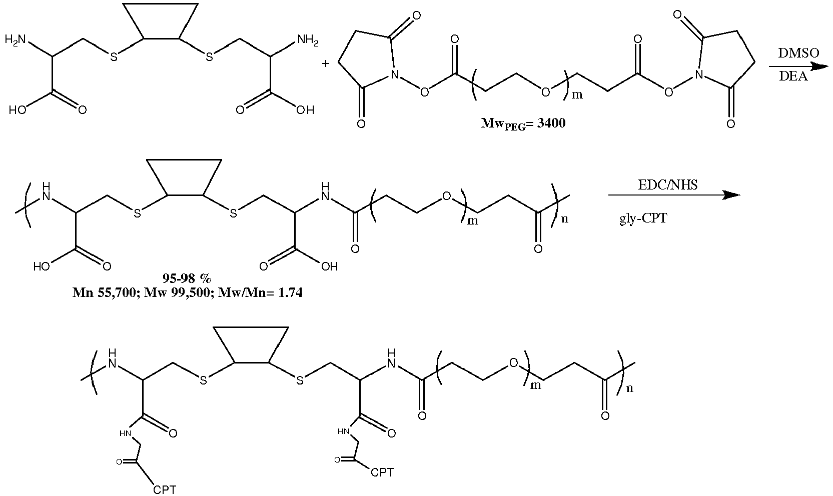 Scheme XIII, as provided above, includes embodiments where gly-CPT is absent in one or more positions as provided above. This can be achieved, for example, when less than 100% yield is achieved when coupling the CPT to the polymer and/or when less than an equivalent amount of CPT is used in the reaction. Accordingly, the loading of the topoisomerase inhibitor such as camptothecin, by weight of the polymer, can vary. Therefore, while Scheme XIII depicts CPT at each cysteine residue of each polymer subunit, the CDP-CPT conjugate can have less than 2 CPT molecules attached to any given polymer subunit of the CDP. For example, in one embodiment, the CDP-CPT conjugate includes several polymer subunits and each of the polymer subunits can independently include two, one or no CPT attached at each cysteine residue of the polymer subunit. In addition, the particles and compositions can include CDP-CPT conjugates having two, one or no CPT attached at each cysteine residue of each polymer subunit of the CDP-CPT conjugate and the conjugates include a mixture of CDP-CPT conjugates that can vary as to the number of CPTs attached to the gly at each of the polymer subunits of the conjugates in the particle or composition.

In some embodiments, a CDP-topoisomerase inhibitor conjugate can be made by providing a CDP comprising cyclodextrin moieties and comonomers which do not contain cyclodextrin moieties (comonomers), wherein the cyclodextrin moieties and comonomers alternate in the CDP and wherein the CDP comprises at least four cyclodextrin moieties and at least four comonomers; and attaching a topoisomerase inhibitor to the CDP.

In some embodiments, one or more of the topoisomerase inhibitor moieties in the CDP- topoisomerase inhibitor conjugate can be replaced with another therapeutic agent, e.g., another anticancer agent or anti-inflammatory agent.

In some embodiments, the topoisomerase inhibitor is attached to the water soluble linear polymer via a linker. In some embodiments, the topoisomerase inhibitor is attached to the water soluble linear polymer through an attachment that is cleaved under biological conditions to release the topoisomerase inhibitor. In some embodiments, the topoisomerase inhibitor is attached to the water soluble linear polymer at a cyclodextrin moiety or a comonomer. In some embodiments, the topoisomerase inhibitor is attached to the water soluble linear polymer via an optional linker to a cyclodextrin moiety or a comonomer.

In some embodiments, the cyclodextrin moieties comprise linkers to which therapeutic agents are linked.

In some embodiments, the CDP is made by a process comprising: providing cyclodextrin moiety precursors, providing comonomer precursors, and copolymerizing said cyclodextrin moiety precursors and comonomer precursors to thereby make a CDP comprising cyclodextrin moieties and comonomers. In some embodiments, the CDP is conjugated with a topoisomerase inhibitor such as camptothecin to provide a CDP-topoisomerase inhibitor conjugate.

In some embodiments, the method includes providing cyclodextrin moiety precursors modified to bear one reactive site at each of exactly two positions, and reacting the cyclodextrin moiety precursors with comonomer precursors having exactly two reactive moieties capable of forming a covalent bond with the reactive sites under polymerization conditions that promote reaction of the reactive sites with the reactive moieties to form covalent bonds between the comonomers and the cyclodextrin moieties, whereby a CDP comprising alternating units of a cyclodextrin moiety and a comonomer is produced.

In some embodiments, the topoisomerase inhibitor is attached to the CDP via a linker. In some embodiments, the linker is cleaved under biological conditions.

In some embodiments, the topoisomerase inhibitor makes up at least 5%, 10%, 11%, 12%, 13%, 14%, 15%, 20%, 25%, 30%, or even 35% by weight of the CDP-topoisomerase inhibitor conjugate.

In some embodiments, the comonomer comprises polyethylene glycol of molecular weight 3,400 Da, the cyclodextrin moiety comprises beta-cyclodextrin, the theoretical maximum loading of camptothecin on a CDP-camptothecin conjugate is 13%, and camptothecin is 6-10% by weight of the CDP-camptothecin conjugate.

In some embodiments, the comonomer precursor is a compound containing at least two functional groups through which reaction and thus linkage of the cyclodextrin moieties is achieved. In some embodiments, the functional groups, which may be the same or different, terminal or internal, of each comonomer precursor comprise an amino, acid, imidazole, hydroxyl, thio, acyl halide, -HC=CH-, -C≡C- group, or derivative thereof. In some embodiments, the two functional groups are the same and are located at termini of the comonomer precursor. In some embodiments, a comonomer contains one or more pendant groups with at least one functional group through which reaction and thus linkage of a therapeutic agent is achieved. In some embodiments, the functional groups, which may be the same or different, terminal or internal, of each comonomer pendant group comprise an amino, acid, imidazole, hydroxyl, thiol, acyl halide, ethylene, ethyne group, or derivative thereof. In some embodiments, the pendant group is a substituted or unsubstituted branched, cyclic or straight chain C1-C10 alkyl, or arylalkyl optionally containing one or more heteroatoms within the chain or ring.

In some embodiments, the cyclodextrin moiety comprises an alpha, beta, or gamma cyclodextrin moiety.

In some embodiments, the topoisomerase inhibitor is poorly soluble in water.

In some embodiments, administration of the CDP-topoisomerase inhibitor conjugate, particle or composition to a subject results in release of the topoisomerase inhibitor over a period of at least 6 hours. In some embodiments, administration of the CDP-topoisomerase inhibitor conjugate, particle or composition to a subject results in release of the topoisomerase inhibitor over a period of 6 hours to a month. In some embodiments, upon administration of the CDP-topoisomerase inhibitor conjugate, particle or composition to a subject the rate of topoisomerase inhibitor release is dependent primarily upon the rate of hydrolysis as opposed to enzymatic cleavage.

In some embodiments, the CDP-topoisomerase inhibitor conjugate, particle or composition has a molecular weight of 10,000-500,000 amu.

In some embodiments, the cyclodextrin moieties make up at least about 2%, 5%, 10%, 20%, 30%, 50% or 80% of the polymer by weight.

In some embodiments, a CDP-polymer conjugate of the following formula can be made as follows: 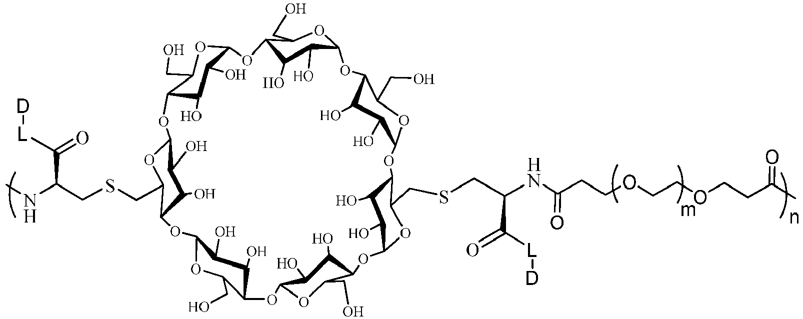 providing a polymer below: 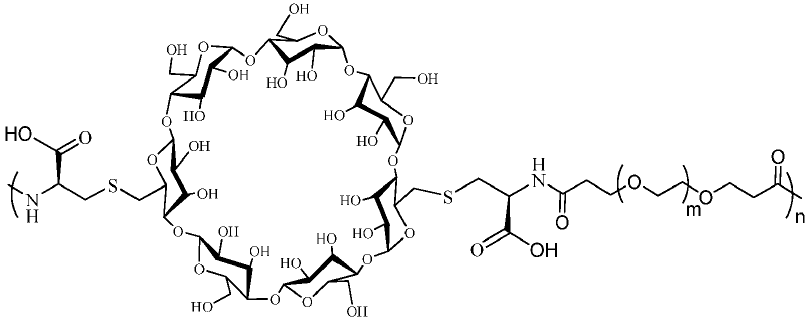 and coupling the polymer with a plurality of L-D moieties, wherein L is a linker, or absent and D is topoisomerase inhibitor such as camptothecin or a camptothecin derivative, to provide: 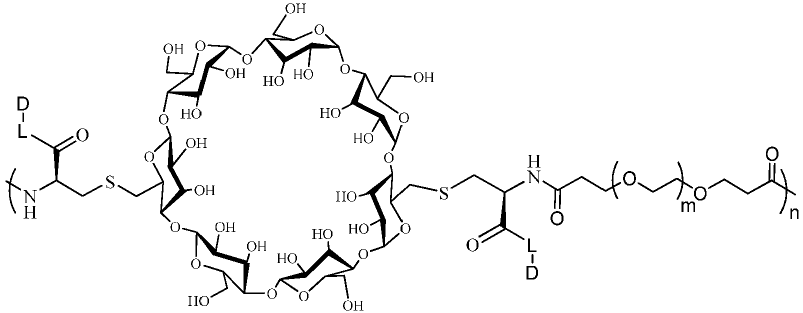 wherein the group 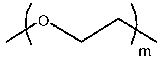 has a Mw of 3.4kDa or less and n is at least 4, wherein on the final product, L can be a linker, a bond, or OH, and D can be a topoisomerase inhibitor (e.g., camptothecin or a camptothecin derivative) or absent.

In some embodiments, one or more of the topoisomerase inhibitor moieties in the CDP-topoisomerase inhibitor conjugate can be replaced with another therapeutic agent, e.g., another anticancer agent or anti-inflammatory agent.

The reaction scheme as provided above includes embodiments where L-D is absent in one or more positions as provided above. This can be achieved, for example, when less than 100% yield is achieved when coupling the topoisomerase inhibitor -linker to the polymer and/or when less than an equivalent amount of topoisomerase inhibitor-linker is used in the reaction. Accordingly, the loading of the topoisomerase inhibitor, by weight of the polymer, can vary, for example, the loading of the topoisomerase inhibitor can be at least about 3% by weight, e.g., at least about 5%, at least about 8%, at least about 10%, at least about 11%, at least about 12%, at least about 13%, at least about 14%, at least about 15%, or at least about 20%.

In some embodiments, at least a portion of the L moieties of L-D is absent. In some embodiments, each L is independently an amino acid or derivative thereof (e.g., glycine).

In some embodiments, the coupling of the polymer with the plurality of L-D moieties results in the formation of a plurality of amide bonds.

In certain instances, the CDPs are random copolymers, in which the different subunits and/or other monomeric units are distributed randomly throughout the polymer chain. Thus, where the formula XₘYₙ-Zₒ appears, wherein X, Y and Z are polymer subunits, these subunits may be randomly interspersed throughout the polymer backbone. In part, the term "random" is intended to refer to the situation in which the particular distribution or incorporation of monomeric units in a polymer that has more than one type of monomeric units is not directed or controlled directly by the synthetic protocol, but instead results from features inherent to the polymer system, such as the reactivity, amounts of subunits and other characteristics of the synthetic reaction or other methods of manufacture, processing, or treatment.

### Pharmaceutical Compositions

In another aspect, the present disclosure provides a composition, e.g., a pharmaceutical composition, comprising a CDP-topoisomerase inhibitor conjugate or particle and a pharmaceutically acceptable carrier or adjuvant.

In some embodiments, a pharmaceutical composition may include a pharmaceutically acceptable salt of a compound described herein, e.g., a CDP-topoisomerase inhibitor conjugate, particle or composition. Pharmaceutically acceptable salts of the compounds described herein include those derived from pharmaceutically acceptable inorganic and organic acids and bases. Examples of suitable acid salts include acetate, adipate, benzoate, benzenesulfonate, butyrate, citrate, digluconate, dodecylsulfate, formate, fumarate, glycolate, hemisulfate, heptanoate, hexanoate, hydrochloride, hydrobromide, hydroiodide, lactate, maleate, malonate, methanesulfonate, 2-naphthalenesulfonate, nicotinate, nitrate, palmoate, phosphate, picrate, pivalate, propionate, salicylate, succinate, sulfate, tartrate, tosylate and undecanoate. Salts derived from appropriate bases include alkali metal (e.g., sodium), alkaline earth metal (e.g., magnesium), ammonium and N-(alkyl)₄⁺ salts. This disclosure also envisions the quaternization of any basic nitrogen-containing groups of the compounds described herein. Water or oil-soluble or dispersible products may be obtained by such quaternization.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gailate, aipha-tocopherol, and the like; and (3) metal chelating agents, such as citric acid, ethylenediamine tetraacetic acid (EDTA), sorbitol, tartaric acid, phosphoric acid, and the like.

A composition may include a liquid used for suspending a CDP-topoisomerase inhibitor conjugate, particle or composition, which may be any liquid solution compatible with the CDP- topoisomerase inhibitor conjugate, particle or composition, which is also suitable to be used in pharmaceutical compositions, such as a pharmaceutically acceptable nontoxic liquid. Suitable suspending liquids including but are not limited to suspending liquids selected from the group consisting of water, aqueous sucrose syrups, corn syrups, sorbitol, polyethylene glycol, propylene glycol, and mixtures thereof.

A composition described herein may also include another component, such as an antioxidant, antibacterial, buffer, bulking agent, chelating agent, an inert gas, a tonicity agent and/or a viscosity agent.

In one embodiment, the CDP-topoisomerase inhibitor conjugate, particle or composition is provided in lyophilized form and is reconstituted prior to administration to a subject. The lyophilized CDP-topoisomerase inhibitor conjugate, particle or composition can be reconstituted by a diluent solution, such as a salt or saline solution, e.g., a sodium chloride solution having a pH between 6 and 9, lactated Ringer's injection solution, or a commercially available diluent, such as PLASMA-LYTE A Injection pH 7.4® (Baxter, Deerfield, IL).

In one embodiment, a lyophilized formulation includes a lyoprotectant or stabilizer to maintain physical and chemical stability by protecting the CDP-topoisomerase inhibitor conjugate, particle or composition from damage from crystal formation and the fusion process during freeze-drying. The lyoprotectant or stabilizer can be one or more of polyethylene glycol (PEG), a PEG lipid conjugate (e.g., PEG-ceramide or D-alpha-tocopheryl polyethylene glycol 1000 succinate), poly(vinyl alcohol) (PVA), poly(vinylpyrrolidone) (PVP), polyoxyethylene esters, poloxomers, Tweens, lecithins, saccharides, oligosaccharides, polysaccharides and polyols (e.g., trehalose, mannitol, sorbitol, lactose, sucrose, glucose and dextran), salts and crown ethers. In one embodiment, the lyoprotectant is mannitol.

In some embodiments, the lyophilized CDP-topoisomerase inhibitor conjugate, particle or composition is reconstituted with a mixture of equal parts by volume of Dehydrated Alcohol, USP and a nonionic surfactant, such as a polyoxyethylated castor oil surfactant available from GAF Corporation, Mount Olive, N.J., under the trademark, Cremophor EL. In some embodiments, the lyophilized CDP-topoisomerase inhibitor conjugate, particle or composition is reconstituted in water for infusion. The lyophilized product and vehicle for reconstitution can be packaged separately in appropriately light-protected vials, e.g., amber or other colored vials. To minimize the amount of surfactant in the reconstituted solution, only a sufficient amount of the vehicle may be provided to form a solution having a concentration of about 2 mg/mL to about 4 mg/mL of the CDP-topoisomerase inhibitor conjugate, particle or composition. Once dissolution of the drug is achieved, the resulting solution is further diluted prior to injection with a suitable parenteral diluent. Such diluents are well known to those of ordinary skill in the art. These diluents are generally available in clinical facilities. It is, however, within the scope of the present disclosure to package the subject CDP-topoisomerase inhibitor conjugate, particle or composition with a third vial containing sufficient parenteral diluent to prepare the final concentration for administration. A typical diluent is Lactated Ringer's Injection.

The final dilution of the reconstituted CDP-topoisomerase inhibitor conjugate, particle or composition may be carried out with other preparations having similar utility, for example, 5% Dextrose Injection, Lactated Ringer's and Dextrose for Injection (D5W), Sterile Water for Injection, and the like. However, because of its narrow pH range, pH 6.0 to 7.5, Lactated Ringer's Injection is most typical. Per 100 mL, Lactated Ringer's Injection contains Sodium Chloride USP 0.6 g, Sodium Lactate 0.31 g, Potassium chloride USP 0.03 g and Calcium Chloride2H2O USP 0.02 g. The osmolarity is 275 mOsmol/L, which is very close to isotonicity.

The compositions may conveniently be presented in unit dosage form and may be prepared by any methods well known in the art of pharmacy. The dosage form can be, e.g., in a bog, e.g., a bag for infusion or intraperitoneal administration. The amount of active ingredient which can be combined with a carrier material to produce a single dosage form will vary depending upon the host being treated, the particular mode of administration. The amount of active ingredient which can be combined
with a carrier material to produce a single dosage form will generally be that amount of the compound which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

### Routes of Administration

The pharmaceutical compositions described herein may be administered orally, parenterally (e.g., via intravenous, subcutaneous, intracutaneous, intramuscular, intraarticular, intraarterial, intraperitoneal, intrasynovial, intrasternal, intrathecal, intralesional or intracranial injection), topically, mucosally (e.g., rectally or vaginally), nasally, buccally, ophthalmically, via inhalation spray (e.g., delivered via nebulzation, propellant or a dry powder device) or via an implanted reservoir. Typically, the compositions are in the form of injectable or infusible solutions. The preferred mode of administration is, e.g., intravenous, subcutaneous, intraperitoneal, intramuscular.

Pharmaceutical compositions suitable for parenteral administration comprise one or more CDP-topoisomerase inhibitor conjugate(s), particle(s) or composition(s) in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the agent from subcutaneous or intramuscular injection. This may be accomplished by the use of a liquid suspension of crystalline or amorphous material having poor water solubility. The rate of absorption of the CDP-topoisomerase inhibitor conjugate, particle or composition then depends upon its rate of dissolution which, in turn, may depend upon crystal size and crystalline form. Alternatively, delayed absorption of a parenterally administered drug form is accomplished by dissolving or suspending the CDP-topoisomerase inhibitor conjugate, particle or composition in an oil vehicle.

Pharmaceutical compositions suitable for oral administration may be in the form of capsules, cachets, pills, tablets, gums, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouthwashes and the like, each containing a predetermined amount of an agent as an active ingredient. A compound may also be administered as a bolus, electuary or paste.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered peptide or peptidomimetic moistened with an inert liquid diluent.

Tablets, and other solid dosage forms, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration include pharmaceutically acceptable emulsions, microemulsions, solutions, suspensions, syrups and elixirs. In addition to the CDP-topoisomerase inhibitor conjugate, particle or composition, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the CDP-topoisomerase inhibitor conjugate, particle or composition may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Pharmaceutical compositions suitable for topical administration are useful when the desired treatment involves areas or organs readily accessible by topical application. For application topically to the skin, the pharmaceutical composition should be formulated with a suitable ointment containing the active components suspended or dissolved in a carrier. Carriers for topical administration of the a particle described herein include, but are not limited to, mineral oil, liquid petroleum, white petroleum, propylene glycol, polyoxyethylene polyoxypropylene compound, emulsifying wax and water. Alternatively, the pharmaceutical composition can be formulated with a suitable lotion or cream containing the active particle suspended or dissolved in a carrier with suitable emulsifying agents. Suitable carriers include, but are not limited to, mineral oil, sorbitan monostearate, polysorbate 60, cetyl esters wax, cetearyl alcohol, 2-octyldodecanol, benzyl alcohol and water. The pharmaceutical compositions described herein may also be topically applied to the lower intestinal tract by rectal suppository formulation or in a suitable enema formulation. Topically-transdermal patches are also included herein.

The pharmaceutical compositions described herein may be administered by nasal aerosol or inhalation. Such compositions are prepared according to techniques well-known in the art of pharmaceutical formulation and maybe prepared as solutions in saline, employing benzyl alcohol or other suitable preservatives, absorption promoters to enhance bioavailability, fluorocarbons, and/or other solubilizing or dispersing agents known in the art.

The pharmaceutical compositions described herein may also be administered in the form of suppositories for rectal or vaginal administration. Suppositories may be prepared by mixing one or more CDP-topoisomerase inhibitor conjugate, particle or composition described herein with one or more suitable non-irritating excipients which is solid at room temperature, but liquid at body temperature. The composition will therefore melt in the rectum or vaginal cavity and release the CDP-topoisomerase inhibitor conjugate, particle or composition. Such materials include, for example, cocoa butter, polyethylene glycol, a suppository wax or a salicylate. Compositions of the present disclosure which are suitable for vaginal administration also include pessaries, tampons, creams, gels, pastes, foams or spray formulations containing such carriers as are known in the art to be appropriate.

Ophthalmic formulations, eye ointments, powders, solutions and the like, are also contemplated as being within the scope of the disclosure.

### Dosages and Dosing Regimens

The CDP-topoisomerase inhibitor conjugate, particle or composition can be formulated into pharmaceutically acceptable dosage forms by conventional methods known to those of skill in the art.

Actual dosage levels of the active ingredients in the pharmaceutical compositions of this disclosure may be varied so as to obtain an amount of the active ingredient which is effective to achieve the desired therapeutic response for a particular subject, composition, and mode of administration, without being toxic to the subject.

In one embodiment, the CDP-topoisomerase inhibitor conjugate, particle or composition is administered to a subject at a dosage of, e.g., about 1 to 40 mg/m², about 3 to 35 mg/m², about 9 to 40 mg/m², e.g., about 1, 3, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 mg/m² of the topoisomerase inhibitor. Administration can be at regular intervals, such as weekly, or every 2, 3, 4, 5 or 6 weeks. The administration can be over a period of from about 10 minutes to about 6 hours, e.g., from about 30 minutes to about 2 hours, from about 45 minutes to 90 minutes, e.g., about 30 minutes, 45 minutes, 1 hour, 2 hours, 3 hours, 4 hours, 5 hours or more. The CDP-topoisomerase inhibitor conjugate, particle or composition can be administered, e.g., by intravenous or intraperitoneal administration.

In one embodiment, the CDP-topoisomerase inhibitor conjugate, particle or composition is administered as a bolus infusion or intravenous push, e.g., over a period of 15 minutes, 10 minutes, 5 minutes or less. In one embodiment, the CDP-topoisomerase inhibitor conjugate, particle or composition is administered in an amount such the desired dose of the agent is administered. Preferably the dose of the CDP-topoisomerase inhibitor conjugate, particle or composition is a dose described herein.

In one embodiment, the subject receives 1, 2, 3, up to 10 treatments, or more, or until the disorder or a symptom of the disorder is cured, healed, alleviated, relieved, altered, remedied, ameliorated, palliated, improved or affected. For example, the subject receives an infusion once every 1, 2, 3 or 4 weeks until the disorder or a symptom of the disorder is cured, healed, alleviated, relieved, altered, remedied, ameliorated, palliated, improved or affected. Preferably, the dosing schedule is a dosing schedule described herein.

The CDP-topoisomerase inhibitor conjugate, particle or composition can be administered as a first line therapy, e.g., alone or in combination with an additional agent or agents. In other embodiments, a CDP-topoisomerase inhibitor conjugate, particle or composition is administered after a subject has developed resistance to, has failed to respond to or has relapsed after a first line therapy. The CDP-topoisomerase inhibitor conjugate, particle or composition can be administered in combination with a second agent. Preferably, the CDP-topoisomerase inhibitor conjugate, particle or composition is administered in combination with a second agent described herein.

### Kits

A CDP-topoisomerase inhibitor conjugate, particle or composition described herein may be provided in a kit. The kit includes a CDP-topoisomerase inhibitor conjugate, particle or composition described herein and, optionally, a container, a pharmaceutically acceptable carrier and/or informational material. The informational material can be descriptive, instructional, marketing or other material that relates to the methods described herein and/or the use of the CDP-topoisomerase inhibitor conjugate, particle or composition for the methods described herein.

The informational material of the kits is not limited in its form. In one embodiment, the informational material can include information about production of the CDP-topoisomerase inhibitor conjugate, particle or composition, physical properties of the CDP-topoisomerase inhibitor conjugate, particle or composition, concentration, date of expiration, batch or production site information, and so forth. In one embodiment, the informational material relates to methods for administering the CDP-topoisomerase inhibitor conjugate, particle or composition, e.g., by a route of administration described herein and/or at a dose and/or dosing schedule described herein.

In one embodiment, the informational material can include instructions to administer a CDP-topoisomerase inhibitor conjugate, particle or composition described herein in a suitable manner to perform the methods described herein, e.g., in a suitable dose, dosage form, or mode of administration (e.g., a dose, dosage form, or mode of administration described herein). In another embodiment, the informational material can include instructions to administer a CDP-topoisomerase inhibitor conjugate, particle or composition described herein to a suitable subject, e.g., a human, e.g., a human having or at risk for a disorder described herein. In another embodiment, the informational material can include instructions to reconstitute a CDP-topoisomerase inhibitor conjugate, particle or composition described herein into a pharmaceutically acceptable composition.

In one embodiment, the kit includes instructions to use the CDP-topoisomerase inhibitor conjugate, particle or composition, such as for treatment of a subject. The instructions can include methods for reconstituting or diluting the CDP-topoisomerase inhibitor conjugate, particle or composition for use with a particular subject or in combination with a particular chemotherapeutic agent. The instructions can also include methods for reconstituting or diluting the CDP-topoisomerase inhibitor conjugate, particle or composition for use with a particular means of administration, such as by intravenous infusion or intraperitoneal administration.

In another embodiment, the kit includes instructions for treating a subject with a particular indication, such as a particular cancer, or a cancer at a particular stage. For example, the instructions can be for a cancer or cancer at stage described herein, e.g., lung cancer (e.g., non small cell lung cancer and/or small cell lung cancer, e.g., squamous cell non-small cell and/or small cell lung cancer) or ovarian cancer. The instructions may also address first line treatment of a subject who has a particular cancer, or cancer at a stage described herein. The instructions can also address treatment of a subject who has been non-responsive to a first line therapy or has become sensitive (e.g., has one or more unacceptable side effect) to a first line therapy, such as a taxane, an anthracycline, an antimetabolite, a vinca alkaloid, a vascular endothelial growth factor (VEGF) pathway inhibitor, an epidermal growth factor (EGF) pathway inhibitor, an alkylating agent, a platinum-based agent, a vinca alkaloid. In another embodiment, the instructions will describe treatment of selected subjects with the CDP-topoisomerase inhibitor conjugate, particle or composition. For example, the instructions can describe treatment of one or more of: a subject having a cancer that has increased levels of KRAS and/or ST expression, e.g., as compared to a reference standard.

The informational material of the kits is not limited in its form. In many cases, the informational material, e.g., instructions, is provided in printed matter, e.g., a printed text, drawing, and/or photograph, e.g., a label or printed sheet. However, the informational material can also be provided in other formats, such as Braille, computer readable material, video recording, or audio recording. In another embodiment, the informational material of the kit is contact information, e.g., a physical address, email address, website, or telephone number, where a user of the kit can obtain substantive information about a CDP-topoisomerase inhibitor conjugate, particle or composition described herein and/or its use in the methods described herein. The informational material can also be provided in any combination of formats.

In addition to a CDP-topoisomerase inhibitor conjugate, particle or composition described herein, the composition of the kit can include other ingredients, such as a surfactant, a lyoprotectant or stabilizer, an antioxidant, an antibacterial agent, a bulking agent, a chelating agent, an inert gas, a tonicity agent and/or a viscosity agent, a solvent or buffer, a stabilizer, a preservative, a flavoring agent (e.g., a bitter antagonist or a sweetener), a fragrance, a dye or coloring agent, for example, to tint or color one or more components in the kit, or other cosmetic ingredient, a pharmaceutically acceptable carrier and/or a second agent for treating a condition or disorder described herein. Alternatively, the other ingredients can be included in the kit, but in different compositions or containers than a CDP-topoisomerase inhibitor conjugate, particle or composition described herein. In such embodiments, the kit can include instructions for admixing a CDP-topoisomerase inhibitor conjugate, particle or composition described herein and the other ingredients, or for using a CDP-topoisomerase inhibitor conjugate, particle or composition described herein together with the other ingredients. For example, the kit can include an agent which reduces or inhibits one or more symptom of hypersensitivity, a polysaccharide, and/or an agent which increases urinary excretion and/or neutralizes one or more urinary metabolite.

In another embodiment, the kit includes a second therapeutic agent, such as a second chemotherapeutic agent, e.g., a chemotherapeutic agent or combination of chemotherapeutic agents described herein. In one embodiment, the second agent is in lyophilized or in liquid form. In one embodiment, the CDP-topoisomerase inhibitor conjugate, particle or composition and the second therapeutic agent are in separate containers, and in another embodiment, the CDP-topoisomerase inhibitor conjugate, particle or composition and the second therapeutic agent are packaged in the same container.

In some embodiments, a component of the kit is stored in a sealed vial, e.g., with a rubber or silicone closure (e.g., a polybutadiene or polyisoprene closure). In some embodiments, a component of the kit is stored under inert conditions (e.g., under Nitrogen or another inert gas such as Argon). In some embodiments, a component of the kit is stored under anhydrous conditions (e.g., with a desiccant). In some embodiments, a component of the kit is stored in a light blocking container such as an amber vial.

A CDP-topoisomerase inhibitor conjugate, particle or composition described herein can be provided in any form, e.g., liquid, frozen, dried or lyophilized form. It is preferred that a composition including the conjugate, particle or composition, e.g., a composition comprising a particle or particles that include a conjugate described herein be substantially pure and/or sterile. When a CDP-topoisomerase inhibitor conjugate, particle or composition described herein is provided in a liquid solution, the liquid solution preferably is an aqueous solution, with a sterile aqueous solution being preferred. In one embodiment, the CDP-topoisomerase inhibitor conjugate, particle or composition is provided in lyophilized form and, optionally, a diluent solution is provided for reconstituting the lyophilized agent. The diluent can include for example, a salt or saline solution, e.g., a sodium chloride solution having a pH between 6 and 9, lactated Ringer's injection solution, D5W, or PLASMA-LYTE A Injection pH 7.4® (Baxter, Deerfield, IL).

The kit can include one or more containers for the composition containing a CDP-topoisomerase inhibitor conjugate, particle or composition described herein. In some embodiments, the kit contains separate containers, dividers or compartments for the composition and informational material. For example, the composition can be contained in a bottle, vial, IV admixture bag, IV infusion set, piggyback set or syringe, and the informational material can be contained in a plastic sleeve or packet. In other embodiments, the separate elements of the kit are contained within a single, undivided container. For example, the composition is contained in a bottle, vial or syringe that has attached thereto the informational material in the form of a label. In some embodiments, the kit includes a plurality (e.g., a pack) of individual containers, each containing one or more unit dosage forms (e.g., a dosage form described herein) of a CDP-topoisomerase inhibitor conjugate, particle or composition described herein. For example, the kit includes a plurality of syringes, ampules, foil packets, or blister packs, each containing a single unit dose of a particle described herein. The containers of the kits can be air tight, waterproof (e.g., impermeable to changes in moisture or evaporation), and/or light-tight.

The kit optionally includes a device suitable for administration of the composition, e.g., a syringe, inhalant, pipette, forceps, measured spoon, dropper (e.g., eye dropper), swab (e.g., a cotton swab or wooden swab), or any such delivery device. In one embodiment, the device is a medical implant device, e.g., packaged for surgical insertion.

### Combination therapy

The CDP-topoisomerase inhibitor conjugate, particle or composition may be used in combination with other known therapies. Administered "in combination", as used herein, means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

The CDP-topoisomerase inhibitor conjugate, particle or composition and the at least one additional therapeutic agent can be administered simultaneously, in the same or in separate compositions, or sequentially. For sequential administration, the CDP-topoisomerase inhibitor conjugate, particle or composition can be administered first, and the additional agent can be administered second, or the order of administration can be reversed.

In some embodiments, the CDP-topoisomerase inhibitor conjugate, particle or composition is administered in combination with other therapeutic treatment modalities, including surgery, radiation, cryosurgery, and/or thermotherapy. Such combination therapies may advantageously utilize lower dosages of the administered agent and/or other chemotherapeutic agent, thus avoiding possible toxicities or complications associated with the various monotherapies. The phrase "radiation" includes, but is not limited to, external-beam therapy which involves three dimensional, conformal radiation therapy where the field of radiation is designed to conform to the volume of tissue treated; interstitial-radiation therapy where seeds of radioactive compounds are implanted using ultrasound guidance; and a combination of external-beam therapy and interstitial-radiation therapy.

In some embodiments, the CDP-topoisomerase inhibitor conjugate, particle or composition is administered with at least one additional therapeutic agent, such as a chemotherapeutic agent. In certain embodiments, the CDP-topoisomerase inhibitor conjugate, particle or composition is administered in combination with one or more additional chemotherapeutic agent, e.g., with one or more chemotherapeutic agents described herein. Exemplary classes of chemotherapeutic agents include, e.g., the following:
alkylating agents (including, without limitation, nitrogen mustards, ethylenimine derivatives, alkyl sulfonates, nitrosoureas and triazenes): uracil mustard (Aminouracil Mustard®, Chlorethaminacil®, Demethyldopan®, Desmethyldopan®, Haemanthamine®, Nordopan®, Uracil nitrogen mustard®, Uracillost®, Uracilmostaza®, Uramustin®, Uramustine®), chlormethine (Mustargen®), cyclophosphamide (Cytoxan®, Neosar®, Clafen®, Endoxan®, Procytox®, Revimmune™), ifosfamide (Mitoxana®), melphalan (Alkeran®), Chlorambucil (Leukeran®), pipobroman (Amedel®, Vercyte®), triethylenemelamine (Hemel®, Hexalen®, Hexastat®), triethylenethiophosphoramine, Temozolomide (Temodar®), thiotepa (Thioplex®), busulfan (Busilvex®, Myleran®), carmustine (BiCNU®), lomustine (CeeNU®), streptozocin (Zanosar®), and Dacarbazine (DTIC-Dome®).

anti-EGFR antibodies (e.g., cetuximab (Erbitux®) and panitumumab (Vectibix®).

anti-HER-2 antibodies (e.g., trastuzumab (Herceptin®).

antimetabolites (including, without limitation, folic acid antagonists (also referred to herein as antifolates), pyrimidine analogs, purine analogs and adenosine deaminase inhibitors): methotrexate (Rheumatrex®, Trexall®), 5-fluorouracil (Adrucil®, Efudex®, Fluoroplex®), floxuridine (FUDF®), cytarabine (Cytosar-U®, Tarabine PFS), 6-mercaptopurine (Puri-Nethol®)), 6-thioguanine (Thioguanine Tabloid®), fludarabine phosphate (Fludara®), pentostatin (Nipent®), pemetrexed (Alimta®), raltitrexed (Tomudex®), cladribine (Leustatin®), clofarabine (Clofarex®, Clolar®), mercaptopurine (Puri-Nethol®), capecitabine (Xeloda®), nelarabine (Arranon®), azacitidine (Vidaza®) and gemcitabine (Gemzar®). Preferred antimetabolites include, e.g., 5-fluorouracil (Adrucil®, Efudex®, Fluoroplex®), floxuridine (FUDF®), capecitabine (Xeloda®), pemetrexed (Alimta®), raltitrexed (Tomudex®) and gemcitabine (Gemzar®).

vinca alkaloids: vinblastine (Velban®, Velsar®), vincristine (Vincasar®, Oncovin®), vindesine (Eldisine®), vinorelbine (Navelbine®).

platinum-based agents: carboplatin (Paraplat®, Paraplatin®), cisplatin (Platinol®), oxaliplatin (Eloxatin®).

anthracyclines: daunorubicin (Cerubidine®, Rubidomycin®), doxorubicin (Adriamycin®), epirubicin (Ellence®), idarubicin (Idamycin®), mitoxantrone (Novantrone®), valrubicin (Valstar®). Preferred anthracyclines include daunorubicin (Cerubidine®, Rubidomycin®) and doxorubicin (Adriamycin®).

topoisomerase inhibitors: topotecan (Hycamtin®), irinotecan (Camptosar®), etoposide (Toposar®, VePesid®), teniposide (Vumon®), lamellarin D, SN-38, camptothecin.

taxanes: paclitaxel (Taxol®), docetaxel (Taxotere®), larotaxel, cabazitaxel.

epothilones: ixabepilone, epothilone B, epothilone D, BMS310705, dehydelone, ZK-Epothilone (ZK-EPO).

poly ADP-ribose polymerase (PARP) inhibitors: (e.g., BSI 201, Olaparib (AZD-2281), ABT-888, AG014699, CEP 9722, MK 4827, KU-0059436 (AZD2281), LT-673, 3-aminobenzamide).

antibiotics: actinomycin (Cosmegen®), bleomycin (Blenoxane®), hydroxyurea (Droxia®, Hydrea®), mitomycin (Mitozytrex®, Mutamycin®).

immunomodulators: lenalidomide (Revlimid®), thalidomide (Thalomid®).

immune cell antibodies: alemtuzamab (Campath®), gemtuzumab (Myelotarg®), rituximab (Rituxan®), tositumomab (Bexxar®).

interferons (e.g., IFN-alpha (Alferon®, Roferon-A®, Intron®-A) or IFN-gamma (Actimmune®)).

interleukins: IL-1, IL-2 (Proleukin®), IL-24, IL-6 (Sigosix®), IL-12.

HSP90 inhibitors (e.g., geldanamycin or any of its derivatives). In certain embodiments, the HSP90 inhibitor is selected from geldanamycin, 17-alkylamino-17-desmethoxygeldanamycin ("17-AAG") or 17-(2-dimethylaminoethyl)amino-17-desmethoxygeldanamycin ("17-DMAG").

angiogenesis inhibitors which include, without limitation A6 (Angstrom Pharmacueticals), ABT-510 (Abbott Laboratories), ABT-627 (Atrasentan) (Abbott Laboratories/Xinlay), ABT-869 (Abbott Laboratories), Actimid (CC4047, Pomalidomide) (Celgene Corporation), AdGVPEDF.11D (GenVec), ADH-1 (Exherin) (Adherex Technologies), AEE788 (Novartis), AG-013736 (Axitinib) (Pfizer), AG3340 (Prinomastat) (Agouron Pharmaceuticals), AGX1053 (AngioGenex), AGX51 (AngioGenex), ALN-VSP (ALN-VSP 02) (Alnylam Pharmaceuticals), AMG 386 (Amgen), AMG706 (Amgen), Apatinib (YN968D1) (Jiangsu Hengrui Medicine), AP23573 (Ridaforolimus/MK8669) (Ariad Pharmaceuticals), AQ4N (Novavea), ARQ 197 (ArQule), ASA404 (Novartis/Antisoma), Atiprimod (Callisto Pharmaceuticals), ATN-161 (Attenuon), AV-412 (Aveo Pharmaceuticals), AV-951 (Aveo Pharmaceuticals), Avastin (Bevacizumab) (Genentech), AZD2171 (Cediranib/Recentin) (AstraZeneca), BAY 57-9352 (Telatinib) (Bayer), BEZ235 (Novartis), BIBF1120 (Boehringer Ingelheim Pharmaceuticals), BIBW 2992 (Boehringer Ingelheim Pharmaceuticals), BMS-275291 (Bristol-Myers Squibb), BMS-582664 (Brivanib) (Bristol-Myers Squibb), BMS-690514 (Bristol-Myers Squibb), Calcitriol, CCI-779 (Torisel) (Wyeth), CDP-791 (ImClone Systems), Ceflatonin (Homoharringtonine/HHT) (ChemGenex Therapeutics), Celebrex (Celecoxib) (Pfizer), CEP-7055 (Cephalon/Sanofi), CHIR-265 (Chiron Corporation), NGR-TNF, COL-3 (Metastat) (Collagenex Pharaceuticals), Combretastatin (Oxigene), CP-751,871(Figitumumab) (Pfizer), CP-547,632 (Pfizer), CS-7017 (Daiichi Sankyo Pharma), CT-322 (Angiocept) (Adnexus), Curcumin, Dalteparin (Fragmin) (Pfizer), Disulfiram (Antabuse), E7820 (Eisai Limited), E7080 (Eisai Limited), EMD 121974(Cilengitide) (EMD Pharmaceuticals), ENMD-1198 (EntreMed), ENMD-2076 (EntreMed), Endostar (Simcere), Erbitux (ImClone/Bristol-Myers Squibb), EZN-2208 (Enzon Pharmaceuticals), EZN-2968 (Enzon Pharmaceuticals), GC1008 (Genzyme), Genistein, GSK1363089(Foretinib) (GlaxoSmithKline), GW786034 (Pazopanib) (GlaxoSmithKline), GT-111 (Vascular Biogenics Ltd.), IMC--1121B (Ramucirumab) (ImClone Systems), IMC-18F1 (ImClone Systems), IMC-3G3 (ImClone LLC), INCB007839 (Incyte Corporation), INGN 241 (Introgen Therapeutics), Iressa (ZD1839/Gefitinib), LBH589 (Faridak/Panobinostst) (Novartis), Lucentis (Ranibizumab) (Genentech/Novartis), LY317615 (Enzastaurin) (Eli Lilly and Company), Macugen (Pegaptanib) (Pfizer), MEDI522 (Abegrin) (MedImmune), MLN518(Tandutinib) (Millennium), Neovastat (AE941/Benefin) (Aeterna Zentaris), Nexavar (Bayer/Onyx), NM-3 (Genzyme Corporation), Noscapine (Cougar Biotechnology), NPI-2358 (Nereus Pharmaceuticals), OSI-930 (OSI), Palomid 529 (Paloma Pharmaceuticals, Inc.), Panzem Capsules (2ME2) (EntreMed), Panzem NCD (2ME2) (EntreMed), PF-02341066 (Pfizer), PF-04554878 (Pfizer), PI-88 (Progen Industries/Medigen Biotechnology), PKC412 (Novartis), Polyphenon E (Green Tea Extract) (Polypheno E International, Inc), PPI-2458 (Praecis Pharmaceuticals), PTC299 (PTC Therapeutics), PTK787 (Vatalanib) (Novartis), PXD101 (Belinostat) (CuraGen Corporation), RAD001 (Everolimus) (Novartis), RAF265 (Novartis), Regorafenib (BAY73-4506) (Bayer), Revlimid (Celgene), Retaane (Alcon Research), SN38 (Liposomal) (Neopharm), SNS-032 (BMS-387032) (Sunesis), SOM230(Pasireotide) (Novartis), Squalamine (Genaera), Suramin, Sutent (Pfizer), Tarceva (Genentech), TB-403 (Thrombogenics), Tempostatin (Collard Biopharmaceuticals), Tetrathiomolybdate (Sigma-Aldrich), TG100801 (TargeGen), Thalidomide (Celgene Corporation), Tinzaparin Sodium, TKI258 (Novartis), TRC093 (Tracon Pharmaceuticals Inc.), VEGF Trap (Aflibercept) (Regeneron Pharmaceuticals), VEGF Trap-Eye (Regeneron Pharmaceuticals), Veglin (VasGene Therapeutics), Bortezomib (Millennium), XL184 (Exelixis), XL647 (Exelixis), XL784 (Exelixis), XL820 (Exelixis), XL999 (Exelixis), ZD6474 (AstraZeneca), Vorinostat (Merck), and ZSTK474.

anti-androgens which include, without limitation nilutamide (Nilandron®) and bicalutamide (Caxodex®).

antiestrogens which include, without limitation tamoxifen (Nolvadex®), toremifene (Fareston®), letrozole (Femara®), testolactone (Teslac®), anastrozole (Arimidex®), bicalutamide (Casodex®), exemestane (Aromasin®), flutamide (Eulexin®), fulvestrant (Faslodex®), raloxifene (Evista®, Keoxifene®) and raloxifene hydrochloride.

anti-hypercalcaemia agents which include without limitation gallium (III) nitrate hydrate (Ganite®) and pamidronate disodium (Aredia®).

apoptosis inducers which include without limitation ethanol, 2-[[3-(2,3-dichlorophenoxy)propyl]amino]-(9Cl), gambogic acid, embelin and arsenic trioxide (Trisenox®).

Aurora kinase inhibitors which include without limitation binucleine 2.

Bruton's tyrosine kinase inhibitors which include without limitation terreic acid.

calcineurin inhibitors which include without limitation cypermethrin, deltamethrin, fenvalerate and tyrphostin 8.

CaM kinase II inhibitors which include without limitation 5-Isoquinolinesulfonic acid, 4-[{2S)-2-[(5-isoquinolinylsulfonyl)methylamino]-3-oxo-3-{4-phenyl-1-piperazinyl)propyl]phenyl ester and benzenesulfonamide.

CD45 tyrosine phosphatase inhibitors which include without limitation phosphonic acid.

CDC25 phosphatase inhibitors which include without limitation 1,4-naphthalene dione, 2,3-bis[(2-hydroxyethyl)thio]-(9Cl).

CHK kinase inhibitors which include without limitation debromohymenialdisine.

cyclooxygenase inhibitors which include without limitation 1H-indole-3-acetamide, 1-(4-chlorobenzoyl)-5-methoxy-2-methyl-N-(2-phenylethyl)-(9Cl), 5-alkyl substituted 2-arylaminophenylacetic acid and its derivatives (e.g., celecoxib (Celebrex®), rofecoxib (Vioxx®), etoricoxib (Arcoxia®), lumiracoxib (Prexige®), valdecoxib (Bextra®) or 5-alkyl-2-arylaminophenylacetic acid).

cRAF kinase inhibitors which include without limitation 3-(3,5-dibromo-4-hydroxybenzylidene)-5-iodo-1,3-dihydroindol-2-one and benzamide, 3-(dimethylamino)-N-[3-[(4-hydroxybenzoyl)amino]-4-methylphenyl]-(9Cl).

cyclin dependent kinase inhibitors which include without limitation olomoucine and its derivatives, purvalanol B, roascovitine (Seliciclib®), indirubin, kenpaullone, purvalanol A and indirubin-3'-monooxime.

cysteine protease inhibitors which include without limitation 4-morpholinecarboxamide, N-[(1S)-3-fluoro-2-oxo-1-(2-phenylethyl)propyl]amino]-2-oxo-1-(phenylmethyl)ethyl]-(9Cl).

DNA intercalators which include without limitation plicamycin (Mithracin®) and daptomycin (Cubicin®).

DNA strand breakers which include without limitation bleomycin (Blenoxane®).

E3 ligase inhibitors which include without limitation N-((3,3,3-trifluoro-2-trifluoromethyl)propionyl)sulfanilamide.

EGF Pathway Inhibitors which include, without limitation tyrphostin 46, EKB-569, erlotinib (Tarceva®), gefitinib (Iressa®), lapatinib (Tykerb®) and those compounds that are generically and specifically disclosed in WO 97/02266, EP 0 564 409, WO 99/03854, EP 0 520 722, EP 0 566 226, EP 0 787 722, EP 0 837 063, US 5,747,498, WO 98/10767, WO 97/30034, WO 97/49688, WO 97/38983 and WO 96/33980.

farnesyltransferase inhibitors which include without limitation A-hydroxyfarnesylphosphonic acid, butanoic acid, 2-[(2S)-2-[[(2S,3S)-2-[[(2R)-2-amino-3-mercaptopropyl]amino]-3-methylpentyl]oxy]-1-oxo-3-phenylpropyl]amino]-4-(methylsulfonyl)-1-methylethylester (2S)-(9Cl), and manumycin A.

Flk-1 kinase inhibitors which include without limitation 2-propenamide, 2-cyano-3-[4-hydroxy-3,5-bis(1-methylethyl)phenyl]-N-(3-phenylpropyl)-(2E)-(9Cl).

glycogen synthase kinase-3 (GSK3) inhibitors which include without limitation indirubin-3' -monooxime.

histone deacetylase (HDAC) inhibitors which include without limitation suberoylanilide hydroxamic acid (SAHA), [4-(2-amino-phenylcarbamoyl)-benzyl]-carbamic acid pyridine-3-ylmethylester and its derivatives, butyric acid, pyroxamide, trichostatin A, oxamflatin, apicidin, depsipeptide, depudecin, trapoxin and compounds disclosed in WO 02/22577.

I-kappa B-alpha kinase inhibitors (IKK) which include without limitation 2-propenenitrile, 3-[(4-methylphenyl)sulfonyl]-(2E)-(9Cl).

imidazotetrazinones which include without limitation temozolomide (Methazolastone®, Temodar® and its derivatives (e.g., as disclosed generically and specifically in US 5,260,291) and Mitozolomide.

insulin tyrosine kinase inhibitors which include without limitation hydroxyl-2-naphthalenylmethylphosphonic acid.

c-Jun-N-terminal kinase (JNK) inhibitors which include without limitation pyrazoleanthrone and epigallocatechin gallate.

mitogen-activated protein kinase (MAP) inhibitors which include without limitation benzenesulfonamide, N-[2-[[[3-(4-chlorophenyl)-2-propenyl]methyl]amino]methyl]phenyl]-N-(2-hydroxyethyl)-4-methoxy-(9Cl).

MDM2 inhibitors which include without limitation trans-4-iodo, 4'-boranyl-chalcone.

MEK inhibitors which include without limitation butanedinitrile, bis[amino[2-aminophenyl)thio]methylene]-(9Cl).

MMP inhibitors which include without limitation Actinonin, epigallocatechin gallate, collagen peptidomimetic and non-peptidomimetic inhibitors, tetracycline derivatives marimastat (Marimastat®), prinomastat, incyclinide (Metastat®), shark cartilage extract AE-941 (Neovastat®), Tanomastat, TAA211, MMI270B or AAJ996.

mTor inhibitors which include without limitation rapamycin (Rapamune®), and analogs and derivatives thereof, AP23573 (also known as ridaforolimus, deforolimus, or MK-8669), CCI-779 (also known as temsirolimus) (Torisel®) and SDZ-RAD.

NGFR tyrosine kinase inhibitors which include without limitation tyrphostin AG 879.

p38 MAP kinase inhibitors which include without limitation Phenol, 4-[4-(4-fluorophenyl)-5-(4-pyridinyl)-1H-imidazol-2-yl]-(9Cl), and benzamide, 3-(dimethylamino)-N-[3-[(4-hydroxylbenzoyl)amino]-4-methylphenyl]-(9Cl).

p56 tyrosine kinase inhibitors which include without limitation damnacanthal and tyrphostin 46.

PDGF pathway inhibitors which include without limitation tyrphostin AG 1296, tyrphostin 9, 1,3-butadiene-1,1,3-tricarbonitrile, 2-amino-4-(1H-indol-5-yl)-(9Cl), imatinib (Gleevec®) and gefitinib (Iressa®) and those compounds generically and specifically disclosed in European Patent No.: 0 564 409 and PCT Publication No.: WO 99/03854.

phosphatidylinositol 3-kinase inhibitors which include without limitation wortmannin, and quercetin dihydrate.

phosphatase inhibitors which include without limitation cantharidic acid, cantharidin, and L-leucinamide.

protein phosphatase inhibitors which include without limitation cantharidic acid, cantharidin, L-P-bromotetramisole oxalate, 2(5H)-furanone, 4-hydroxy-5-(hydroxymethyl)-3-(1-oxohexadecyl)-(5R)-(9Cl) and benzylphosphonic acid.

PKC inhibitors which include without limitation 1-H-pyrollo-2,5-dione,3-[1-[3-(dimethylamino)propyl]-1H-indol-3-yl]-4-(1H-indol-3-yl)-(9Cl), Bisindolylmaleimide IX, Sphinogosine, staurosporine, and Hypericin.

PKC delta kinase inhibitors which include without limitation rottlerin.

polyamine synthesis inhibitors which include without limitation DMFO.

proteasome inhibitors which include, without limitation aclacinomycin A, gliotoxin and bortezomib (Velcade®).

PTP1B inhibitors which include without limitation L-leucinamide.

protein tyrosine kinase inhibitors which include, without limitation tyrphostin Ag 216, tyrphostin Ag 1288, tyrphostin Ag 1295, geldanamycin, genistein and 7H-pyrollo[2,3-d]pyrimidine derivatives of formula I as generically and specifically described in PCT Publication No.: WO 03/013541 and U.S. Publication No.: 2008/0139587 :

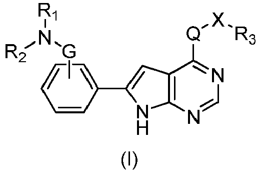

Publication No.: 2008/0139587 discloses the various substituents, e.g., R₁, R₂, etc.

SRC family tyrosine kinase inhibitors which include without limitation PP1 and PP2.

Syk tyrosine kinase inhibitors which include without limitation piceatannol.

Janus (JAK-2 and/or JAK-3) tyrosine kinase inhibitors which include without limitation tyrphostin AG 490 and 2-naphthyl vinyl ketone.

retinoids which include without limitation isotretinoin (Accutane®, Amnesteem®, Cistane®, Claravis®, Sotret®) and tretinoin (Aberel®, Aknoten®, Avita®, Renova®, Retin-A®, Retin-A MICRO®, Vesanoid®).

RNA polymerase II elongation inhibitors which include without limitation 5,6-dichloro-1-beta-D-ribofuranosylbenzimidazole.

serine/threonine kinase inhibitors which include without limitation 2-aminopurine.

sterol biosynthesis inhibitors which include without limitation squalene epoxidase and CYP2D6.

VEGF pathway inhibitors which include without limitation anti-VEGF antibodies, e.g., bevacizumab, and small molecules, e.g., sunitinib (Sutent®), sorafinib (Nexavar®), ZD6474 (also known as vandetanib) (Zactima™), SU6668, CP-547632, AV-951 (tivozanib) and AZD2171 (also known as cediranib) (Recentin™).

Examples of chemotherapeutic agents are also described in the scientific and patent literature, see, e.g., Bulinski (1997) J. Cell Sci. 110:3055-3064; Panda (1997) Proc. Natl. Acad. Sci. USA 94:10560-10564; Muhlradt (1997) Cancer Res. 57:3344-3346; Nicolaou (1997) Nature 387:268-272; Vasquez (1997) Mol. Biol. Cell. 8:973-985; Panda (1996) J. Biol. Chem 271:29807-29812.

In some embodiment, the CDP-topoisomerase inhibitor conjugate, particle or composition is administered instead of another topoisomerase inhibitor, e.g., instead of a topoisomerase inhibitor as a first line therapy or a second line therapy. For example, the CDP-topoisomerase inhibitor conjugate, particle or composition can be used instead of any of the following topoisomerase inhibitors: a topoisomerase I inhibitor, e.g., camptothecin, irinotecan, SN-38, topotecan, lamellarin D; a topoisomerase II inhibitor, e.g., etoposide, tenoposide, doxorubicin.

In some cases, a hormone and/or steriod can be administered in combination with a CDP-topoisomerase inhibitor conjugate, particle or composition. Examples of hormones and steroids include: 17a-ethinylestradiol (Estinyl®, Ethinoral®, Feminone®, Orestralyn®), diethylstilbestrol (Acnestrol®, Cyren A®, Deladumone®, Diastyl®, Domestrol®, Estrobene®, Estrobene®, Estrosyn®, Fonatol®, Makarol®, Milestrol®, Milestrol®, Neo-Oestronol I®, Oestrogenine®, Oestromenin®, Oestromon®, Palestrol®, Stilbestrol®, Stilbetin®, Stilboestroform®, Stilboestrol®, Synestrin®, Synthoestrin®, Vagestrol®), testosterone (Delatestryl®, Testoderm®, Testolin®, Testostroval®, Testostroval-PA®, Testro AQ®), prednisone (Delta-Dome®, Deltasone®, Liquid Pred®, Lisacort®, Meticorten®, Orasone®, Prednicen-M®, Sk-Prednisone®, Sterapred®), Fluoxymesterone (Android-F®, Halodrin®, Halotestin®, Ora-Testryl®, Ultandren®), dromostanolone propionate (Drolban®, Emdisterone®, Masterid®, Masteril®, Masteron®, Masterone®, Metholone®, Permastril®), testolactone (Teslac®), megestrolacetate (Magestin®, Maygace®, Megace®, Megeron®, Megestat®, Megestil®, Megestin®, Nia®, Niagestin®, Ovaban®, Ovarid®, Volidan®), methylprednisolone (Depo-Medrol®, Medlone 21®, Medrol®, Meprolone®, Metrocort®, Metypred®, Solu-Medrol®, Summicort®), methyl-testosterone (Android®, Testred®, Virilon®), prednisolone (Cortalone®, Delta-Cortef®, Hydeltra®, Hydeltrasol®, Meti-derm®, Prelone®), triamcinolone (Aristocort®), chlorotrianisene (Anisene®, Chlorotrisin®, Clorestrolo®, Clorotrisin®, Hormonisene®, Khlortrianizen®, Merbentul®, Metace®, Rianil®, Tace®, Tace-Fn®, Trianisestrol®), hydroxyprogesterone (Delalutin®, Gestiva™), aminoglutethimide (Cytadren®, Elipten®, Orimeten®), estramustine (Emcyt®), medroxyprogesteroneacetate (Provera®, Depo-Provera®), leuprolide (Lupron®, Viadur®), flutamide (Eulexin®), toremifene (Fareston®), and goserelin (Zoladex®).

In certain embodiments, the CDP-topoisomerase inhibitor conjugate, particle or composition is administered in combination with an anti-microbial (e.g., leptomycin B).

In another embodiment, the CDP-topoisomerase inhibitor conjugate, particle or composition is administered in combination with an agent or procedure to mitigate potential side effects from the agent compositions such as cystisis, hypersensitivity, diarrhea, nausea and vomiting.

Cystisis can be mitigated with an agent that increases urinary excretion and/or neutralizes one or more urinary metabolite. For example, cystisis can be mitigated or treated with MESNA.

Diarrhea may be treated with antidiarrheal agents including, but not limited to opioids (e.g., codeine (Codicept®, Coducept®), oxicodeine, percocet, paregoric, tincture of opium, diphenoxylate (Lomotil®), diflenoxin), and loperamide (Imodium A-D®), bismuth subsalicylate, lanreotide, vapreotide (Sanvar®, Sanvar IR®), motiln antagonists, COX2 inhibitors (e.g., celecoxib (Celebrex®), glutamine (NutreStore®), thalidomide (Synovir®, Thalomid®), traditional antidiarrhea remedies (e.g., kaolin, pectin, berberine and muscarinic agents), octreotide and DPP-IV inhibitors.

DPP-IV inhibitors employed in the present disclosure are generically and specifically disclosed in PCT Publication Nos.: WO 98/19998, DE 196 16 486 A1, WO 00/34241 and WO 95/15309.

Nausea and vomiting may be treated with antiemetic agents such as dexamethasone (Aeroseb-Dex®, Alba-Dex®, Decaderm®, Decadrol®, Decadron®, Decasone®, Decaspray®, Deenar®, Deronil®, Dex-4®, Dexace®, Dexameth®, Dezone®, Gammacorten®, Hexadrol®, Maxidex®, Sk-Dexamethasone®), metoclopramide (Reglan®), diphenylhydramine (Benadryl®, SK-Diphenhydramine®), lorazepam (Ativan®), ondansetron (Zofran®), prochlorperazine (Bayer A 173®, Buccastem®, Capazine®, Combid®, Compazine®, Compro®, Emelent®, Emetiral®, Eskatrol®, Kronocin®, Meterazin®, Meterazin Maleate®, Meterazine®, Nipodal®, Novamin®, Pasotomin®, Phenotil®, Stemetil®, Stemzine®, Tementil®, Temetid®, Vertigon®), thiethylperazine (Norzine®, Torecan®), and dronabinol (Marinol®).

In some embodiments, the CDP-topoisomerase inhibitor conjugate, particle or composition is administered in combination with an immunosuppressive agent. Immunosuppressive agents suitable for the combination include, but are not limited to natalizumab (Tysabri®), azathioprine (Imuran®), mitoxantrone (Novantrone®), mycophenolate mofetil (Cellcept®), cyclosporins (e.g., Cyclosporin A (Neoral®, Sandimmun®, Sandimmune®, SangCya®), cacineurin inhibitors (e.g., Tacrolimus (Prograf®, Protopic®), sirolimus (Rapamune®), everolimus (Afinitor®), cyclophosphamide (Clafen®, Cytoxan®, Neosar®), or methotrexate (Abitrexate®, Folex®, Methotrexate®, Mexate®)), fingolimod, mycophenolate mofetil (CellCept®), mycophenolic acid (Myfortic®), anti-CD3 antibody, anti-CD25 antibody (e.g., Basiliximab (Simulect®) or daclizumab (Zenapax®)), and anti-TNFa antibody (e.g., Infliximab (Remicade®) or adalimumab (Humira®)).

In some embodiments, a CDP-topoisomerase inhibitor conjugate, particle or composition is administered in combination with a CYP3A4 inhibitor (e.g., ketoconazole (Nizoral®, Xolegel®), itraconazole (Sporanox®), clarithromycin (Biaxin®), atazanavir (Reyataz®), nefazodone (Serzone®, Nefadar®), saquinavir (Invirase®), telithromycin (Ketek®), ritonavir (Norvir®), amprenavir (also known as Agenerase, a prodrug version is fosamprenavir (Lexiva®, Telzir®), indinavir (Crixivan®), nelfinavir (Viracept®), delavirdine (Rescriptor®) or voriconazole (Vfend®)).

When employing the methods or compositions, other agents used in the modulation of tumor growth or metastasis in a clinical setting, such as antiemetics, can also be administered as desired.

When formulating the pharmaceutical compositions featured in the disclosure the clinician may utilize preferred dosages as warranted by the condition of the subject being treated. For example, in one embodiment, a CDP-topoisomerase inhibitor conjugate, particle or composition may be administered at a dosing schedule described herein, e.g., once every one, two, three or four weeks.

Also, in general, a CDP-topoisomerase inhibitor conjugate, particle or composition and an additional chemotherapeutic agent(s) do not have to be administered in the same pharmaceutical composition, and may, because of different physical and chemical characteristics, have to be administered by different routes. For example, the CDP-topoisomerase inhibitor conjugate, particle or composition may be administered intravenously while the chemotherapeutic agent(s) may be administered orally. The determination of the mode of administration and the advisability of administration, where possible, in the same pharmaceutical composition, is well within the knowledge of the skilled clinician. The initial administration can be made according to established protocols known in the art, and then, based upon the observed effects, the dosage, modes of administration and times of administration can be modified by the skilled clinician.

In one embodiment, a CDP-topoisomerase inhibitor conjugate, particle or composition is administered once every three weeks and an additional therapeutic agent (or additional therapeutic agents) may also be administered every three weeks for as long as treatment is required. Examples of other chemotherapeutic agents which are administered one every three weeks include: an antimetabolite (e.g., floxuridine (FUDF®), pemetrexed (ALIMTA®), 5FU (Adrucil®, Efudex®, Fluoroplex®)); an anthracycline (e.g., daunorubicin (Cerubidine®, Rubidomycin®), epirubicin (Ellence®), idarubicin (Idamycin®), mitoxantrone (Novantrone®), valrubicin (Valstar®)); a vinca alkaloid (e.g., vinblastine (Velban®, Velsar®), vincristine (Vincasar®, Oncovin®), vindesine (Eldisine®) and vinorelbine (Navelbine®)); a taxane (e.g., paclitaxel, docetaxel, larotaxel and cabazitaxel); and a platinum-based agent (e.g., cisplatin (Platinol®), carboplatin (Paraplat®, Paraplatin®), oxaliplatin (Eloxatin®)).

In another embodiment, the CDP-topoisomerase inhibitor conjugate, particle or composition is administered once every two weeks in combination with one or more additional chemotherapeutic agent that is administered orally. For example, the CDP- topoisomerase inhibitor conjugate, particle or composition can be administered once every two weeks in combination with one or more of the following chemotherapeutic agents: capecitabine (Xeloda®), estramustine (Emcyt®), erlotinib (Tarceva®), rapamycin (Rapamune®), SDZ-RAD, CP-547632; AZD2171, sunitinib (Sutent®), sorafenib (Nexavar®) and everolimus (Afinitor®).

The actual dosage of the CDP-topoisomerase inhibitor conjugate, particle or composition and/or any additional chemotherapeutic agent employed may be varied depending upon the requirements of the subject and the severity of the condition being treated. Determination of the proper dosage for a particular situation is within the skill of the art. Generally, treatment is initiated with smaller dosages which are less than the optimum dose of the compound. Thereafter, the dosage is increased by small amounts until the optimum effect under the circumstances is reached.

In some embodiments, when a CDP- topoisomerase inhibitor conjugate, particle or composition is administered in combination with one or more additional chemotherapeutic agent, the additional chemotherapeutic agent (or agents) is administered at a standard dose. For example, a standard dosage for cisplatin is 75-120 mg/m² administered every three weeks; a standard dosage for carboplatin is within the range of 200-600 mg/m² or an AUC of 0.5-8 mg/ml x min; e.g., at an AUC of 4-6 mg/ml x min; a standard dosage for irinotecan is within 100-125 mg/m², once a week; a standard dosage for gemcitabine is within the range of 80-1500 mg/m² administered weekly; a standard dose for UFT is within a range of 300-400 mg/m² per day when combined with leucovorin administration; a standard dosage for leucovorin is 10-600 mg/m² administered weekly.

The disclosure also encompasses a method for the synergistic treatment of cancer wherein a CDP- topoisomerase inhibitor conjugate, particle or composition is administered in combination with an additional chemotherapeutic agent or agents.

The particular choice of conjugate, particle or composition and antiproliferative cytotoxic agent(s) or radiation will depend upon the diagnosis of the attending physicians and their judgment of the condition of the subject and the appropriate treatment protocol.

If the CDP- topoisomerase inhibitor conjugate, particle or composition and the chemotherapeutic agent(s) and/or radiation are not administered simultaneously or essentially simultaneously, then the initial order of administration of the CDP-topoisomerase inhibitor conjugate, particle or composition, and the chemotherapeutic agent(s) and/or radiation, may be varied. Thus, for example, the CDP- topoisomerase inhibitor conjugate, particle or composition may be administered first followed by the administration of the chemotherapeutic agent(s) and/or radiation; or the chemotherapeutic agent(s) and/or radiation may be administered first followed by the administration of the CDP-topoisomerase inhibitor conjugate, particle or composition. This alternate administration may be repeated during a single treatment protocol. The determination of the order of administration, and the number of repetitions of administration of each therapeutic agent during a treatment protocol, is well within the knowledge of the skilled physician after evaluation of the disease being treated and the condition of the subject.

Thus, in accordance with experience and knowledge, the practicing physician can modify each protocol for the administration of a component (CDP-topoisomerase inhibitor conjugate, particle or composition, anti-neoplastic agent(s), or radiation) of the treatment according to the individual subject's needs, as the treatment proceeds.

The attending clinician, in judging whether treatment is effective at the dosage administered, will consider the general well-being of the subject as well as more definite signs such as relief of disease-related symptoms, inhibition of tumor growth, actual shrinkage of the tumor, or inhibition of metastasis. Size of the tumor can be measured by standard methods such as radiological studies, e.g., CAT or MRI scan, and successive measurements can be used to judge whether or not growth of the tumor has been retarded or even reversed. Relief of disease-related symptoms such as pain, and improvement in overall condition can also be used to help judge effectiveness of treatment.

### Indications

The disclosed CDP-topoisomerase inhibitor conjugates, particles and compositions are useful in treating proliferative disorders, e.g., treating a tumor, e.g., a primary tumor, and/or metastases thereof, wherein the tumor is a primary tumor or a metastases thereof, e.g., a cancer described herein or a metastases of a cancer described herein.

The methods described herein can be used to treat a solid tumor, a soft tissue tumor or a liquid tumor. Exemplary solid tumors include malignancies (e.g., sarcomas and carcinomas (e.g., adenocarcinoma or squamous cell carcinoma)) of the various organ systems, such as those of brain, lung, breast, lymphoid, gastrointestinal (*e.g.,* colon), and genitourinary (*e.g.,* renal, urothelial, or testicular tumors) tracts, pharynx, prostate, and ovary. Exemplary adenocarcinomas include colorectal cancers, renal-cell carcinoma, liver cancer, non-small cell carcinoma of the lung, and cancer of the small intestine. The disclosed methods are also useful in evaluating or treating soft tissue tumors such as those of the tendons, muscles or fat, and liquid tumors.

The methods described herein can be used with any cancer, for example those described by the National Cancer Institute. The cancer can be a carcinoma, a sarcoma, a myeloma, a leukemia, a lymphoma or a mixed type. Exemplary cancers described by the National Cancer Institute include:
Digestive/gastrointestinal cancers such as anal cancer; bile duct cancer; extrahepatic bile duct cancer; appendix cancer; carcinoid tumor, gastrointestinal cancer; colon cancer; colorectal cancer including childhood colorectal cancer; esophageal cancer including childhood esophageal cancer; gallbladder cancer; gastric (stomach) cancer including childhood gastric (stomach) cancer; hepatocellular (liver) cancer including childhood hepatocellular (liver) cancer; pancreatic cancer including childhood pancreatic cancer; sarcoma, rhabdomyosarcoma; pancreatic cancer, islet cell; rectal cancer; and small intestine cancer;
Endocrine cancers such as islet cell carcinoma (endocrine pancreas); adrenocortical carcinoma including childhood adrenocortical carcinoma; gastrointestinal carcinoid tumor; parathyroid cancer; pheochromocytoma; pituitary tumor; thyroid cancer including childhood thyroid cancer; childhood multiple endocrine neoplasia syndrome; and childhood carcinoid tumor;
Eye cancers such as intraocular melanoma; and retinoblastoma;
Musculoskeletal cancers such as Ewing's family of tumors; osteosarcoma/malignant fibrous histiocytoma of the bone; rhabdomyosarcoma including childhood rhabdomyosarcoma; soft tissue sarcoma including childhood soft tissue sarcoma; clear cell sarcoma of tendon sheaths; and uterine sarcoma;
Breast cancer such as breast cancer and pregnancy including childhood and male breast cancer;
Neurologic cancers such as childhood brain stem glioma; brain tumor; childhood cerebellar astrocytoma; childhood cerebral astrocytoma/malignant glioma; childhood ependymoma; childhood medulloblastoma; childhood pineal and supratentorial primitive neuroectodermal tumors; childhood visual pathway and hypothalamic glioma; other childhood brain cancers; adrenocortical carcinoma; central nervous system lymphoma, primary; childhood cerebellar astrocytoma; neuroblastoma; craniopharyngioma; spinal cord tumors; central nervous system atypical teratoid/rhabdoid tumor; central nervous system embryonal tumors; and supratentorial primitive neuroectodermal tumors including childhood and pituitary tumor;
Genitourinary cancers such as bladder cancer including childhood bladder cancer; renal cell (kidney) cancer; ovarian cancer including childhood ovarian cancer; ovarian epithelial cancer; ovarian low malignant potential tumor; penile cancer; prostate cancer; renal cell cancer including childhood renal cell cancer; renal pelvis and ureter, transitional cell cancer; testicular cancer; urethral cancer; vaginal cancer; vulvar cancer; cervical cancer; Wilms tumor and other childhood kidney tumors; endometrial cancer; and gestational trophoblastic tumor;
Germ cell cancers such as childhood extracranial germ cell tumor; extragonadal germ cell tumor; ovarian germ cell tumor; and testicular cancer;
Head and neck cancers such as lip and oral cavity cancer; childhood oral cancer; hypopharyngeal cancer; laryngeal cancer including childhood laryngeal cancer; metastatic squamous neck cancer with occult primary; mouth cancer; nasal cavity and paranasal sinus cancer; nasopharyngeal cancer including childhood nasopharyngeal cancer; oropharyngeal cancer; parathyroid cancer; pharyngeal cancer; salivary gland cancer including childhood salivary gland cancer; throat cancer; and thyroid cancer;
Hematologic/blood cell cancers such as a leukemia (e.g., acute lymphoblastic leukemia in adults and children; acute myeloid leukemia, e.g., in adults and children; chronic lymphocytic leukemia; chronic myelogenous leukemia; and hairy cell leukemia); a lymphoma (e.g., AIDS-related lymphoma; cutaneous T-cell lymphoma; Hodgkin's lymphoma including Hodgkin's lymphoma in adults and children; Hodgkin's lymphoma during pregnancy; non-Hodgkin's lymphoma including non-Hodgkin's lymphoma in adults and children; non-Hodgkin's lymphoma during pregnancy; mycosis fungoides; Sézary syndrome; Waldenstrom's macroglobulinemia; and primary central nervous system lymphoma); and other hematologic cancers (e.g., chronic myeloproliferative disorders; multiple myeloma/plasma cell neoplasm; myelodysplastic syndromes; and myelodysplastic/myeloproliferative disorders);
Lung cancer such as non-small cell lung cancer; and small cell lung cancer;
Respiratory cancers such as malignant mesothelioma including malignant mesothelioma in adults and children; malignant thymoma; childhood thymoma; thymic carcinoma; bronchial adenomas/carcinoids including childhood bronchial adenomas/carcinoids; pleuropulmonary blastoma; non-small cell lung cancer; and small cell lung cancer;
Skin cancers such as Kaposi's sarcoma; Merkel cell carcinoma; melanoma; and childhood skin cancer;
AIDS-related malignancies;
Other childhood cancers, unusual cancers of childhood and cancers of unknown primary site;
and metastases of the aforementioned cancers can also be treated or prevented in accordance with the methods described herein.

The CDP-topoisomerase inhibitor conjugates, particles and compositions described herein are particularly suited to treat accelerated or metastatic cancers of gastric cancer, colorectal cancer, non-small cell lung cancer, ovarian cancer, and breast cancer.

In one embodiment, a method is provided for a combination treatment of a cancer, such as by treatment with a CDP-topoisomerase inhibitor conjugate, particle or composition and a second therapeutic agent. Various combinations are described herein. The combination can reduce the development of tumors, reduces tumor burden, or produce tumor regression in a mammalian host.

Unless otherwise defined, all technical and scientific terms used herein have the same meaning as commonly understood by one of ordinary skill in the art to which this disclosure belongs. In case of conflict with the publications, patent applications, patents, and other references mentioned herein, the present specification, including definitions, will control. In addition, the materials, methods, and examples are illustrative only and not intended to be limiting.

### EXAMPLES

### Example 1: Human Phase 1 Study of CRLX101

The below example describes the first human phase 1 study of CRLX101. The study was composed of two parts. Part 1 of the Phase 1 study had a primary objective to determine the safety profile, toxicity, and pharmacokinetics of CRLX101 when administered weekly for 3 consecutive weeks of every 4-week cycle (the initial dosing regimen, sometimes referred to herein as "weekly x3"). In part 2, after the first twelve patients were enrolled, an every other week schedule was initiated for the remainder of the Phase 1 program (sometimes referred to herein as "biweekly").

### PATIENTS AND METHODS

**Eligibility Criteria.** Patients with histologically or cytologically confirmed metastatic or unresectable solid tumors refractory to standard therapy, or for which no standard curative or palliative therapy existed, were eligible for this trial. Prior treatment with topoisomerase inhibitors was allowed. Main eligibility criteria included male or female patients, at least 18 years of age, with advanced, histologically confirmed solid tumor refractory to standard treatment, or for which no standard therapy existed, measurable or evaluable disease, Eastern Cooperative Oncology Group (ECOG) Performance Status ≤ 2, acceptable organ and bone marrow function, no evidence of clinically significant conduction abnormalities or ischemia, and ejection fraction ≥ 45%. Prior chemotherapy, radiation therapy, or investigational
therapy had to be completed within a prescribed interval before enrollment, could not have included high dose chemotherapy with autologous stem cell rescue bone marrow transplantation, and patients could not have been refractory to prior treatment with a topoisomerase I inhibitor.

This trial was conducted at City of Hope (COH) (Duarte, CA) following approval of the Clinical Protocol Review and Monitoring Committee (CPRMC) and the Institutional Review Board (IRB). After the initial 18 patients were enrolled, an additional site was added at TGen (Scottsdale, AZ) following the approval of WIRB (Western Institutional Review Board).

**Study design and drug administration.** This was an open-labeled, single-arm dose-escalation phase 1 study of CRLX101. In part 1, CRLX101 was administered at 6, 12, or 18 mg/m² on a weekly schedule three weeks per month. In part 2, CRLX101 was administered at 12, 15 or 18 mg/m² on an every other week schedule.

In part 1, CRLX101 was administered as an intravenous infusion over 90 minutes on days 1, 8, and 15 followed by a 7 day rest period (28 day cycle). In part 2, CRLX101 was administered as an intravenous infusion over 90 minutes on days 1 and 15, every 28 days. CRLX101 was administered as an intravenous infusion over 60 minutes on days 1 and 15, every 28 days for the last six patients in part 2 of the Phase 1 study.

The starting dose in part 1 was 6 mg/m². Dose escalation was done using an accelerated Simon design (Simon et al., "Accelerated Titration Designs for Phase I Clinical Trials in Oncology" J Natl Cancer Inst. 89:1138-47, 1997) with a modified Fibonacci dose escalation scheme. In brief, patients were accrued in cohorts of one at escalating doses until the occurrence of a dose limiting toxicity (DLT) in the first cycle. Once a DLT occurred, additional patients were accrued in that dosage group to provide for either 3 or 6 patients as provided in a standard dose escalation with the intent of determining the maximum tolerated dose. Concurrent accrual was allowed within the same dose level. No intrapatient dose escalation was permitted.

**Toxicity Assessment.** Toxicity was graded according to the National Cancer Institute (NCI) common toxicity criteria (CTCAE) version 3.0. The DLT in a given patient was defined as any treatment-related grade III non-hematologic toxicity, any grade IV hematologic toxicity, or persisting toxicities of any grade requiring delay of scheduled treatment by more than 2 weeks. DLT was based on the first course of treatment.

**Rules for dose escalation.** One patient was treated at each dose level. If a DLT attributable to the study drug(s) was experienced, up to 5 additional patients were subsequently treated at that dose level. If no additional DLTs were observed at the expanded dose level (*i.e.,* at most 1/6 with an attributable DLT), the dose was escalated. Escalation was terminated when two of six patients experienced any DLT attributable to the study drug at a given dose level. The maximally tolerated dose (MTD) was defined as the dose level preceding the dose at which ≥2/6 patients experienced a first-course DLT. Treatment was continued in an individual patient for a total of 6 cycles at the same dose level if no DLT was observed and if clinical benefit was observed. Therapy was discontinued in any patient if excessive toxicity was experienced. No intra-patient dose escalation was permitted. Patients who completed 6 cycles with clinical benefit had the option of continuing treatment at the same dose level every other week.

**Safety and efficacy evaluations.** Patients were seen, examined and a complete blood count with differential and serum chemistry was obtained before each dose. Radiographic assessments of tumor response (as evaluated by the Response Evaluation Criteria in Solid Tumor criteria RECIST) were performed every two cycles (Therasse et al., "New Guidelines to Evaluate the Response to Treatment in Solid Tumors. European Organization for Research and Treatment of Cancer, National Cancer Institute of the United States, National Cancer Institute of Canada" J. Natl. Cancer Inst. 92:205-16, 2000).

**Treatment modification.** Patients who experienced a DLT during the initial cycle, or a severe or life threatening non-hematologic toxicity at any time during the study, were dose reduced at the next dose administration when their DLT returned to Grade 1. If a non-hematologic DLT occurred in the interval between dose administrations within a cycle, and the toxicity was not life threatening and resolved rapidly, the next dose administration within the cycle was to be at the next lower dose level. If a hematologic DLT occurred in the interval between dose administrations within a cycle, the next dose administration was to be at the next lower dose level, even if the blood cell counts recovered by the date of the next scheduled administration. Dosing was to be held for any non-hematologic toxicity grade ≥ 2, except for grade 2 fatigue and anorexia. After the first cycle, dose modifications were based on interval toxicity, and platelet and granulocyte counts were obtained on the day of treatment.

**Hematologic toxicity:** On day 1 of a cycle, administration of CRLX101 required an absolute granulocyte count (AGC) of ≥1500 and platelet count of ≥100,000. On day 8, if the AGC was ≥1000 and platelet count ≥ 75,000, then CRLX101 was given at full dose and in the same manner for day 15. If the AGC was <1000, the treating physicians could delay the dose of CRLX101 on days 8 and 15. If unacceptable hematologic toxicity persisted >7 days, the CRLX101 dose was reduced to the next lower dose level. Similar standards were used in the biweekly part.

**Non-hematologic toxicity:** Any grade 2 toxicity that was intolerable to the patients, or any grade 3 or greater non-hematologic toxicity that was attributed to CRLX101, had to return to grade 1 before a new cycle of treatment was started. Any treatment delay >2 weeks because of toxicity due to CRLX101 would result in the patient being removed from the study. If a patient experienced any grade 3 genitourinary (GU) toxicity, the dose of CRLX101 was reduced to the next lower dose level, as CPT has been reported to cause hematuria, cystitis or other GU toxicities, which can be irreversible. If the patient experienced grade 3 non-hematologic toxicities that did not recover to grade 1 before the next treatment, treatment was held until recovery and then reduced by one dose level. If the patient experienced grade 4 non-hematologic toxicities that did not recover to grade 1 before the next treatment, then the dose was reduced by 50% in subsequent cycles. If there were conflicting dose attenuations by hematologic and non-hematologic toxicity, the greater dose reduction was applied. All dose modifications were permanent. Criteria for removal from treatment included disease progression, clinical progression, excessive toxicity, or patient withdrawal.

**Plasma/urine sampling and analysis.** Whole blood samples (5 mL) were collected in heparinized tubes at the following times during cycle one: pre-dose, during dosing at 30 minutes, 60 minutes and just prior to end of administration (90 minutes), and then at 0.25, 0.5, 1, 2, 3, 4, 6, 8, 24, (48-72 hr optional), 168 hours (1 week), and 336 hours (only for patients on biweekly schedule) following the first dose. A spot trough PK blood sample was collected prior to dosing on Day 1 and Day 15 of every cycle. Plasma was separated by centrifugation at 1,300 x g for 10 minutes at 4°C. Plasma was immediately frozen at -20°C, and stored frozen until analysis. For determination of urinary excretion of polymer conjugated and unbound CPT, total urine collections were performed following dose 1 of the first cycle (0-24 and 24-48 hours post administration. A spot urine sample (15 mL) was collected at 8, 24, 48, 168 (1 wk), and 336 hours (pre-second dose) for more accurate determination of total to free CPT ratio. Once each urine collection was complete, the actual collection times and total volume of urine was recorded and a 5-10 mL aliquot of each was frozen for subsequent drug level analysis.

Samples were extracted and analyzed by LC-MS/MS (liquid chromatography-mass spectrometry/mass spectrometry) using a validated method. In order to determine total CPT in samples, a 20µL aliquot was incubated with 5µL 0.2N NaOH for 1 hour to release all CPT from the polymer conjugate. The solution was acidified with 7 µL 44% formic acid. Proteins were precipitated by addition of 160 µL cold (<-20°C) methanol containing the internal standard 9-nitro camptothecin (9-NC) at a concentration of 8 ng/mL, incubation for 30 minutes on ice, and centrifugation at 14,000 rpm. The resulting supernatant was diluted with an equal volume of 0.5 mM ammonium acetate buffer, pH 3.5, and analyzed by LC-MS/MS.

In order to determine unconjugated CPT in samples, a 20µL aliquot was acidified with 3 µL 44% formic acid. Proteins were precipitated by addition of 160 µL cold (<-20°C) methanol containing 9-NC at a concentration of 8 ng/mL, incubation for 30 minutes on ice, and centrifugation at 14,000 rpm. The resulting supernatant was diluted with an equal volume of 0.5 mM ammonium acetate buffer, pH 3.5 and analyzed by LC-MS/MS.

LC-MS/MS was performed on an Agilent 1100 series HPLC system (Palo Alto, CA) coupled to a Micromass Quattro Ultima Triple Quadrupole Mass Spectrometer (Micromass, Inc., Beverly, MA). HPLC separation was achieved using a Synergy Hydro-RP 4µm 75 x 2 mm analytical column (Phenomenex, Torrance, CA) preceded by a C₁₈ guard column (Phenomenex, Torrance, CA). The isocratic mobile phase consisted of 34% acetonitrile, 66% 0.5 mM ammonium acetate, pH 3.5 at a flow rate of 0.2 mL/min at room temperature. MassLynx version 3.5 software was used for data acquisition and processing.

**Immunohistochemical analysis of topoisomerase I expression.** Ascites fluid from one patient with ovarian cancer was obtained pre-treatment and on day 2 post treatment with CRLX101 at 6 mg/m². Cells were pelleted by centrifugation and the pellet frozen. The pellet was formalin fixed and paraffin embedded. Immunohistochemical staining was performed on 5 µm thick sections. Sections were deparaffinized in xylene followed by 100% ethanol. Samples were then quenched in 3% hydrogen peroxide and pretreated to promote antigen retrieval by steam in DIVA/citrate buffer (pH 6.0, Biocare Medical, Concord, CA) solution. After antigen retrieval, slides were incubated in Protein Block for 20 minutes. Slides were then incubated with primary antibody overnight at 4°C. Topoisomerase I antibody was rabbit polyclonal from Abcam (Cambridge, MA).

The next day, slides were washed in Dako Buffer (DB) and incubated with the appropriate secondary antibodies for 30 minutes at room temperature. After washes in DB, slides were incubated with the chromogen diaminobenzidine tetrahydrochloride (DAB), counterstained with hematoxylin, and mounted.

**Topoisomerase I enzymatic activity assay.** Lysates containing total cellular protein were made from the plain frozen ascites cells according to the method of Minagawa et al. ("Enhanced Topoisomerase I Activity and Increased Topoisomerase II Alpha Content in Cisplatin-Resistant Cancer Cell Lines" Jpn J Cancer Res. 88:1218-23, 1997), a procedure used specifically for frozen cells.

The catalytic activity of topoisomerase I was determined by measuring the relaxation of supercoiled plasmid substrate DNA using the Topo I assay kit (TopoGEN, Port Orange, FL) following the manufacturer's instructions. Briefly, reaction mixtures consisted of supercoiled plasmid substrate DNA (0.5 µg), whole cell lysate (0.25 µg or 0.5 µg, as indicated in data) and the assay buffer (final concentrations: 10 mM Tris HCl [pH 7.9], 1 mM EDTA, 150 mM NaCl, 0.1% BSA, 0.1 mM spermidine, and 5% glycerol). Reaction mixtures were incubated at 37°C for 30 minutes, and terminated by adding 5 µL stop buffer/gel loading buffer. Samples were loaded onto a 1% agarose gel in 1x TAE buffer (40 mM Tris base [pH 8.3, adjusted in the 50x stock buffer using glacial acetic acid], 2.5 mM NaOAc, and 0.05 mM EDTA) and electrophoresed at 4-5 volts per centimeter for 3-4 hours. Supercoiled plasmid DNA (0.5 µg) and relaxed DNA (0.5 µg) provided by the Topo I assay kit were used as the control markers. The gel was stained with 0.2 µg/mL ethidium bromide for 20 minutes at room temperature, destained in water for 20 minutes and photographed under ultraviolet (UV) light.

**Pharmacokinetic and statistical analysis.** Plasma concentration versus time data were analyzed using the ADAPT II (Biomedical Simulations Resource, Los Angeles) non-compartmental model. Results are summarized using descriptive statistics.

### RESULTS

**Patient Enrollment.** Twenty-four patients were enrolled from June 2006 to April 2010. Patient characteristics are summarized in Table 1. The patients had a variety of solid tumors, and lung cancer was the most common tumor type. All twenty-four patients were considered evaluable for toxicity having received two completed cycles of therapy.

**Table 1. Patient demographics**

| | | | | |
|---|---|---|---|---|
| Total number of patients | | | | 24 |
| Number of evaluable patients | | | | 24 (100%) |
| Median age (range) | | | | 61 (46-79) |
| Gender -Male/Female | | | | 13/11 |
| Ethnicity | - Caucasian | | | 16 (66%) |
| | - Asian | | | 6 (25%) |
| | - Others | | | 2 (9%) |
| Performance status (ECOG) | | | - 0 | 11 (46%) |
| | | | - 1 | 12 (50%) |
| | | | - 2 | 1 (4%) |
| Tumor types | | - Lung | | 6 |
| | | - Breast | | 3 |
| | | - Urinary/Renal | | 3 |
| | | - Liver | | 2 |
| | | - Pancreatic | | 5 |
| | | - Ovarian | | 1 |
| | | - Thyroid | | 1 |
| | | - Head and Neck | | 1 |
| | | - GI | | 1 |
| | | - Endometrial | | 1 |
| Prior chemotherapy regimens | | | 0 | 1 |
| | | | 1 | 5 |
| | | | 2 | 2 |
| | | | 3 | 4 |
| greater than or equal to 4 | | | | 12 |

**Tumor response.** Patient dosing and data are shown in Table 2. At the first dose schedule (part 1), one patient with metastatic pancreatic cancer that had spread to lungs and liver experienced stable disease and received compassionate treatment for a total of 22 cycles before disease progression. The results of a CT scan performed on this patient are shown in FIGs. 1A and 1B. Four patients experienced prolonged (>6 months) SD (stable disease), one patient with renal cancer with lung metastases, two patients with non-small cell lung cancer patient, and one patient with adenocarcinoma of the pancreas. (Table 3). All patients had been heavily pretreated for metastatic disease (see Table 3). Although the protocol allowed for prior treatment with CPT, none of these patients had received prior CPT treatment.

**Table 2. Analysis of population and patient disposition.**

| | Number of patients. | Number of patients completed cycle 1 | Number of patients completed cycle 6 | No. Cycles Median (range) | Pts. w. DR AE leading to discontinu -ation | Discontinuation AE description | Best Responses During therapy (all eligible patients) | Number of Pts requiring dose modification for toxicity |
|---|---|---|---|---|---|---|---|---|
| | | | | | | | | |
| 6 mg/m² weekly x3 | 6 | 6 | 1 (17%) | 2.0 (1-6) | 0 | - | 1 SD (17%) | 0 |
| 12 mg/m² weekly x3 | 3 | 3 | 1 (33%) | 2.0 (2-6) | 2 (67%) | Anemia, cystitis | 2 SD (67%) | 1 (33%) |
| 18 mg/m² weekly x3 | 3 | 3 | 1 (33%) | 2.0 (1-6) | 2 (67%) | Neutropenia, thrombocytopenia, upper resp. tract infection | 2 SD (67%) | 2 (67%) |
| 12 mg/m² biweekly | 3 | 3 | 0 | 3.0 (2-4) | 0 | - | - | 0 |
| 15 mg/ m² biweekly | 6 | 6 | 1 (16%) | 3.0 (2-6) | 1 | Cystitis | 4 SD (67%) | 1 (17%) |
| 18 mg/ m² biweekly | 3 | 3 | 0 | 2 (1-5) | 1 | Thrombocyto penia | 1 SD (33%) | 2 (67%) |

| | | | | | | | | |
|---|---|---|---|---|---|---|---|---|
| Note: One patient still active - presently in cycle 5 | | | | | | | | |

Four patients who completed 6 cycles of therapy continued to receive CRLX101 every other week on a compassionate use basis. Abbreviations: AE = adverse event, SD = stable disease, PD = progressive disease, DR = drug related, Pt = patient

**Table 3. CRLX101 showed activity in this heavily pre-treated patient population with 4 long-term progression free survivors, two of which showed minor responses.**

| **Tumor Type** | **Dose / Schedule** | **Activity** | **Prior Agents** | **Duration of Prior Response** |
|---|---|---|---|---|
| Pancreatic | 6 mg/m² weekly x3 | 22.8 mo PFS, CT scan ↓ 16% | 5-FU, cisplatin, gemcitabine, radiation | 10 mo |
| Non-small cell lung | 18 mg/m² weekly x3 | 9.7 mo PFS, CT scan ↓ 6% | Getfitinib, carboplatin, paclitaxel, pemetrexed, vinorelbine, gemictabine | 6 mo |
| Renal | 12 mg/m² / weekly x3 | 7.7 mo PFS, SD | Sunitinib | 18 mo |
| Non-small cell lung | 15 mg/m² biweekly | 12.0 mo PFS, SD | Carboplatin, paclitaxel, experimental Rx, pemetrexed, gemcitabine, vinorelbine, erlotinib | 7 mo |

| | | | | |
|---|---|---|---|---|
| Abbreviations: PFS = progression free survival, SD = stable disease | | | | |

**Toxicity Evaluation.** All patients in the first twelve enrolled (weekly x3) experienced grade 3 or 4 toxicities at doses above 6 mg/m². The weekly x3 schedule was stopped after these first twelve patients due to the severe toxicity observed in all patients treated at doses higher than 6 mg/m². One patient tolerated 9 mg/m² on a weekly x3 schedule for five cycles (three doses per cycle).

No patients in the second twelve patients enrolled at the bi-weekly schedule experienced severe toxicity until the MTD was reached. Grade 2 DLTs included the following.

One bladder cancer patient experienced grade 2 anemia in cycle 2, day 8. This grade 2 hematologic toxicity resolved in two weeks.

One lung cancer patient experienced elevated amylase in laboratory tests but no clinical manifestation of pancreatitis.

One head and neck cancer patient (nasopharyngeal with heavy previous chemotherapy) experienced delayed onset grade 2 transaminitis in cycle 2, day 1.

One lung cancer patient experienced grade 2 hematologic toxicity which resolved in two weeks.

At the highest dose tested, two patients experienced DLT hematological toxicities. The first patient, a breast cancer patient, who had metastatic disease to lung and other organs and had previously received multiple chemotherapy regimens, developed grade 3 anemia, neutropenia, and thrombocytopenia. This patient required platelet transfusions. The second patient had metastatic lung cancer, and had previously received carboplatin and paclitaxel with a brief response. This patient developed grade 3 neutropenia, requiring dose reduction and granulocyte colony-stimulating factor (G-CSF) support. This hematological toxicity in 2 of 3 patients established 18 mg/m² as the DLT level.

One pancreatic cancer patient experienced grade 2 anemia on cycle 5, day 1.

One patient with hepatocellular cancer passed away one week after cycle 3, day 1 due to progressive disease.

One patient with lung cancer who had received multiple prior regimens, developed grade 2 neutropenia on cycle 3, day 15. This patient required a dose reduction and G-CSF support.

Based on the long terminal half-life of polymer conjugated, and especially unconjugated, CPT (see FIGs. 2A and 2B), the protocol was amended in part 2 to evaluate an every other week schedule. A total of 12 patients were treated on this schedule, three at 12mg/m² and six at 15 mg/m² and three at 18mg/m². Except for one occurrence of grade 3 neutropenia, no grade 3/4 hematologic events were recorded at dose level 12mg/m² and 15mg/m². At dose level 18 mg/m² one grade 4 leukopenia, two grade 4 neutropenia, two grade 4 thrombocytopenia and one grade 3 anemia were reported under hematological toxicities. This determined the DLT of bone marrow suppression at 18 mg/m² on this schedule. The only other notable non hematologic event was a grade 3 hypersensitivity reaction. (Table 4).

Table 4 shows a summary of all grade 3/4 treatment related toxicities for all evaluable patients. Four patients on the weekly schedule developed delayed onset (after cycle 4) mild hematuria and mild dysuria that may have been related to treatment as previously reported for CPT (Muggia et al., "Phase I Clinical Trial of Weekly and Daily Treatment with Camptothecin (NSC-100880): Correlation with Preclinical Studies" Cancer Chemother Rep 56:515-521, 1972). However, upon evaluation by a urologist, cystitis could not be confirmed. Thus, at 18 mg/m² biweekly two patients were reported who developed DLT, and the MTD for the biweekly schedule was established as 15 mg/m².

**Table 4: Treatment related grade 3/4 hematologic and non-hematologic adverse events by dose cohort observed during all courses of therapy.**

| **DOSE** | **Grade 3** | **Grade 4** | |
|---|---|---|---|
| **6mg/m²** | | | |
| Elevated CPK | 1 | 0 | |
| Fatigue | 1 | | |
| lymphopenia | 1 | | |
| | | | |
| **12mg/m²** | | | |
| | | | |
| hyponatremia | 2 | 0 | |
| Dysuria/cystitis | 2 | 0 | |
| | | | |
| **18mg/m²** | | | |
| | | | |
| thrombocytope nia | 2 | 3 | |
| leukopenia | 4 | 1 | |
| neutropenia | 4 | 3 | |
| anemia | 3 | 0 | |
| fatigue | 3 | 0 | |
| **dehydration** | **1** | **0** | |
| rash | 1 | 0 | |
| SOB | 1 | 0 | |
| Hypersensitivity reaction | 1 | 0 | |
| | | | |
| | | | |
| | | | |
| | | | |

**Pharmacokinetic and toxicokinetic analysis.** Samples for pharmacokinetic analysis were collected from all patients during cycle 1. Results from this analysis are summarized in FIGs. 2A, 2B and Table 5. The mean elimination half-lives were 31.8 ± 5.7 hr and 43.8 ± 9.7 hr for conjugated and unconjugated CPT, respectively. Volume of distribution of the polymer conjugate was 4.2 ± 1.1 liter and was independent of dose. The low volume of distribution suggests that CRLX101 is initially retained in plasma and avoids rapid first pass clearance. Cₘₐₓ and AUC_{0-inf} were linear across doses and similar when normalized for dose/m2. FIGs. 2A and 2B summarize pharmacokinetic parameters measured for polymer conjugated and unconjugated CPT for both schedules. Unconjugated CPT was slowly released from CRLX101 as shown by increasing plasma concentrations that peaked at 20.2 ± 9.7 hrs. Plasma concentrations of unconjugated CPT were significantly below the plasma concentrations of conjugated CPT at all timepoints, with unconjugated CPT accounting for an average of 8.7 ± 2.7 % of total CPT plasma exposure. FIG. 2A shows average plasma time-concentration curves for the biweekly 12 mg/m cohort Systemic plasma clearance of conjugated CPT was 0.12 ± 0.2 L/h, significantly below the kidney and liver blood flows in humans, and was also independent of dose. FIG. 2B shows average urinary excretion of polymer conjugated and unconjugated CPT in the first 48 hours following CRLX101 administration. Urinary loss of total CPT was variable with an average of 22.8 ± 12.1% of dose excreted during the first 48 hours, of which 78 ± 9% was in the conjugated form. Interestingly, urinary excretion of the polymer conjugate was primarily in the first 24 hours (16.4% ± 10.0% of dose) compared to the second 24 hours (1.5 ± 1.3% of dose) post administration. Urinary excretion of unconjugated CPT remained approximately constant over both 24 hour periods (2.0± 1.1% vs. 2.9± 1.4% of dose). Toxicokinetic analysis of the two schedules showed that the predicted monthly exposure for conjugated and unconjugated CPT was similar for 6 mg/m² weekly vs. 12 mg/m² bi-weekly and 12 mg/m² weekly vs. 15 mg/m² bi-weekly; however, fewer patients experienced cycle one drug related adverse events with the bi-weekly regimen.

**Table 5. Pharmacokinetic parameters and toxicokinetic summary. Values are in geometric means ± standard deviation.**

| **Dose** / **Schedule** | **N** | **Cₘₐₓ mg/L** | | **T_{1/2,β} hr** | | **AUC mg/L/hr** | | **Per cycle AUC mg/L/hr** | | **#Pt with DR AE ≥ Grade. 3** |
|---|---|---|---|---|---|---|---|---|---|---|
| | | Bound | Free | Bound | Free | Bound | Free | Bound | Free | |
| 6 mg/m² weekly x3 | 6 | 3.55 ± 0.46 | 0.10 ± 0.06 | 31.1 ± 5.2 | 43.7 ± 14.6 | 114.4 ± 21.5 | 11.9 ± 7.0 | 343.2 ± 64.5 | 35.7 ± 21.1 | 2 (33%) |
| 12 mg/m² weekly x3 | 3 | 5.55 ± 1.33 | 0.18 ± 0.01 | 33.8 ± 5.9 | 61.5 ± 37.6 | 188.5 ± 56.7 | 18.3 ± 3.8 | 565.6 ± 170.1 | 54.9 ± 11.4 | 1 (33%) |
| 18 mg/m² weekly x3 | 3 | 7.90 ± 1.18 | 0.24 ± 0.06 | 37.7 ± 6.2 | 38.3 ± 4.9 | 248.3 ± 29.2 | 23.7 ± 5.9 | 744.8 ± 87.5 | 71.1 ± 17.8 | 3 (100%) |
| 12 mg/m² biweekly | 3 | 5.56 ± 0.37 | 0.22 ± 0.09 | 27.8 ± 4.3 | 32.5 ± 4.9 | 182.0 ± 21.6 | 14.6 ± 2.5 | 364.0 ± 43.1 | 29.0 ± 5.0 | 0 |
| 15 mg/m² biweekly | 3 | 8.63 ± 0.76 | 0.27 ± 0.13 | 30.4 ± 1.2 | 48.3 ± 6.5 | 276.7 ± 14.2 | 23.5 ± 9.0 | 553.8 ± 28.3 | 47.0 ± 18.0 | 1 (33%) |

**Correlative Studies.** Ascites cells were collected from one patient with ovarian cancer pre-treatment and on Days 2 and 25 post-treatment. Pellets of these cells were frozen for later analysis. Levels of polymer conjugated and unconjugated CPT were also determined in the ascites fluid pre-treatment and on day 2 post-treatment. On day 2, the concentrations detected were 46.6 µg/L for conjugated CPT and 19.6 µg/L for fully active, unconjugated CPT. For each amount of lysate used, there was less topoisomerase I unwinding activity (*i.e.,* more remaining supercoiled DNA) in the Day 2 samples than in the pretreatment or Day 25 samples. This suggests an inhibitory effect of CRLX101 on these cells at this early time point after administration. The ascites cells were also used for immunohistochemistry (IHC) to assess the levels of topoisomerase I. Basic agreement was observed between the topoisomerase I activity assay and topoisomerase I IHC. As seen in FIGs. 3A and 3B, there was a reduction of approximately 30% in staining in the nucleus of ovarian cancer cells isolated from the patient's ascites fluid 2 days after treatment (FIG. 3B), compared to a similar sample taken before drug administration (FIG. 3A). The decrease in topoisomerase I levels directly seen by IHC in these cells at 48 hours explains why much of the supercoiled DNA remains present in lanes of FIG. 3C with reactions from Day 2 lysates; *i.e.,* there is much less enzyme available to act on these substrate molecules.

**Summary.** In the phase I trial reported above, two dosing schedules, weekly x3 and every other week (biweekly), were investigated. In the weekly x3 schedule the maximum tolerated dose was approximately 9 mg/m². Hematologic toxicity and cystitis were the DLTs in this schedule. Non-hematologic grade 3/4 adverse events included fatigue in 3 patients (25%), delayed onset hematuria/dysuria in 2 patients (17%), elevated CPK (creatine phosphokinase) in 1 patient (8%), and dehydration in 1 patient (8%), all of which were reversible. Cumulative bladder toxicity in some patients on this schedule was primarily observed post cycle one and may have been related to the long terminal half-life of unconjugated CPT, which is primarily cleared through the kidneys, leading to cumulative bladder irritation. Based on this observation, it was decided to investigate a biweekly schedule as a strategy to reduce cumulative toxicity while maintaining dose delivery.

The biweekly schedule allowed for similar per cycle plasma exposure to be achieved in patients but with a significantly reduced incidence of adverse events. The only observed grade 3/4 adverse event was grade 3 neutropenia in one patient that was reversible. One dose reduction was required on this schedule. On this schedule, CRLX101 was well tolerated without the toxicities normally associated with camptothecin analogs, such as severe diarrhea and hemorrhagic cystitis. The MTD on this schedule was determined to be 15 mg/m².

Pharmacokinetic analysis of CRLX101 was performed after the first dose for all patients. Consistent with preclinical data, the pharmacokinetics of CRLX101 were characterized by a low volume of distribution and limited systemic clearance. Preclinical studies showed accumulation of CRLX101 in tumors and tissues of the reticuloendothelial system such as liver and spleen. Increased release of active CPT from the conjugate was also observed in these tissues. This study also confirmed that release kinetics of CPT were such that plasma levels of unconjugated CPT remained significantly below levels of conjugate at all times. After one week, approximately 10% of the maximum recorded concentration of unconjugated CPT was still detected in plasma, possibly leading to the cumulative toxicity observed on the weekly x3 schedule. On the biweekly schedule however, unconjugated CPT levels dropped below the limit of quantitation before the second dose, which may explain the lack of urinary side effects on this schedule.

In general, CRLX101 was well tolerated and myelosuppression was the DLT. Ten out of twenty-four patients demonstrated stable disease on CT scan evaluation at the end of cycle 2. One pancreatic cancer patient remained stable for 22.8 months. Serum and urine PK data from all the treated patients indicated that the mean elimination half-lives for conjugated and unconjugated CPT were 31.8 hr and 43.8 hr, respectively. Cₘₐₓ and AUC_{0-inf} were linear across doses and similar when normalized for dose/m². The biweekly schedule allowed for similar per cycle plasma exposure to be achieved in patients, but with a significantly reduced incidence of adverse events.

### Example 2: Synthesis of 6^{A},6^{D}-Bis-(2-amino-2-carboxylethylthio)-6^{A,}6^{D}-dideoxy-β-cyclodextrin, 4 (CD-BisCys)

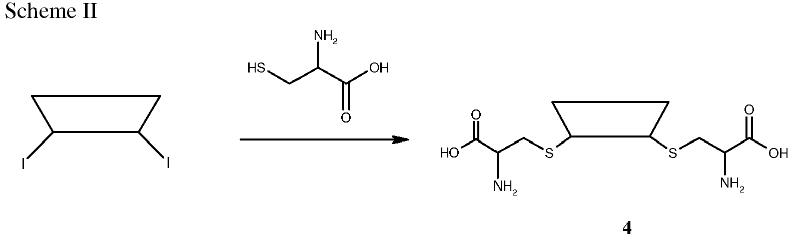

167 mL of 0.1 M sodium carbonate buffer were degassed for 45 minutes in a 500 mL 2-neck round bottom flask equipped with a magnetic stir bar, a condenser and septum. To this solution were added 1.96 g (16.2 mmol) of L-cysteine and 10.0 g (73.8 mmol) of diiodo, deoxy-β-cyclodextrin **2.** The resulting suspension was heated at a reflux temperature for 4.5 h until the solution turned clear (colorless). The solution was then cooled to room temperature and acidified to pH 3 using IN HCl. The product was precipitated by slow addition of acetone (3 times weight ratio of the solution). This afforded 9.0 g crude material containing CD-biscysteine (90.0%), unreacted cyclodextrin, CD-mono-cysteine and cystine. The resulting solid was subjected to anionic exchange column chromatography (SuperQ650M, Tosoh Bioscience) using a gradient elution of 0-0.4M ammonium bicarbonate. All fractions were analyzed by HPLC. The desired fractions were combined and the solvent was reduced to 100 mL under vacuum. The final product was either precipitated by adding acetone or by adding methanol (3 times weight ratio of the solution). **4** was obtained in 60-90% yield. ¹H NMR (D₂O) δ 5.08 (m, 7H, CD-2-CH), 3.79-3.94 (m, 30H, CD-3,4-CH, CD-CH₂, Cys-CH), 3.49-3.62 (m, 14H, CD-5, 6-CH), 2.92-3.30(m, 4H, Cys-CH₂). ¹³C NMR (D₂O) δ 172.3, 101.9, 83.9, 81.6, 81.5, 73.3, 72.2, 72.0, 60.7, 54.0, 34.0, 30.6. ESI/MS (*m*/*z*): 1342 [M]⁺, 1364 [M + Na]⁺. Purity of **4** was confirmed by HPLC.

### Example 3: Synthesis of Gly-CPT (Structure 11) (Greenwald et al., Bioorg. Med. Chem., 1998, 6, 551-562

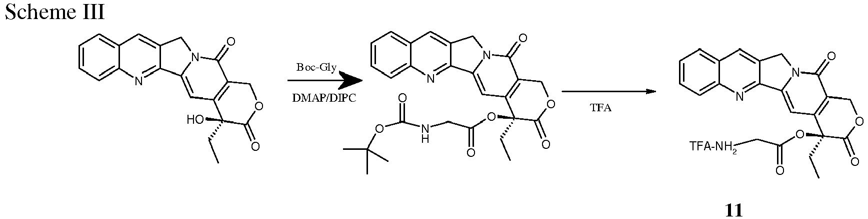

*t*-Boc-glycine (0.9 g, 4.7 mmol) was dissolved in 350 mL of anhydrous methylene chloride at room temperature, and to this solution were added DIPC (0.75 mL, 4.7 mmol), DMAP (382 mg, 3.13 mmol) and camptothecin (0.55g, 1.57 mmol) at 0 °C. The reaction mixture was allowed to warm to room temperature and left for 16 h. The solution was washed with 0.1 N HCl, dried and evaporated under reduced pressure to yield a white solid, which was recrystallized from methanol to give camptothecin-20-ester of *t*-Boc-glycine: ¹H NMR(DMSO-*d*₆) 7.5-8.8 (m), 7.3 (s),5.5 (s), 5.3 (s), 4 (m), 2.1 (m), 1.6 (s), 1.3 (d), 0.9 (t). Camptothecin-20-ester of *t-*Boc-glycine (0.595 g, 1.06 mmol) was dissolved in a mixture of methylene chloride (7.5 mL) and TFA (7.5 mL) and stirred at room temperature for 1 h. Solvent was removed and the residue was recrystallized from methylene chloride and ether to give 0.45 g of **11.** ¹H NMR (DMSO-*d*₆) δ7.7-8.5 (m); 7.2 (s), 5.6 (s), 5.4 (s), 4.4 (m), 2.2 (m), 1.6 (d), 1.0 (t), ¹³C NMR (DMSO-*d*₆) δ168.6, 166.6, 156.5, 152.2, 147.9, 146.2, 144.3, 131.9, 130.6, 129.7, 128.8, 128.6, 128.0, 127.8, 119.0, 95.0, 77.6, 66.6, 50.5, 47.9, 30.2, 15.9, 7.9. ESI/MS (*m*/*z*) expected 405; Found 406 (M+H).

### Example 4: Synthesis and Characterization of CD-BisCys-Peg3400 Copolymers 36 and their CPT Conjugates 37.

### A. Synthesis and Characterization of CD-BisCys-Peg3400 Copolymers 36

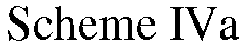

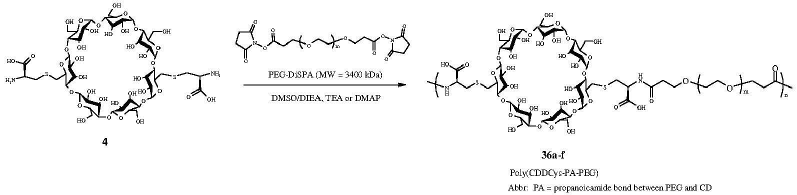

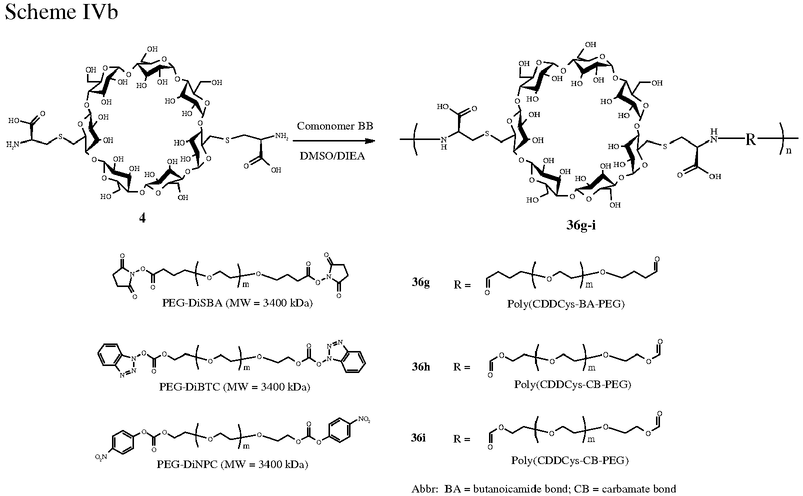

**Synthesis of Poly(CDDCys-PA-PEG), 36a 4** (after precipitation with acetone, 63 mg, 0.047 mmol) and PEG-DiSPA (MW 3400, 160 mg, 0.047 mmol) were dried under vacuum for 8 hours. Anhydrous DMSO (1.26 mL) was added to the mixture under argon. After 10 minutes of stirring, anhydrous diisopropylethylamine (DIEA, 19 *µ*L, 2.3 eq.) was added under argon. The reaction mixture was stirred under argon for 120 h. The polymer containing solution was dialyzed using a 10,000 MWCO membrane (Spectra/Por 7) against water for 48 h and lyophilized to yield 196 mg **36a** (90%, Table 1). *M_{w}* = 57.4 kDa, *Mₙ* = 41.7 kDa, *M_{w}*/*Mₙ* = 1.38. ¹H NMR (D₂O) δ 5.08 (m, CD-2-H), 4.27 (m, Cys-CH), 2.72-3.76 (m, CD-3,4,5,6-CH, CD-CH₂, PEG-CH₂), 2.44 (m, Cys-CH₂).

Synthesis of other poly(CDDCys-PA-PEG) **(36b-f),** Poly(CDDCys-BA-PEG) (**36g**) Poly(CDDCys-CB-PEG) **(36h-i)** were achieved under polymerization condition similar to that of **36a.** Details for the polymerization conditions, monomer selection, polymer molecular weight, polydispersity and yields are listed in Table 6. **36g:** ¹H NMR (D₂O) δ 5.10 (m, CD-2-H), 4.25-4.37 (m, Cys-CH), 2.72-3.86 (m, CD-3,4,5,6-CH, CD-CH₂, PEG-CH₂), 2.21 (m, Cys-CH₂). **36h-i:** ¹H NMR (D₂O) δ 5.05 (m, CD-2-H), 4.56 (m, Cys-CH), 2.70-3.93 (m, CD-3,4,5,6-CH, CD-CH₂, PEG-CH₂), 2.38 (m, -OC*H*₂CH₂CH₂C(O)-NH-), 2.34 (m, Cys-CH₂), 1.90 (m, - OCH₂C*H*₂CH₂C(O)-NH-).

Addition of a non-nucleophilic organic base (such as DIEA) was essential for this polymerization as no viscosity changes of the polymerization solutions were observed after 48 hours if no base was added. When 2.3 eq. of DIEA were added, the viscosity of the polymerization solution increased dramatically after 4-6 hours of reaction. DIEA deprotonates the amino groups of **4** to render them more nucleophilic for coupling with PEG-DiSPA. There were essentially no differences in the polymerizations if other bases, such as TEA or DMAP, were used (**36b-c,** Table 6). Polymerization using **4** recovered by the two different precipitation methods (acetone and methanol) produced polymers with different MWs. **4** that was purified by the methanol-precipitation method (contains no free cystine) gave higher MW polymer (**36d-e**) as compared to the less pure **4** that was obtained from the acetone-precipitation method (**36a**). Polymerization of **4** with PEG-DiSPA typically produced polymer yields greater than 90%.

**4** was polymerized with other activated monomers such as PEG-DiSBA, PEG-DiBTC, and PEG-DiNPC. Reaction of **4** with PEG-DiSBA gave polymer **36g** with similar linkages as **36a-f** (amide bond, but one more -CH₂ group than **36a-f** at the linker) with *M_{w}* over 100 kDa, while reaction of **4** with PEG-DiBTC and PEG-DiNPC generated polymers **36h** and **36i,** respectively, with connecting carbamate moiety and *M_{w}*'s over 50 kDa (Table 6).

**Table 6. Polymerization of 4 with difunctionalized PEG**

| CDP | PEG Comonomer | Base | Polymerization time (h) | *M_{w}* (kDa) | *Mₙ* (kDa) | *M_{w}*/ *Mₙ* | Yield (%) |
|---|---|---|---|---|---|---|---|
| **36a**^{a} | PEG-DiSPA | DIEA | 120 | 57.4 | 41.7 | 1.38 | 90 |
| **36b**^{a} | PEG-DiSPA | DMAP | 120 | 54.2 | 38.1 | 1.42 | 91 |
| **36c**^{a} | PEG-DiSPA | TEA | 120 | 57.4 | 42.6 | 1.35 | 91 |
| **36d**^{b} | PEG-DiSPA | DIEA | 120 | 93.6 | 58.0 | 1.48 | 96 |
| **36e**^{b} | PEG-DiSPA | DIEA | 144 | 97.3 | 58.0 | 1.67 | 94 |
| **36f**^{b} | PEG-DiSPA | DIEA | 2 | 35.3 | 25.6 | 1.38 | 95 |
| **36g** | PEG-DiSBA | DIEA | 120 | 114.7 | 77.9 | 1.47 | 96 |
| **36h** | PEG-DiBTC | DIEA | 120 | 67.6 | 39.4 | 1.47 | 95 |
| **36i** | PEG-DiNPC | DIEA | 120 | 86.5 | 57.2 | 1.51 | 96 |

| | | | | | | | |
|---|---|---|---|---|---|---|---|
| ^{a}**4** was washed with acetone before polymerization. ^{b}**4** was washed with methanol before polymerization. | | | | | | | |

Polymers **36a-i** are highly soluble in aqueous solution. They can be easily dissolved in water or phosphate buffered saline (PBS) solution at concentrations of at least 200 mg/mL. Solubility of these polymers in aqueous solution at concentrations higher than 200 mg/mL was not attempted due to the high viscosity. These polymers were also soluble in DMF, DMSO and methanol, slightly soluble in CH₃CN and CHCl₃, but insoluble in THF and ethyl ether. **Molecular Weight Control of CD Polymers 4** (after precipitation with methanol) (56.2 mg, 0.0419 mmol) and PEG-DiSPA (147 mg, 0.0419 mmol) were dried under vacuum for 4-8 hours. To the mixture was added dry DMSO (1.1 mL) under argon. After 10 minutes stirring, DIEA (16 *µ*L, 2.2 eq) was added under argon. A portion of polymerization solution (150 *µ*L) was removed and precipitated with ether at selected times (2 h, 18 h, 43 h, 70 h, 168 h and 288 h). MWs of the precipitated polymers were determined as described above.

### B. Synthesis of Poly(CDDCys-PA-PEG)-CPT Conjugates (HGGG6, LGGG10, HG6, HGGG10).

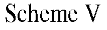

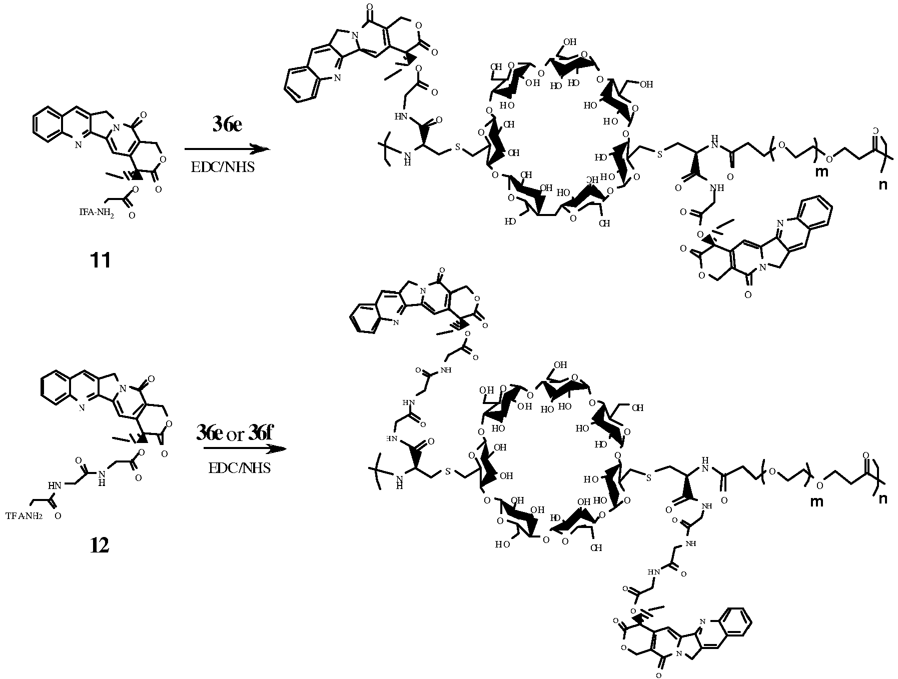

Synthesis of Poly(CDDCys-PA-PEG)-GlyGlyGly-CPT (**HGGG6**) **36e** (1.37 g, 0.30 mmol of repeat unit) was dissolved in dry DMSO (136 mL). The mixture was stirred for 10 minutes. **12** (419 mg, 0.712 mmol, 2.36 eq), DIEA (0.092 mL, 0.712 mmol, 2.36 eq), EDC (172 mg, 0.903 mmol, 3 eq), and NHS (76 mg, 0.662 mmol, 2.2 eq) were added to the polymer solution and stirred for ca. 15 hours. The polymer was precipitated with ethyl ether (1 L). The ether was poured out and the precipitate was washed with CH₃CN (3 × 100 mL). The precipitate was dissolved in water 600 mL. Some insoluble solid was filtered through 0.2 *µ*m filters. The solution was dialyzed using 25,000 MWCO membrane (Spectra/Por 7) for 10 h at 10-15 °C in DI water. Dialysis water was changed every 60 minutes. The polymer-drug conjugate solution was sterilized by passing it through 0.2 µM filters. The solution was lyophilized to yield a yellow solid **HGGG6** (1.42 g, 85% yield).

Synthesis of Poly(CDDCys-PA-PEG)-GlyGlyGly-CPT (**LGGG10**) Conjugation of **12** to **36f** was performed in a manner similar to that used to produce **HGGG6** except that this conjugate was dialyzed with 10,000 MWCO membrane (Spectra/Por 7) instead of with 25,000 MWCO membrane. The yield of **LGGG10** was 83%.
Synthesis of Poly(CDDCys-PA-PEG)-Gly-CPT (**HG6**) Conjugation of **11** to **36e** was performed in a manner similar to that used to produce **HGGG6.** The yield of **HG6** was 83%.

Synthesis of Poly(CDDCys-PA-PEG)-GlyGlyGly-CPT (**HGGG10**) **36e** (1.5 g, 0.33 mmol of repeat unit) was dissolved in dry DMSO (150 mL). The mixture was stirred for 10 minutes. **12** (941 mg, 1.49 mmol, 4.5 eq), DIEA (0.258 mL, 1.49 mmol, 4.5 eq), EDC (283 mg, 1.49 mmol, 4.5 eq), and NHS (113 mg, 0.99 mmol, 3 eq) was added to the polymer solution and stirred for ca. 24 hours. Another portion of EDC (142 mg, 0.75 mmol, 2.3 eq) and NHS (56 mg, 0.5 mmol, 1.5 eq) were added to the conjugation solution. The polymer was stirred for an additional 22 hours. The workup procedure was the same as that for the synthesis of **HGGG6.** The yield of **HGGG10** was 77%.

**Determination of wt% CPT on the Conjugates** Stock solutions of **HGGG6, LGGG10, HG6** and **HGGG10** were prepared at a concentration of 10 mg/mL in DMSO. An aliquot of corresponding stock solution was diluted to 100 *µ*g/mL using 1 N NaOH. CPT was completely hydrolyzed in this basic solution and transformed to its carboxylate form within 2 h at room temperature. An aliquot of this solution was diluted to 10 *µ*g/mL using 8.5% H₃PO₄, and the CPT carboxylate form was transformed to its lactone form. 30 *µ*L of this solution was injected into the HPLC. The peak area from the CPT lactone form was integrated and compared to a standard curve.

**11** and **12** were conjugated to **36e** or **36f** (Table 2) using conventional coupling methods. Due to the instability of the ester linker of **11** and **12** in aqueous solution, the conjugation was conducted in anhydrous DMSO under argon. An organic base was required to deprotonate the TFA salts of **11** and **12** to facilitate the coupling. For polymer conjugation with **12,** the weight percent (wt%) drug loading was around 6-10%. The theoretical maximum drug loading is around 13% using PEG with MW of 3400 Da; maximum values can be increased by decreasing the MW of the PEG segments. Solubilities of all conjugates in water or PBS were more than 200 mg/mL (equivalent to a 12-20 mg CPT/mL for 6-10 wt% drug loading, respectively). Details for the **HGGG6, LGGG10, HG6,** and **HGGG10** are summarized in Table 7.

**Table 7. Properties of polymer-CPT conjugates.**

| Conjugate^{a} | Mw of parent polymer (x 10⁻³) | *Mw*/*Mₙ^{b}* | Linker | CPT (wt%) |
|---|---|---|---|---|
| **HGGG6** | 97 | 1.7 | triglicine | 6.1 |
| **LGGG10** | 35 | 1.6 | triglicine | 10.2 |
| **HG6** | 97 | 1.7 | glicine | 6.8 |
| **HGGG10** | 97 | 1.7 | triglicine | 9.6 |

| | | | | |
|---|---|---|---|---|
| ^{a}Abbreviations: **H** = High *M_{w}* polymer (97 kDa), **L** = Low *M_{w}* polymer (35 kDa), **GGG** = triglycine linker, **G** = glycine linker, **6** = drug loading around 6 wt%, **10** = drug loading around 10 wt%. ^{b}Polymer polydispersity as measured by light scattering techniques(26) | | | | |

### C. Release of CPT from HGGG6 and HG6

*Release of CPT in PBS* **HGGG6** and **HG6** were prepared at 1 mg/mL in PBS (1×, pH 7.4). A 100 µL aliquot of the solution was transferred to a 1.5 mL Eppendorf tube and incubated at 37 °C. The incubated samples were quenched at selected time intervals and stored at -80 °C until the analysis. Each solution was diluted with 8.5% H₃PO₄ to a 5 mL total volume in a volumetric flask. 30 *µ*L of such solution was injected into the HPLC. The peak area from the CPT lactone form was integrated and compared to a standard curve.

Analysis for the release of CPT from **HGGG6** and **HG6** in PBS containing acetyl cholinesterase (an esterase, 100 units/mL), in KH₂PO₄ buffer (pH 6.1, 0.1 M) and in the KH₂PO₄ buffer (pH 6.1, 0.1 M) containing cathepsin B (a cysteine proteinase, 200 *µ*M, preactivated on ice for 30 minutes in this buffer containing 2 mM DTT and 1 mM EDTA) were performed in a manner similar to that described above for PBS alone.

*Release of CPT in Human Plasma* An aliquot of **HGGG6** and **HG6** stock solution were diluted to give final concentration of 0.5 mg/mL in PBS (1×, pH 7.4). This solution was added to a lyophilized powder of human plasma to reconstitute 100% human plasma by the recommended amount. The solution was divided into equal volume (250 *µ*L) to 1.5 mL Eppendorf tubes, incubated at 37 °C, and stopped at selected time point. Samples were stored at -80 °C until the analysis. Samples were separated from plasma by solid phase extraction columns. The solid phase extraction cartridge (Oasis HLB 1cc cartridge from Waters) was pre-conditioned with 1 mL of acetonitrile and then with 1 mL of 8.5% H₃PO₄ before loading. Samples were acidified with equal volume of 8.5% H₃PO₄ prior to loading. After the acidified solution was loaded on the cartridge, the bed was washed with 3 × 1mL of water. Released CPT and polymer conjugate were eluted with 3 × 1 mL of a solution mixture of acetonitrile and potassium phosphate buffer (pH 4.1) (60/40 v/v). The eluted solution was diluted to 5 mL total volume in a 5 mL volumetric flask. 30 *µ*L of such solution was injected into the HPLC. The peak area from the CPT lactone form was integrated and compared to a standard curve.

Release of CPT from **HGGG6** and **HG6** in PBS containing 4% human plasma (PBS/reconstituted human plasma solution = 96/4 (v/v)), in mouse plasma and in reconstituted human albumin (PBS solution) were performed in a manner similar to that described above for pure human plasma.

In PBS (1×, pH 7.4), the half-lives (*t*_{1/2}) for releasing CPT from **HG6** and **HGGG6** were 59h and 32h, respectively. The half-lives decreased to 25h and 22h, respectively, in the presence of 4% human plasma, and to 1.7h and 1.6h, respectively, in 100% human plasma ("HP") and 2.6h and 2.2h, respectively, in 100% mouse plasma ("MP"). CPT release rates for both **HG6** and **HGGG6** in the presence of albumin ("Alb") or acetyl cholinesterase ("Ac Cho") were on the same order of magnitude as in PBS. In a buffer solution at a pH lower than PBS (pH 6.1) with or without the enzyme cathepsin B (active at pH 6.1), less than 50% of total conjugated CPT was released from both **HG6** and **HGGG6** for times up to 144 h (Table 8).

**Table 8. Half-life (t_{1/2}, in hour) of the release of CPT from HG6 and HGGG6^{a}**

| Conjugate | PBS^{b} | 4% HP^{c} | HP^{d} | MP^{e} | Alb^{f} | Ac Cho^{g} | pH 6.1 buffer^{h} | Cath B (pH 6.1)ⁱ |
|---|---|---|---|---|---|---|---|---|
| **HG6** | 59 | 25 | 1.7 | 2.6 | 62 | 33 | >144 | >144 |
| **HGGG6** | 32 | 22 | 1.6 | 2.2 | 73 | 43 | >144 | >144 |

| | | | | | | | | |
|---|---|---|---|---|---|---|---|---|
| ^{a}*t*_{1/2} is defined as time (hours) for the release of half of the total conjugated CPT. Abbreviations: HP means human plasma, MP means mouse plasma. ^{b}pH 7.4 PBS 1x buffer. ^{c}Reconstituted human plasma mixed with PBS (v/v = 4/96). ^{d}Reconstituted human plasma ^{e}Fresh mouse plasma ^{f}In reconstituted human albumin PBS buffer ^{g}In the presence of acetyl cholinesterase PBS solution (100 units/mL). ^{h}pH 6.1 phosphate buffer (0.1M) ⁱpH 6.1 phosphate buffer in the presence of Cathepsin B | | | | | | | | |

### Release of CPT in Solution at Different pH.

**HGGG6** and **HG6** were prepared at 1 mg/mL in buffer solution with pHs ranging from acidic (pH = 1.2) to basic (pH = 13.1) and incubated at 37 °C for 24h. An aliquot of each solution was diluted with 8.5% H₃PO₄ to about 100 *µ*g/mL. 30 *µ*L of such solution was injected into HPLC. The peak area from the CPT lactone form was integrated and compared to a standard curve.

The pH of aqueous solution has a significant effect on the CPT release rates from both **HG6** and **HGGG6.** The amounts of CPT released from **HG6** and **HGGG6** at 37 °C after 24 h in buffer solutions with pHs ranging from 1.1 to 13.1 are illustrated in Figure 6. The glycinyl-CPT ester bonds of both **HG6** and **HGGG6** were very stable in acidic pH (1.1 to 6.4) as less than 7% of CPT were released in 24 h.

### Methods for increasing drug weight percent loading

### Method I. Synthesis of CD-BisCys-Peg Copolymer with a short Peg linkage and its GlyCPT conjugate

### Example 5: Synthesis of CD-BisCys-Peg (short PEG, e.g., Peg200-Peg2000) and its CPT Conjugate 42

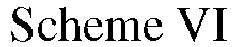

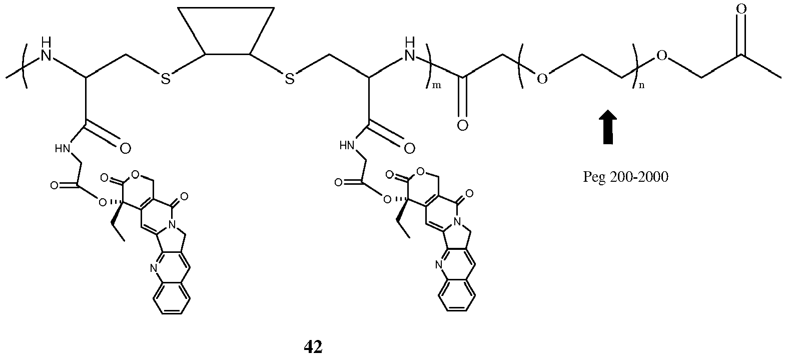

Synthesis of polymer and drug conjugate **42** are same as **36, 37,** and **38**

While Scheme VI shows that the drug is attached at all available positions, not all positions may be reacted. Therefore, a particle comprising conjugates described above may include a conjugate reacted at all positions available for attachment and particles that have less than all of the positions available for attachment containing the drug, e.g., the particle can include CPD reacted at one or none of the positions available for attachment. Thus, while Scheme VI depicts CPT at every point of attachment of each polymer subunit, the CDP-CPT conjugate can have less than 2 CPT molecules attached to any given polymer subunit of the CDP. For example, in one embodiment, the CDP-CPT conjugate includes several polymer subunits and each of the polymer subunits can independently include two, one or no CPT attached at each point of attachment of the polymer subunit. In addition, the particles and compositions can include CDP-CPT conjugates having two, one or no CPT attached to each polymer subunit of the CDP-CPT conjugate and the conjugates can also include a mixture of CDP-CPT conjugates that can vary as to the number of CPTs attached at each point of attachment of the polymer subunits of the conjugates in the particle or composition.

### Method II. Synthesis of CD-BisCys-Peg Copolymer with multiple drug molecules on each loading site.

### Example 6: Synthesis of CD-BisCys-Peg and its GluBis(GlyCPT) Conjugate 43.

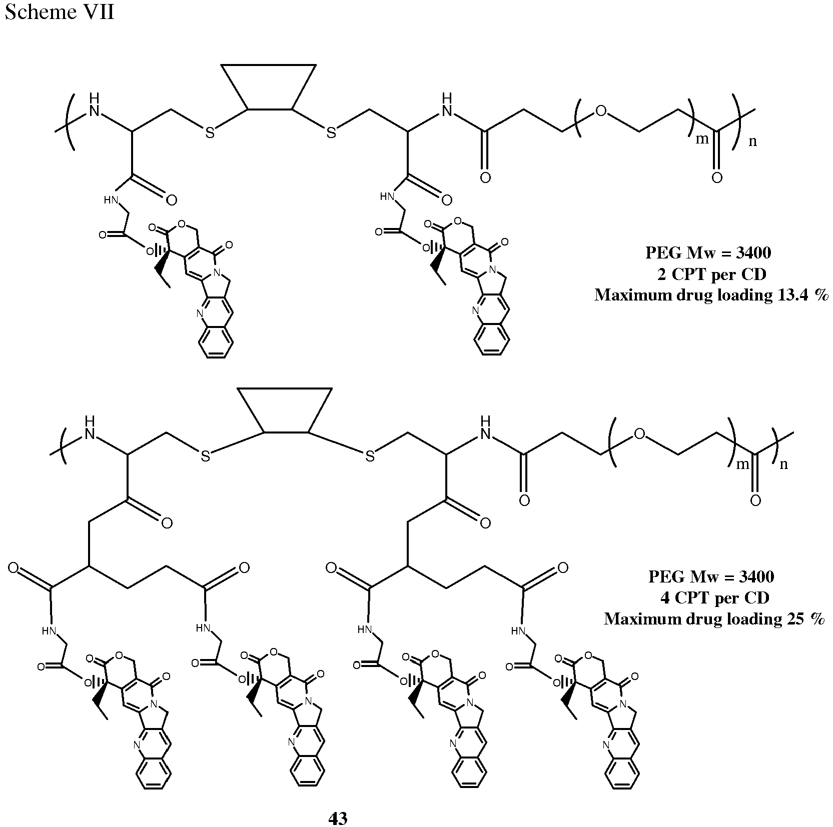

**36** and Glu-Bis(Gly-CPT) **17** are dissolved in DMSO. EDC (3 eq), NHS (2.2 eq), and DIEA (2.2 eq) are added to the solution. CD-BisCys-Peg-GluBis(GlyCPT) **43** is precipitated with CH₃CN and washed with the same solvent until no free drug is detected using UV or TLC. **43** is dried under high vacuum. While Scheme VII shows that the drug is attached at all available positions, not all positions may be reacted. Therefore, a particle comprising conjugates described above may include a conjugate reacted at all positions available for attachment and particles that have less than all of the positions available for attachment containing the drug, e.g., the particle can include CDP reacted at three, two, one or none of the positions available for attachment. Thus, while Scheme VII depicts CPT at every point of attachment of each polymer subunit, the CDP-CPT conjugate can have less than 4 CPT molecules attached to any given polymer subunit of the CDP. For example, in one embodiment, the CDP-CPT conjugate includes several polymer subunits and each of the polymer subunits can independently include four, three, two, one or no CPT attached at each point of attachment of the polymer subunit. In addition, the particles and compositions can include CDP-CPT conjugates having four, three, two, one or no CPT attached to each polymer subunit of the CDP-CPT conjugate and the conjugates can also include a mixture of CDP-CPT conjugates that can vary as to the number of CPTs attached at each point of attachment of the polymer subunits of the conjugates in the particle or composition.

### Example 7: Synthesis and In vitro Analysis of CDP-Gly-SN-38

SN-38 was derivatized with the amino acid glycine at the 20-OH position as shown in Scheme VIII. Briefly, 20(S)-7-ethyl-10-hydroxycamptothecin (SN-38, 1.0g, 2.5 mmol) was dissolved in a mixture of 70 mL dimethylformamide (DMF) and 30 mL pyridine. A solution of di-tert-butyl-dicarbonate (0.83 g, 3.8 mmol) in 10 mL DMF was added and the mixture stirred at room temperature overnight (12 hours). The solvent was removed under vacuum to yield a yellow solid and re-crystallized from boiling 2-propanol (75 mL) to yield 20(s)-10-tert-butoxycarbonyloxy-7-ethyl-camptothecin (Boc-SN-38) as a yellow solid (0.6 g, 48% yield).

Boc-SN-38 (0.73g, 1.5 mmol), N-(tertbutoxycarbonyl)glycine (0.26g, 1.5 mmol) and 4- dimethylaminopyridine (DMAP, 0.18g, 1.5 mmol) were dissolved in anhydrous methylene chloride (30 mL) and chilled to 0°C. 1,3-Diisopropylcarbodiimide (DIPC, 0.19g, 1.5 mmol) was added, the mixture stirred at 0°C for 30 minutes followed by stirring for 4 hours at room temperature. The mixture was diluted with methylene chloride to 100 mL, washed twice with an aqueous solution of 0.1N hydrochloric acid (25 mL), dried over magnesium sulfate and the solvent removed under vacuum. The resulting yellow solid was purified by flash chromatography in methylene chloride:acetone (9:1) followed by solvent removal under vacuum to yield 20-O-(N-(tert-butoxycarbonyl) glycyl)-10-tert-butyoxycarbonyloxy-7-ethylcamptothecin (diBoc-Gly-SN-38, 640 mg, 67% yield). **Scheme VIII:** Derivatization of SN-38 to 20-O-(N-(tert-butoxycarbonyl) glycyl)-10-tert-butyoxycarbonyloxy-7-ethylcamptothecin (diBOC-Gly-SN-38) 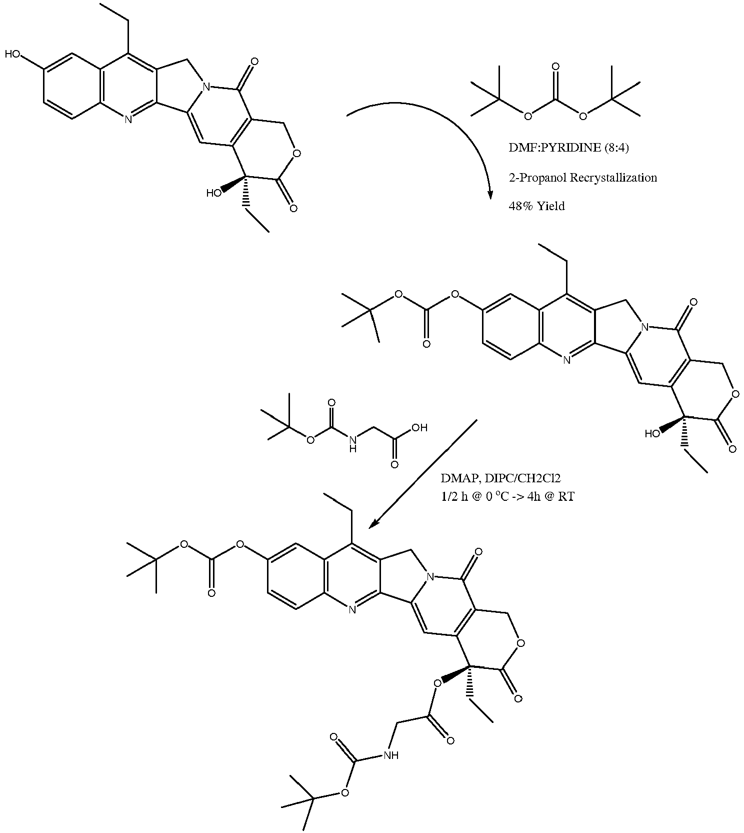 CDP was synthesized as previously described (Cheng et al. (2003) Bioconjugate Chemistry 14(5):1007-1017). diBOC-Gly-SN-38 (0.62g, 0.77 mmol) was deprotected in 15 mL of a 1:1 mixture of methylene chloride:trifluoroacetic acid (TFA) at room temperature for 1 hour. 20-O-trifluoroglycine-10-hydroxy-7-ethylcamptothecin (TFA-Gly-SN-38, 0.57g, 97% yield) was isolated as a yellow solid by precipitation with ethanol (100 mL), followed by two washes with ethanol (30 mL each), dissolution in methylene chloride and removal of solvent under vacuum. ESI/MS expected 449.4; Found 471.66 (M + Na).

CDP-Gly-SN-38 (Poly-CD-PEG-Gly-SN-38, scheme IX) was synthesized as follows: CDP (270 mg, 0.056 mmol), TFA-Gly-SN-38 (70 mg, 0.12 mmol), N-hydroxy-succinimide (14 mg, 0.12 mmol), and 1-ethyl-3-(3-dimethylaminopropyl) carbodiimide (EDCI, 32 mg, 0.17 mmol) were dissolved in dimethylformamide (10 mL) and stirred for 4 hours at room temperature. The polymer was precipitated by addition of 50 mL acetone followed by 50 mL diethyl ether. Precipitate was centrifuged, washed twice with 20 mL acetone each, and dissolved in water acidified to pH 3.0 with hydrochloric acid. Polymer solution was dialized for 24 hours against pH 3.0 water using a 25 kDa MWCO dialysis membrane. The resulting solution was lyophilized to yield CDP-Gly-SN-38 (180 mg, 67% yield). The polymer was analyzed for total and free SN-38 content by HPLC using SN-38 as a standard curve as previously described (Cheng et al. (2003) Bioconjugate Chemistry 14(5):1007-1017). Total SN-38 content was 7.66% w/w of which 97.4% was polymer bound. Average particle size was determined by dynamic light scattering to be 27.9 nm. **Scheme IX:** Structure of a subunit of CDP-Gly-SN-38

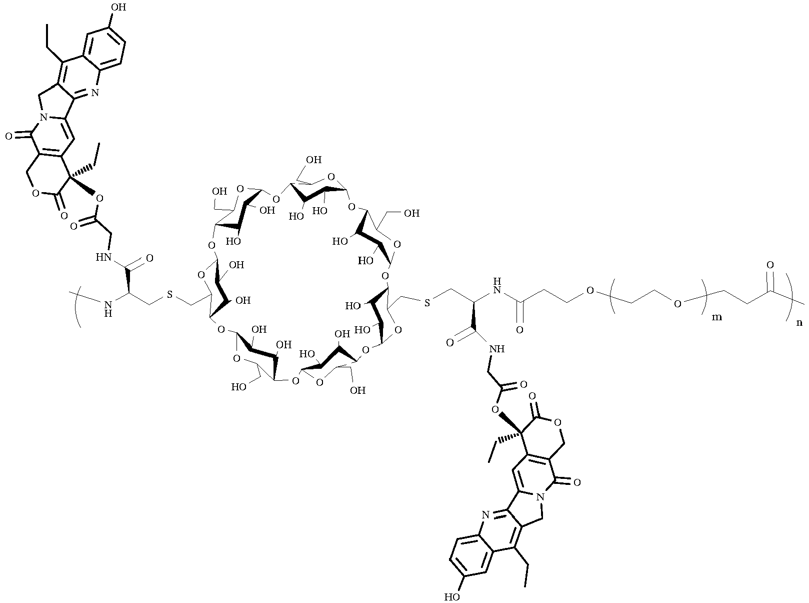

While Scheme IX shows that the drug is attached at all available positions in the subunit, not all positions may be reacted. Therefore, a particle comprising conjugates described above may include a conjugate reacted at all positions available for attachment and particles that have less than all of the positions available for attachment containing the drug, e.g., the particle can include CPD reacted at one or none of the positions available for attachment. Thus, while Scheme IX depicts SN-38 at every point of attachment of each polymer subunit, the CDP-SN-38 conjugate can have less than 2 SN-38 molecules attached to any given polymer subunit of the CDP. For example, in one embodiment, the CDP-SN-38 conjugate includes several polymer subunits and each of the polymer subunits can independently include two, one or no Sn-38 attached at each point of attachment of the polymer subunit. In addition, the particles and compositions can include CDP-SN-38 conjugates having two, one or no Sn-38 attached to each polymer subunit of the CDP-Sn-38 conjugate and the conjugates can also include a mixture of CDP-Sn-38 conjugates that can vary as to the number of Sn-38s attached at each point of attachment of the polymer subunits of the conjugates in the particle or composition.

### In vitro evaluation of CDP-Gly-SN-38

CDP-Gly-SN-38 was evaluated in A2780 human ovarian cancer cell lines *in vitro* as follows:
The human ovarian carcinoma A2780 cells were obtained from the American Type Culture Collection. Cells were seeded in 96-well plates at a concentration of 5,000 cells per well and grown in medium containing 10% fetal bovine serum at 37°C for 24 h in a humidified 5% CO₂ atmosphere. The medium was replaced with fresh medium containing the test compound at concentrations ranging from 0.01 nmol/L to 1 µmol/L. Triplicate wells per plate were treated at each concentration. Controls were vehicle-treated cells and medium only blank. Plates were incubated at 37°C for 72 h. MTS assay reagent was prepared by diluting CellTiter 96 AQueous One Solution (Promega) 5-fold into PBS/glucose (4.5 g/L). Cell culture medium was aspirated and 100 µL of MTS reagent were added to each well. Plates were incubated at 37°C for 1 h. The plates were shaken for 5 min and the absorbance was measured at 485 nm using a SPECTRAFluor Plus plate reader (Tecan). The percentage of cell survival was calculated relative to untreated cells, and IC50s were estimated from the graphs of log dose (nmol/L) versus % cell survival (GraphPad Prizm).

The results of this experiment are shown in Table 9 below.

**Table 9: IC50 of SN-38 and CDP-Gly-SN-38 on A2780 ovarian cells.**

| Compound | IC50 (nM) |
|---|---|
| SN-38 | 2.44 |
| CDP-PEG-Gly-SN-38 | 7.22 |

Other embodiments are in the claims.

## Claims

1. A cyclodextrin-containing polymer (CDP)-topoisomerase inhibitor conjugate for use in a method of treating a cancer in a subject, wherein the method comprises:
providing an initial administration of said CDP-topoisomerase inhibitor conjugate to said subject at a dosage of 12 mg/m², 13 mg/m², 14 mg/m² or 15 mg/m², wherein said dosage is expressed in mg of topoisomerase inhibitor, as opposed to mg of conjugate; and
providing more than one subsequent administration of said CDP-topoisomerase inhibitor conjugate at a dosage of 12 mg/m², 13 mg/m², 14 mg/m² or 15 mg/m², wherein each subsequent administration is provided, independently, between 12, 13, 14, 15 or 16 days after the previous administration;
wherein the method comprises administering said CDP-topoisomerase inhibitor conjugate in combination with one or more additional therapeutic agents;
wherein the CDP-topoisomerase inhibitor conjugate is a CDP-camptothecin conjugate; and
wherein the CDP-camptothecin conjugate is CRLX101.

2. The CDP-topoisomerase inhibitor conjugate for use of claim 1, wherein the conjugate is administered by intravenous administration over a period equal to or less than 30 minutes, 45 minutes or 60 minutes.

3. The CDP-topoisomerase inhibitor conjugate for use of any one of the preceding claims, wherein the one or more additional therapeutic agents include a chemotherapeutic agent.

4. The CDP-topoisomerase inhibitor conjugate for use of claim 3, wherein the chemotherapeutic agent is an angiogenesis inhibitor.

5. The CDP-topoisomerase inhibitor conjugate for use of claim 4, wherein the angiogenesis inhibitor is a vascular endothelial growth factor (VEGF) pathway inhibitor.

6. The CDP-topoisomerase inhibitor conjugate for use of claim 5, wherein the VEGF pathway inhibitor is an anti-VEGF antibody.

7. The CDP-topoisomerase inhibitor conjugate for use of claim 4, wherein the angiogenesis inhibitor is sunitinib, sorafenib, ZD6474 (vandetanib), SU6668 (orantinib), CP-547632, or AZD2171 (cediranib).

8. The CDP-topoisomerase inhibitor conjugate for use of claim 4, wherein the angiogenesis inhibitor is pazopanib, aflibercept, or regorafenib.

9. The CDP-topoisomerase inhibitor conjugate for use of claim 6, wherein the VEGF pathway inhibitor is bevacizumab.

10. The CDP-topoisomerase inhibitor conjugate for use of any one of the preceding claims, wherein the one or more additional therapeutic agents include an agent that reduces one or more side effects associated with the administration of the CDP-topoisomerase inhibitor conjugate.

11. The CDP-topoisomerase inhibitor conjugate for use of any one of the preceding claims, wherein the one or more additional therapeutic agents include an agent that reduces or inhibits one or more symptoms of hypersensitivity.

12. The CDP-topoisomerase inhibitor conjugate for use of claim 11, wherein the one or more symptoms of hypersensitivity are selected from injection site reaction, dyspnea, hypotension, angioedema, urticaria, bronchospasm and erythema.

13. The CDP-topoisomerase inhibitor conjugate for use of claim 11 or claim 12, wherein the agent that reduces or inhibits one or more symptoms of hypersensitivity is a corticosteroid, an antihistamine, and/or an H2 antagonist.

14. The CDP-topoisomerase inhibitor conjugate for use of any one of claims 11 to 13, wherein the agent that reduces or inhibits one or more symptoms of hypersensitivity is dexamethasone, ranitidine, and/or diphenhydramine.

15. The CDP-topoisomerase inhibitor conjugate for use of any one of claims 11 to 14, wherein the agent that reduces or inhibits one or more symptoms of hypersensitivity is administered prior to administration with the CDP-topoisomerase inhibitor conjugate.

16. The CDP-topoisomerase inhibitor conjugate for use of any one of the preceding claims, wherein the cancer is bladder cancer, breast cancer, colon cancer, gastrointestinal cancer, kidney cancer, liver cancer, lung cancer, ovarian cancer, cervical cancer, prostate cancer, lymphoma, rectal cancer, larynx cancer, pancreatic cancer, stomach cancer, gall bladder cancer, thyroid cancer, head and neck cancer, or renal cell carcinoma.

17. The CDP-topoisomerase inhibitor conjugate for use of any one of claims 1 to 15, wherein the cancer is colorectal cancer.

18. The CDP-topoisomerase inhibitor conjugate for use of claim 17, wherein the CDP-topoisomerase inhibitor conjugate is administered in combination with a VEGF pathway inhibitor, e.g., bevacizumab.

19. The CDP-topoisomerase inhibitor conjugate for use of claim 16, wherein the cancer is ovarian cancer.

20. The CDP-topoisomerase inhibitor conjugate for use of claim 16, wherein the cancer is renal cancer, e.g., renal cell carcinoma.

21. The CDP-topoisomerase inhibitor conjugate for use of claim 16, wherein the cancer is rectal cancer.

22. Use of a CDP-topoisomerase inhibitor conjugate for the manufacture of a medicament for the treatment of a cancer in a subject, wherein the treatment comprises:
providing an initial administration of said CDP-topoisomerase inhibitor conjugate to said subject at a dosage of 12 mg/m², 13 mg/m², 14 mg/m² or 15 mg/m², wherein said dosage is expressed in mg of topoisomerase inhibitor, as opposed to mg of conjugate; and
providing more than one subsequent administration of said CDP-topoisomerase inhibitor conjugate, at a dosage of 12 mg/m², 13 mg/m², 14 mg/m² or 15 mg/m², wherein each subsequent administration is provided, independently, between 12, 13, 14, 15 or 16 days after the previous administration;
wherein the treatment comprises administering said CDP-topoisomerase inhibitor conjugate in combination with one or more additional therapeutic agents;
wherein the CDP-topoisomerase inhibitor conjugate is a CDP-camptothecin conjugate; and
wherein the CDP-camptothecin conjugate is CRLX101.

## Patentansprüche

1. Cyclodextrin-enthaltendes Polymer(CDP)-Topoisomerase-Inhibitor-Konjugat zur Verwendung in einem Verfahren zur Behandlung eines Krebses bei einem Patienten, wobei das Verfahren Folgendes umfasst:
Bereitstellen einer initialen Verabreichung des genannten CDP-Topoisomerase-Inhibitor-Konjugats an den genannten Patienten in einer Dosierung von 12 mg/m², 13 mg/m², 14 mg/m² oder 15 mg/m², wobei die genannte Dosierung in mg Topoisomerase-Inhibitor, im Gegensatz zu mg Konjugat, ausgedrückt ist; und
Bereitstellen von mehr als einer anschließenden Verabreichung von genanntem CDP-Topoisomerase-Inhibitor-Konjugat in einer Dosierung von 12 mg/m², 13 mg/m², 14 mg/m² oder 15 mg/m², wobei jede anschließende Verabreichung unabhängig zwischen 12, 13, 14, 15 und 16 Tagen nach der vorherigen Verabreichung bereitgestellt wird;
wobei das Verfahren die Verabreichung von genanntem CDP-Topoisomerase-Inhibitor-Konjugat in Kombination mit einem oder mehr zusätzlichen therapeutischen Mittel(n) umfasst;
wobei das CDP-Topoisomerase-Inhibitor-Konjugat ein CDP-Camptothecin-Konjugat ist; und
wobei das CDP-Camptothecin-Konjugat CRLX101 ist.

2. CDP-Topoisomerase-Inhibitor-Konjugat zur Verwendung nach Anspruch 1, wobei das Konjugat durch intravenöse Verabreichung über eine Zeitdauer von gleich oder weniger als 30 Minuten, 45 Minuten oder 60 Minuten verabreicht wird.

3. CDP-Topoisomerase-Inhibitor-Konjugat zur Verwendung nach einem der vorangehenden Ansprüche, wobei das eine oder mehr zusätzliche therapeutische Mittel ein chemotherapeutisches Mittel einschließt/einschließen.

4. CDP-Topoisomerase-Inhibitor-Konjugat zur Verwendung nach Anspruch 3, wobei das chemotherapeutische Mittel ein Angiogeneseinhibitor ist.

5. CDP-Topoisomerase-Inhibitor-Konjugat zur Verwendung nach Anspruch 4, wobei der Angiogeneseinhibitor ein Inhibitor des vaskulären endothelialen Wachstumsfaktor(VEGF)-Pfads ist.

6. CDP-Topoisomerase-Inhibitor-Konjugat zur Verwendung nach Anspruch 5, wobei der Inhibitor des VEGF-Pfads ein Anti-VEGF-Antikörper ist.

7. CDP-Topoisomerase-Inhibitor-Konjugat zur Verwendung nach Anspruch 4, wobei der Angiogeneseinhibitor Sunitinib, Sorafenib, ZD6474 (Vandetanib), SU6668 (Orantinib), CP-547632 oder AZD2171 (Cediranib) ist.

8. CDP-Topoisomerase-Inhibitor-Konjugat zur Verwendung nach Anspruch 4, wobei der Angiogeneseinhibitor Pazopanib, Aflibercept oder Regorafenib ist.

9. CDP-Topoisomerase-Inhibitor-Konjugat zur Verwendung nach Anspruch 6, wobei der Inhibitor des VEGF-Pfads Bevacizumab ist.

10. CDP-Topoisomerase-Inhibitor-Konjugat zur Verwendung nach einem der vorangehenden Ansprüche, wobei das eine oder mehr zusätzliche therapeutische Mittel ein Mittel einschließt/einschließen, das eine oder mehr Nebenwirkung(en), assoziert mit der Verabreichung des CDP-Topoisomerase-Inhibitor-Konjugats, reduziert.

11. CDP-Topoisomerase-Inhibitor-Konjugat zur Verwendung nach einem der vorangehenden Ansprüche, wobei das eine oder mehr zusätzliche therapeutische Mittel ein Mittel einschließt/einschließen, das ein oder mehr Überempfindlichkeitssymptom(e) reduziert oder inhibiert.

12. CDP-Topoisomerase-Inhibitor-Konjugat zur Verwendung nach Anspruch 11, wobei das eine oder mehr Überempfindlichkeitssymptom(e) ausgewählt ist/sind aus einer Reaktion an der Injektionstelle, Dyspnoe, Hypotonie, einem Angioödem, Urtikaria, Bronchospasmus und einem Erythem.

13. CDP-Topoisomerase-Inhibitor-Konjugat zur Verwendung nach Anspruch 11 oder Anspruch 12, wobei das Mittel, das ein oder mehr Überempfindlichkeitssymptom(e) reduziert oder inhibiert, ein Kortikosteroid, ein Antihistamin und/oder ein H₂-Antagonist ist.

14. CDP-Topoisomerase-Inhibitor-Konjugat zur Verwendung nach einem der Ansprüche 11 bis 13, wobei das Mittel, das ein oder mehr Überempfindlichkeitsymptom(e) reduziert oder inhibiert Dexamethason, Ranitidin und/oder Diphenhydramin ist.

15. CDP-Topoisomerase-Inhibitor-Konjugat zur Verwendung nach einem der Ansprüche 11 bis 14, wobei das Mittel, das ein oder mehr Überempfindlichkeitsymptom(e) reduziert oder inhibiert, vor der Verabreichung mit dem CDP-Topoisomerase-Inhibitor-Konjugat verabreicht wird.

16. CDP-Topoisomerase-Inhibitor-Konjugat zur Verwendung nach einem der vorangehenden Ansprüche, wobei der Krebs Blasenkrebs, Brustkrebs, Kolonkrebs, Gastrointestinalkrebs, Nierenkrebs, Leberkrebs, Lungenkrebs, Ovarialkrebs, Zervikalkrebs, Prostatakrebs, ein Lymphom, Rektalkrebs, Larynxkrebs, Pankreaskrebs, Magenkrebs, Gallenblasenkrebs, Schilddrüsenkrebs, Kopf- und Halskrebs oder ein Nierenzellkarzinom ist.

17. CDP-Topoisomerase-Inhibitor-Konjugat zur Verwendung nach einem der Ansprüche 1 bis 15, wobei der Krebs Kolorektalkrebs ist.

18. CDP-Topoisomerase-Inhibitor-Konjugat zur Verwendung nach Anspruch 17, wobei das CDP-Topoisomerase-Inhibitor-Konjugat in Kombination mit einem Inhibitor des VEGF-Pfads, wie zum Beispiel Bevacizumab, verabreicht wird.

19. CDP-Topoisomerase-Inhibitor-Konjugat zur Verwendung nach Anspruch 16, wobei der Krebs Ovarialkrebs ist.

20. CDP-Topoisomerase-Inhibitor-Konjugat zur Verwendung nach Anspruch 16, wobei der Krebs Nierenkrebs, wie zum Beispiel ein Nierenzellkarzinom, ist.

21. CDP-Topoisomerase-Inhibitor-Konjugat zur Verwendung nach Anspruch 16, wobei der Krebs Rektalkrebs ist.

22. Verwendung von einem CDP-Topoisomerase-Inhibitor-Konjugat zur Herstellung eines Arzneimittels für die Behandlung eines Krebses bei einem Patienten, wobei die Behandlung Folgendes umfasst:
Bereitstellen einer initialen Verabreichung des genannten CDP-Topoisomerase-Inhibitor-Konjugats an den genannten Patienten in einer Dosierung von 12 mg/m², 13 mg/m², 14 mg/m² oder 15 mg/m², wobei die genannte Dosierung in mg Topoisomerase-Inhibitor, im Gegensatz zu mg Konjugat, ausgedrückt ist; und
Bereitstellen von mehr als einer anschließenden Verabreichung von genanntem CDP-Topoisomerase-Inhibitor-Konjugat in einer Dosierung von 12 mg/m², 13 mg/m², 14 mg/m² oder 15 mg/m², wobei jede anschließende Verabreichung unabhängig zwischen 12, 13, 14, 15 und 16 Tagen nach der vorherigen Verabreichung bereitgestellt wird;
wobei das Verfahren die Verabreichung von genanntem CDP-Topoisomerase-Inhibitor-Konjugat in Kombination mit einem oder mehr zusätzlichen therapeutischen Mittel(n) umfasst;
wobei das CDP-Topoisomerase-Inhibitor-Konjugat ein CDP-Camptothecin-Konjugat ist; und
wobei das CDP-Camptothecin-Konjugat CRLX101 ist.

## Revendications

1. Conjugué de polymère contenant de la cyclodextrine (CDP)-inhibiteur de la topoisomérase destiné à une utilisation dans une méthode de traitement d'un cancer chez un sujet, la méthode comprenant :
le fait de pratiquer une administration initiale dudit conjugué de CDP-inhibiteur de la topoisomérase audit sujet à une dose de 12 mg/m², 13 mg/m², 14 mg/m² ou 15 mg/m², ladite dose étant exprimée en mg d'inhibiteur de la topoisomérase, et non en mg de conjugué ; et
le fait de pratiquer plus d'une administration ultérieure dudit conjugué de CDP-inhibiteur de la topoisomérase à une dose de 12 mg/m², 13 mg/m², 14 mg/m² ou 15 mg/m², chaque administration ultérieure étant pratiquée, indépendamment, entre 12, 13, 14, 15 ou 16 jours après l'administration précédente ;
la méthode comprenant l'administration dudit conjugué de CDP-inhibiteur de la topoisomérase en combinaison avec un ou plusieurs agents thérapeutiques additionnels ;
le conjugué de CDP-inhibiteur de la topoisomérase étant un conjugué de CDP-camptothécine ; et
le conjugué de CDP-camptothécine étant CRLX101.

2. Conjugué de CDP-inhibiteur de la topoisomérase destiné à une utilisation selon la revendication 1, le conjugué étant administré par voie intraveineuse sur une période égale ou inférieure à 30 minutes, 45 minutes ou 60 minutes.

3. Conjugué de CDP-inhibiteur de la topoisomérase destiné à une utilisation selon l'une quelconque des revendications précédentes, lesdits un ou plusieurs agents thérapeutiques additionnels comprenant un agent de chimiothérapie.

4. Conjugué de CDP-inhibiteur de la topoisomérase destiné à une utilisation selon la revendication 3, l'agent de chimiothérapie étant un inhibiteur de l'angiogenèse.

5. Conjugué de CDP-inhibiteur de la topoisomérase destiné à une utilisation selon la revendication 4, l'inhibiteur de l'angiogenèse étant un inhibiteur de la voie du facteur de croissance endothélial vasculaire (VEGF).

6. Conjugué de CDP-inhibiteur de la topoisomérase destiné à une utilisation selon la revendication 5, l'inhibiteur de la voie du VEGF étant un anticorps anti-VEGF.

7. Conjugué de CDP-inhibiteur de la topoisomérase destiné à une utilisation selon la revendication 4, l'inhibiteur de l'angiogenèse étant le sunitinib, le sorafénib, le ZD6474 (vandétanib), le SU6668 (orantinib), le CP-547632 ou l'AZD2171 (cédiranib).

8. Conjugué de CDP-inhibiteur de la topoisomérase destiné à une utilisation selon la revendication 4, l'inhibiteur de l'angiogenèse étant le pazopanib, l'aflibercept ou le régorafénib.

9. Conjugué de CDP-inhibiteur de la topoisomérase destiné à une utilisation selon la revendication 6, l'inhibiteur de la voie du VEGF étant le bevacizumab.

10. Conjugué de CDP-inhibiteur de la topoisomérase destiné à une utilisation selon l'une quelconque des revendications précédentes, lesdits un ou plusieurs agents thérapeutiques additionnels comprenant un agent qui atténue un ou plusieurs effets secondaires associés à l'administration du conjugué de CDP-inhibiteur de la topoisomérase.

11. Conjugué de CDP-inhibiteur de la topoisomérase destiné à une utilisation selon l'une quelconque des revendications précédentes, lesdits un ou plusieurs agents thérapeutiques additionnels comprenant un agent qui atténue ou inhibe un ou plusieurs symptômes d'hypersensibilité.

12. Conjugué de CDP-inhibiteur de la topoisomérase destiné à une utilisation selon la revendication 11, lesdits un ou plusieurs symptômes d'hypersensibilité étant sélectionnés parmi une réaction au niveau d'un site d'injection, une dyspnée, une hypotension, un angio-oedème, une urticaire, un bronchospasme et un érythème.

13. Conjugué de CDP-inhibiteur de la topoisomérase destiné à une utilisation selon la revendication 11 ou la revendication 12, l'agent qui atténue ou inhibe un ou plusieurs symptômes d'hypersensibilité étant un corticostéroïde, un antihistaminique et/ou un antagoniste de H2.

14. Conjugué de CDP-inhibiteur de la topoisomérase destiné à une utilisation selon l'une quelconque des revendications 11 à 13, l'agent qui atténue ou inhibe un ou plusieurs symptômes d'hypersensibilité étant la dexaméthasone, la ranitidine, et/ou la diphénhydramine.

15. Conjugué de CDP-inhibiteur de la topoisomérase destiné à une utilisation selon l'une quelconque des revendications 11 à 14, l'agent qui atténue ou inhibe un ou plusieurs symptômes d'hypersensibilité étant administré avant l'administration du conjugué de CDP-inhibiteur de la topoisomérase.

16. Conjugué de CDP-inhibiteur de la topoisomérase destiné à une utilisation selon l'une quelconque des revendications précédentes, le cancer étant un cancer de la vessie, un cancer du sein, un cancer du côlon, un cancer gastro-intestinal, un cancer du rein, un cancer du foie, un cancer du poumon, un cancer ovarien, un cancer du col de l'utérus, un cancer de la prostate, un lymphome, un cancer rectal, un cancer du larynx, un cancer du pancréas, un cancer de l'estomac, un cancer de la vésicule biliaire, un cancer de la thyroïde, un cancer des voies aérodigestives supérieures, ou un carcinome des cellules rénales.

17. Conjugué de CDP-inhibiteur de la topoisomérase destiné à une utilisation selon l'une quelconque des revendications 1 à 15, le cancer étant un cancer colorectal.

18. Conjugué de CDP-inhibiteur de la topoisomérase destiné à une utilisation selon la revendication 17, le conjugué de CDP-inhibiteur de la topoisomérase étant administré en combinaison avec un inhibiteur de la voie du VEGF, par exemple le bevacizumab.

19. Conjugué de CDP-inhibiteur de la topoisomérase destiné à une utilisation selon la revendication 16, le cancer étant un cancer ovarien.

20. Conjugué de CDP-inhibiteur de la topoisomérase destiné à une utilisation selon la revendication 16, le cancer étant un cancer rénal, par exemple un carcinome rénal.

21. Conjugué de CDP-inhibiteur de la topoisomérase destiné à une utilisation selon la revendication 16, le cancer étant un cancer rectal.

22. Utilisation d'un conjugué de CDP-inhibiteur de la topoisomérase pour la fabrication d'un médicament destiné au traitement d'un cancer chez un sujet, le traitement comprenant :
le fait de pratiquer une administration initiale dudit conjugué de CDP-inhibiteur de la topoisomérase audit sujet à une dose de 12 mg/m², 13 mg/m², 14 mg/m² ou 15 mg/m², ladite dose étant exprimée en mg d'inhibiteur de la topoisomérase, et non en mg de conjugué ; et
le fait de pratiquer plus d'une administration ultérieure dudit conjugué de CDP-inhibiteur de la topoisomérase à une dose de 12 mg/m², 13 mg/m², 14 mg/m² ou 15 mg/m², chaque administration ultérieure étant pratiquée, indépendamment, entre 12, 13, 14, 15 ou 16 jours après l'administration précédente ;
le traitement comprenant l'administration dudit conjugué de CDP-inhibiteur de la topoisomérase en combinaison avec un ou plusieurs agents thérapeutiques additionnels ;
le conjugué de CDP-inhibiteur de la topoisomérase étant un conjugué de CDP-camptothécine ; et
le conjugué de CDP-camptothécine étant CRLX101.
